# EUROPEAN PATENT APPLICATION

(11) **EP 4 821 495 A2**
(43) Date of publication of application: **14.10.2026**
(21) Application number: 26174326.4
(22) Date of filing: 08.11.2017
(51) Int. Cl.: A61K 31/277

(54) **METHODS OF TREATING ALPORT SYNDROME USING BARDOXOLONE METHYL OR ANALOGS THEREOF**

(30) Priority: 08.11.2016 US 201662419335 P; 21.07.2017 US 201762535663 P; 02.11.2017 US 201762580597 P
(62) Document number(s) of the earlier application(s) in accordance with Art. 76 EPC: 17801274.6 / 3 538 104
(71) Applicant: Reata Pharmaceuticals Holdings, LLC, Plano, TX 75024 (US)
(72) Inventor: MEYER, Colin J., Southlake, 76092 (US)
(74) Representative: Potter Clarkson

(57) **Abstract**

The present invention provides methods of treating or preventing Alport syndrome in a patients in need thereof using bardoxolone methyl or analogs thereof, and/or improving the kidney function of patients who have been diagnosed with Alport syndrome.

## Description

The present application claims the priority benefit of United States provisional application number 62/580,597, filed November 2, 2017, United States provisional application number 62/535,663, filed July 21, 2017, and United States provisional application number 62/419,335, filed November 8, 2016, the entire contents of each of which is incorporated herein by reference.

### BACKGROUND OF THE INVENTION

### 1. Field of the Invention

The present invention relates generally to the fields of biology and medicine. More particularly, it concerns, in some aspects, methods for treating or preventing Alport syndrome or symptoms thereof using bardoxolone methyl and analogs thereof.

### 2. Description of Related Art

Alport syndrome is a rare and serious hereditary disease caused by mutations in the genes encoding Type IV collagen, a major structural component of the glomerular basement membrane (GBM) in the kidney. Patients with Alport syndrome exhibit ultrastructural changes in the GBM due to the abnormal expression of Type IV collagen chains in the kidney. Loss of GBM integrity results in abnormal leakage of proteins, such as albumin, which are then excessively reabsorbed in the tubules and activate pro-inflammatory signaling pathways in glomerular endothelial cells, mesangial cells, and podocytes. Chronic activation of pro-inflammatory pathways in these kidney cells promotes glomerular filtration rate (GFR) loss by several mechanisms: (a) in glomerular endothelial cells, inflammation-associated reactive oxygen species (ROS) induce the production of peroxynitrite, which depletes vasodilatory nitric oxide and results in endothelial dysfunction, vasoconstriction and reduced glomerular surface area for filtration; (b) inflammation-associated ROS induce a contractile response in mesangial cells, further reducing filtration; and (c) ROS-mediated activation of inflammatory pathways leads to fibrosis, promoting structural alterations in the mesangium and GBM thickening that contributes to GFR decline. GFR decline from these processes inevitably leads to end stage renal disease (ESRD).

Bardoxolone methyl has been shown to improve both estimated glomerular filtration rate (eGFR) and measured glomerular filtration rate (mGFR) in patients with CKD due to Type 2 diabetes. Bardoxolone methyl and several of its analogues also have been shown to inhibit pro-fibrotic signaling pathways and reduce oxidative stress and inflammation in multiple models of CKD. These compounds have also shown inhibition of inflammatory signaling and fibrosis in animal models of liver, skin, and lung disorders.

Despite the severe consequences of Alport syndrome including ESRD, there remain only limited treatment options for this condition. Therefore, there remains a therapeutic need to develop new and effective methods of treating and/or preventing Alport syndrome, the associated symptoms thereof, as well as preventing the onset of such symptoms.

### SUMMARY OF THE INVENTION

In one aspect, the present invention provides methods of treating or preventing Alport syndrome in a patients in need thereof, and/or improving the kidney function of patients who has been diagnosed with Alport syndrome. Such methods are described in the sections below, including for example the claims section, which is incorporated herein by reference.

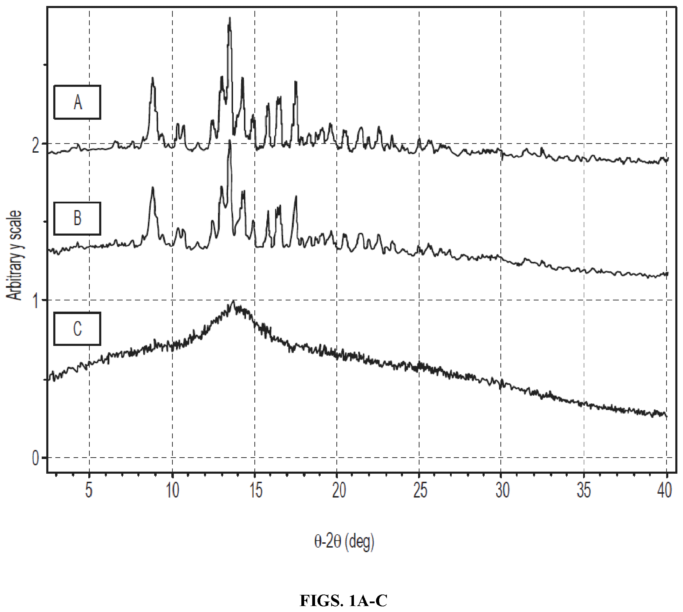

In some embodiments, the compound is bardoxolone methyl (CDDO-Me or RTA 402). And in some of these embodiments, at least a portion of the CDDO-Me is present as a polymorphic form, wherein the polymorphic form is a crystalline form having an X-ray diffraction pattern (CuKα) comprising significant diffraction peaks at about 8.8, 12.9, 13.4, 14.2 and 17.4 °20. In non-limiting examples, the X-ray diffraction pattern (CuKα) is substantially as shown in FIG. 1A or FIG. 1B. In other variations, at least a portion of the CDDO-Me is present as a polymorphic form, wherein the polymorphic form is an amorphous form having an X-ray diffraction pattern (CuKα) with a halo peak at approximately 13.5 °2θ, substantially as shown in FIG. 1C, and a T_{g}. In some variations, the compound is an amorphous form. In some variations, the compound is a glassy solid form of CDDO-Me, having an X-ray powder diffraction pattern with a halo peak at about 13.5 °2θ, as shown in FIG. 1C, and a T_{g}. In some variations, the T_{g} value falls within a range of about 120 °C to about 135 °C. In some variations, the T_{g} value is from about 125 °C to about 130 °C.

In some embodiments, the compound is administered locally. In some embodiments, the compound is administered systemically. In some embodiments, the compound is administered orally, intraadiposally, intraarterially, intraarticularly, intracranially, intradermally, intralesionally, intramuscularly, intranasally, intraocularly, intrapericardially, intraperitoneally, intrapleurally, intraprostatically, intrarectally, intrathecally, intratracheally, intratumorally, intraumbilically, intravaginally, intravenously, intravesicularlly, intravitreally, liposomally, locally, mucosally, orally, parenterally, rectally, subconjunctivally, subcutaneously, sublingually, topically, transbuccally, transdermally, vaginally, in crèmes, in lipid compositions, via a catheter, via a lavage, via continuous infusion, via infusion, via inhalation, via injection, via local delivery, via localized perfusion, bathing target cells directly, or any combination thereof. For example, in some variations, the compound is administered intravenously, intra-arterially or orally. For example, in some variations, the compound is administered orally.

In some embodiments, the compound is formulated as a hard or soft capsule, a tablet, a syrup, a suspension, a solid dispersion, a wafer, or an elixir. In some variations, the soft capsule is a gelatin capsule. In variations, the compound is formulated as a solid dispersion. In some variations the hard capsule, soft capsule, tablet or wafer further comprises a protective coating. In some variations, the formulated compound comprises an agent that delays absorption. In some variations, the formulated compound further comprises an agent that enhances solubility or dispersibility. In some variations, the compound is dispersed in a liposome, an oil-in-water emulsion or a water-in-oil emulsion.

In some embodiments, the pharmaceutically effective amount is a daily dose from about 0.1 mg to about 500 mg of the compound. In some variations, the daily dose is from about 1 mg to about 300 mg of the compound. In some variations, the daily dose is from about 10 mg to about 200 mg of the compound. In some variations, the daily dose is about 25 mg of the compound. In other variations, the daily dose is about 75 mg of the compound. In still other variations, the daily dose is about 150 mg of the compound. In further variations, the daily dose is from about 0.1 mg to about 30 mg of the compound. In some variations, the daily dose is from about 0.5 mg to about 20 mg of the compound. In some variations, the daily dose is from about 1 mg to about 15 mg of the compound. In some variations, the daily dose is from about 1 mg to about 10 mg of the compound. In some variations, the daily dose is from about 1 mg to about 5 mg of the compound. In some variations, the daily dose is from about 2.5 mg to about 30 mg of the compound. In some variations, the daily dose is about 2.5 mg of the compound. In other variations, the daily dose is about 5 mg of the compound. In other variations, the daily dose is about 10 mg of the compound. In other variations, the daily dose is about 15 mg of the compound. In other variations, the daily dose is about 20 mg of the compound. In still other variations, the daily dose is about 30 mg of the compound.

In some embodiments, the pharmaceutically effective amount is a daily dose of 0.01 - 25 mg of compound per kg of body weight. In some variations, the daily dose is 0.05 - 20 mg of compound per kg of body weight. In some variations, the daily dose is 0.1 - 10 mg of compound per kg of body weight. In some variations, the daily dose is 0.1 - 5 mg of compound per kg of body weight. In some variations, the daily dose is 0.1 - 2.5 mg of compound per kg of body weight.

In some embodiments, the pharmaceutically effective amount is administered in a single dose per day. In some embodiments, the pharmaceutically effective amount is administered in two or more doses per day.

In some embodiments, the patient is a mammal such as primate. In some variations, the primate is a human. In other variations, the patient is a cow, horse, dog, cat, pig, mouse, rat or guinea pig.

In some variations of the above methods, the compound is substantially free from optical isomers thereof. In some variations of the above methods, the compound is in the form of a pharmaceutically acceptable salt. In other variations of the above methods, the compound is not a salt.

In some embodiments, the compound is formulated as a pharmaceutical composition comprising (i) a therapeutically effective amount of the compound and (ii) an excipient selected from the group consisting of (A) a carbohydrate, carbohydrate derivative, or carbohydrate polymer, (B) a synthetic organic polymer, (C) an organic acid salt, (D) a protein, polypeptide, or peptide, and (E) a high molecular weight polysaccharide. In some variations, the excipient is a synthetic organic polymer. In some variations, the excipient is selected from the group consisting of a hydroxypropyl methyl cellulose, a poly[1-(2-oxo-1-pyrrolidinyl)ethylene] or copolymer thereof, and a methacrylic acid - methylmethacrylate copolymer. In some variations, the excipient is hydroxypropyl methyl cellulose phthalate ester. In some variations, the excipient is PVP/VA. In some variations, the excipient is a methacrylic acid - ethyl acrylate copolymer. In some variations, the methacrylic acid and ethyl acrylate may be present at a ratio of about 1:1. In some variations, the excipient is copovidone.

Any embodiment discussed herein with respect to one aspect of the invention applies to other aspects of the invention as well, unless specifically noted.

Further aspects and embodiments of this invention are elaborated in greater detail, for example, in the claims section, which is incorporated herein by reference.

Other objects, features and advantages of the present disclosure will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description. Note that simply because a particular compound is ascribed to one particular generic formula does not mean that it cannot also belong to another generic formula.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIGS. 1A-C** - X-ray Powder Diffraction (XRPD) Spectra of Forms A and B of RTA 402. FIG. 1A shows unmicronized Form A; FIG. 1B shows micronized Form A; FIG. 1C shows Form B.

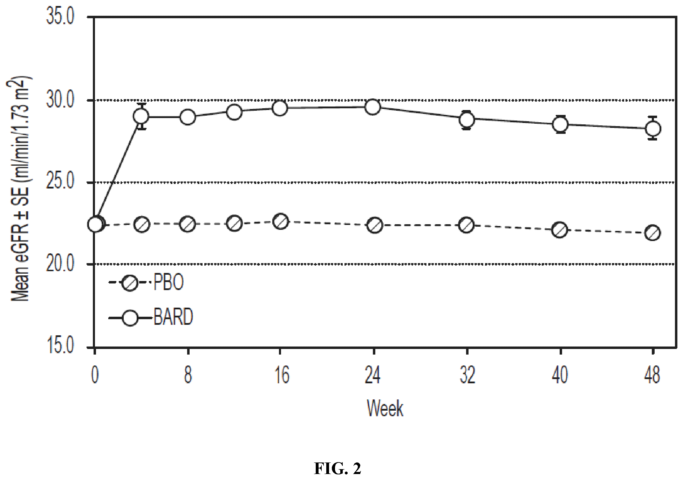

**FIG. 2** - Mean eGFR Over Time in BEACON (Safety Population). Mean observed eGFR over time by treatment week in placebo (PBO) versus bardoxolone methyl (BARD) patients. Only includes assessments of eGFR collected on or before a patient's last dose of study drug. Visits are derived relative to a patient's first dose of study drug. Data are mean ± SE.

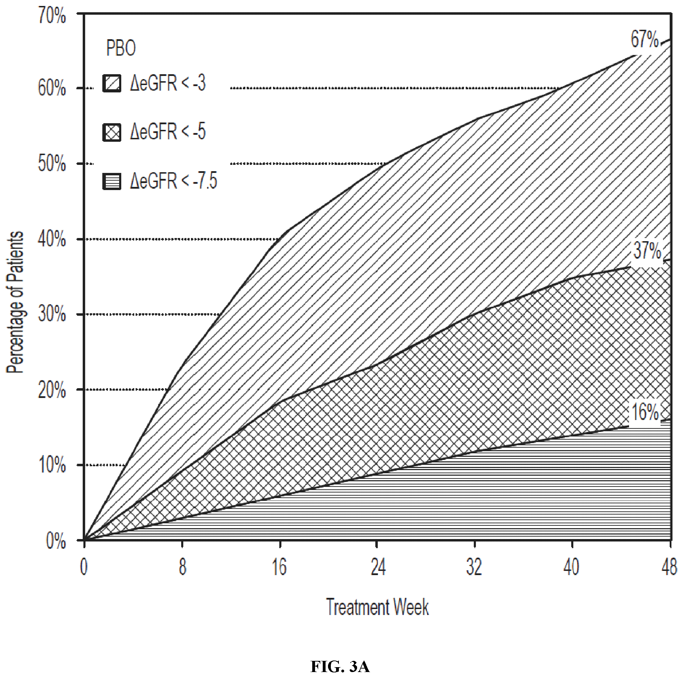

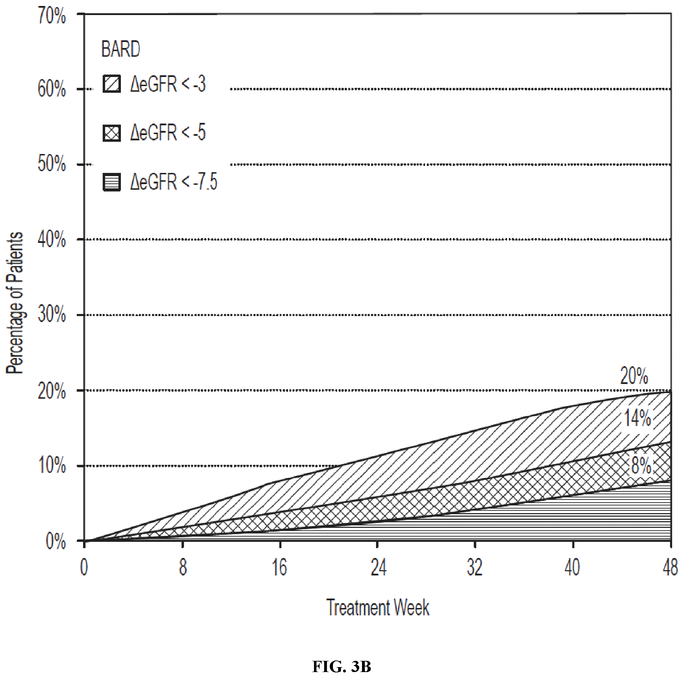

**FIGS. 3A-B** - Percentage of eGFR Decliners in Bardoxolone Methyl vs. Placebo Patients in BEACON (Safety Population). Percentage of patients with changes in eGFR from baseline of < -3, < -5, or < -7.5 mL/min/1.73 m² by treatment week in placebo (PBO) (FIG. 3A) versus bardoxolone methyl (BARD) (FIG. 3B) patients. Only includes assessments of eGFR collected on or before a patient's last dose of study drug. Visits are derived relative to a patient's first dose of study drug. Percentages calculated relative to number of patients with available eGFR data at each visit. The number of placebo patients with data for FIG. 3A were 1093 at Week 0, 1023 at Week 8, 885 at Week 16, 726 at Week 24, 547 at Week 32, 402 at Week 40, and 281 at Week 48. The number of placebo patients with data for FIG. 3B were 1092 at Week 0, 958 at Week 8, 795 at Week 16, 628 at Week 24, 461 at Week 32, 345 at Week 40, and 241 at Week 48.

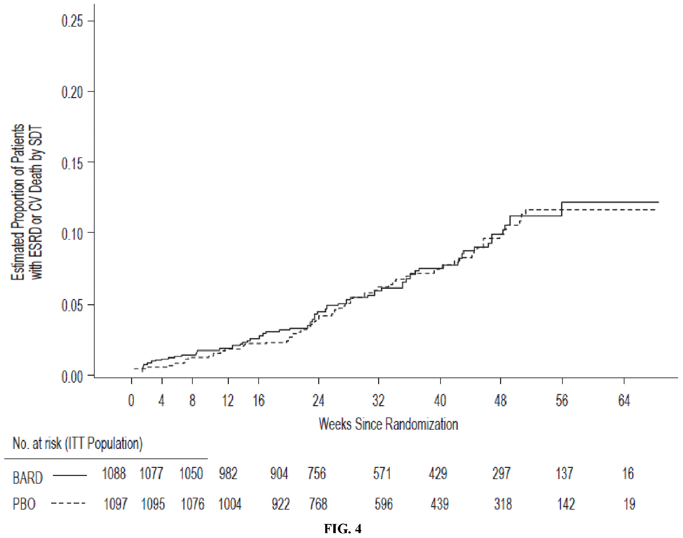

**FIG. 4** - Time to Composite Primary Outcome Event in BEACON (ITT Population). Results from a randomized, double-blind, placebo-controlled phase 3 study in T2D patients with Stage 4 CKD (BEACON, RTA402-C-0903). Patients were administered placebo (PBO) or 20 mg of bardoxolone methyl (BARD) once daily. Analysis includes only end-stage renal disease (ESRD) or cardiovascular (CV) death events occurring on or prior to study drug termination date (SDT) (October 18, 2012) that were positively adjudicated by an independent Event Adjudication Committee, as outlined in the BEACON EAC Charter.

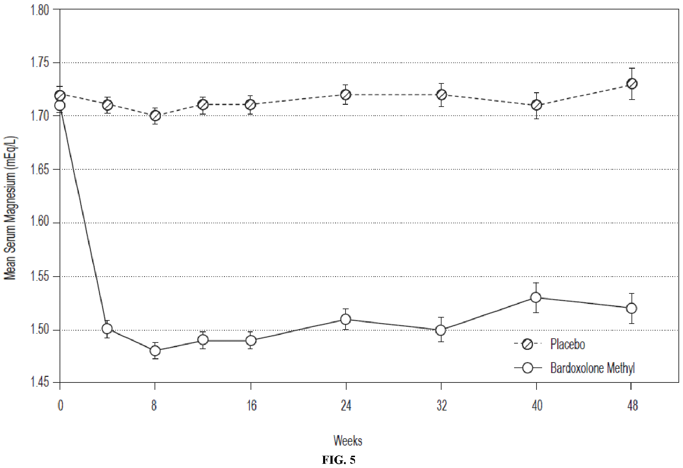

**FIG. 5** - Mean Serum Magnesium Levels in Bardoxolone Methyl vs. Placebo Patients in BEACON. Mean observed serum magnesium levels over time by treatment week in placebo vs. bardoxolone methyl patients. Only includes assessments of serum magnesium collected on or before a patient's last dose of study drug. Visits are derived relative to a patient's first dose of study drug. Data are mean ± SE. Top line is placebo; bottom line is bardoxolone methyl.

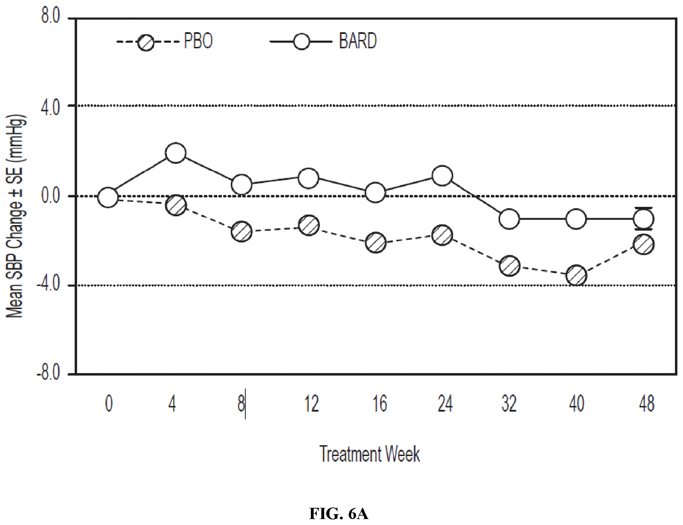

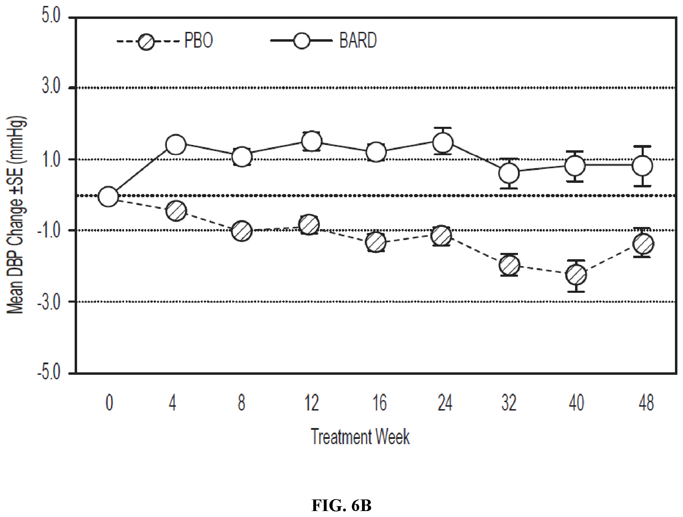

**FIGS. 6A-B** - Changes from Baseline over Time in Systolic Blood Pressure (SBP) (FIG. 6A) and Diastolic Blood Pressure (DBP) (FIG. 6B) in Bardoxolone Methyl (BARD) vs. Placebo (PBO) Patients in BEACON (Safety Population). Data includes only vital assessments collected on or before a patient's last dose of study drug. Visits are derived relative to a patient's first dose of study drug. Data are mean ± SE.

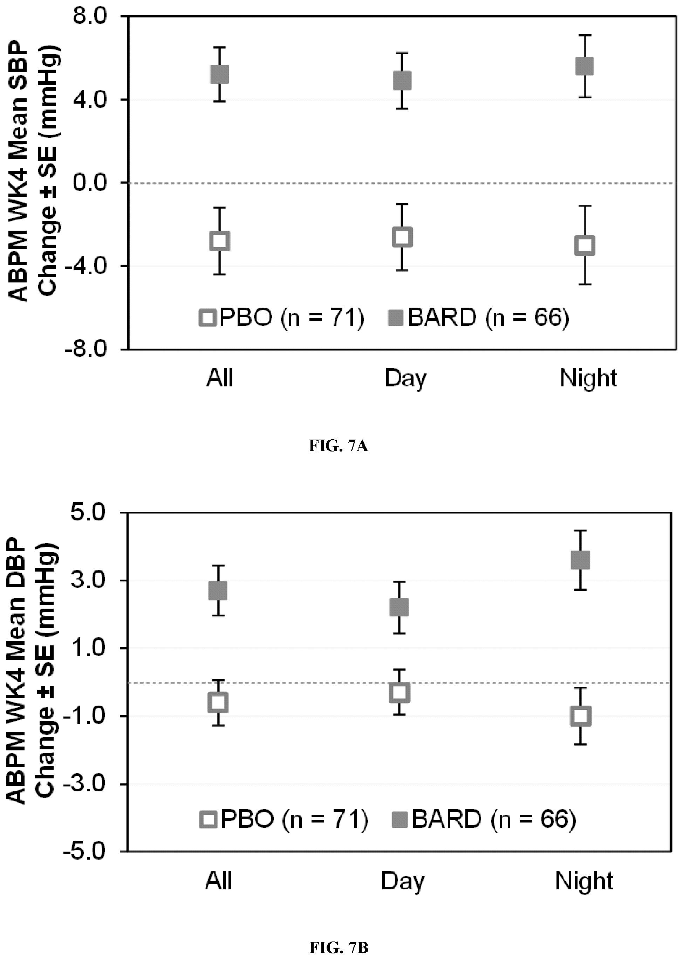

**FIGS. 7A-B** - Twenty-four-hour Ambulatory Blood Pressure Monitoring (ABPM) Sub-Study: Week 4 Changes from Baseline to Week 4 in Systolic Blood Pressure (SBP) (FIG. 7A) and Diastolic Blood Pressure (DBP) (FIG. 7B) in Bardoxolone Methyl (BARD) *vs.* Placebo (PBO) Patients. Data include only patients with baseline and Week 4 24-h ABPM values. Changes in systolic blood pressure are calculated using the averages of all valid measurements taken from a patient's ambulatory blood pressure monitoring device during the entire 24-h period, daytime (6 A.M. to 10 P.M.), or nighttime (10 P.M to 6 A.M. the next day). Data are mean ± SE.

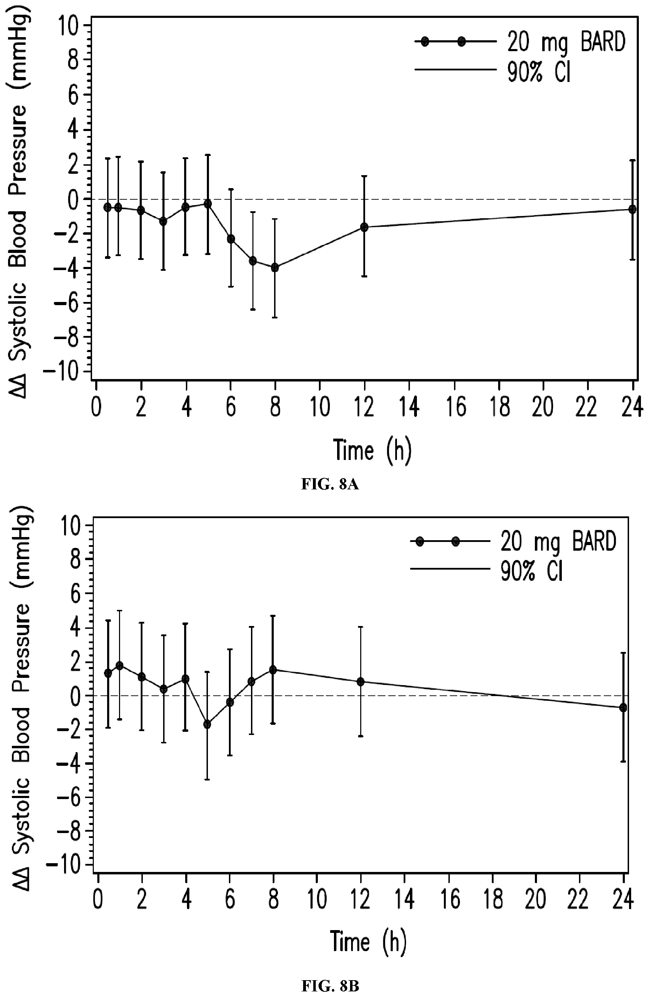

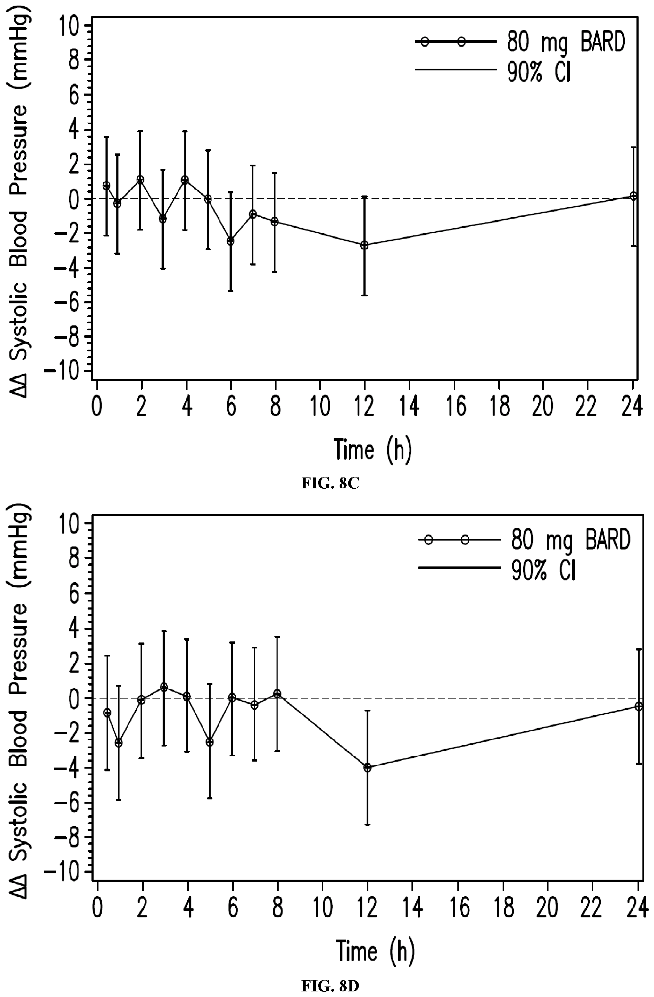

**FIGS. 8A-D** - Placebo-Corrected Changes from Baseline in Systolic Blood Pressure on Study Days 1 and 6 in Healthy Volunteers Administered Bardoxolone Methyl (BARD). Results from a multiple-dose, randomized, double-blind, placebo-controlled thorough QT study in healthy volunteers (RTA402-C-1006). Patients were treated with placebo, 20 mg or 80 mg of bardoxolone methyl, or 400 mg of moxifloxacin (active comparator) once daily for six consecutive days. Data are mean changes (± SD) from baseline 0-24 hours post-dose on Study Day 1 and Study Day 6. FIG. 8A shows dosing with 20 mg BARD on Study Day 1. FIG. 8B shows dosing with 20 mg BARD on Study Day 6. FIG. 8C shows dosing with 80 mg BARD on Study Day 1. FIG. 8D shows dosing with 80 mg BARD on Study Day 6.

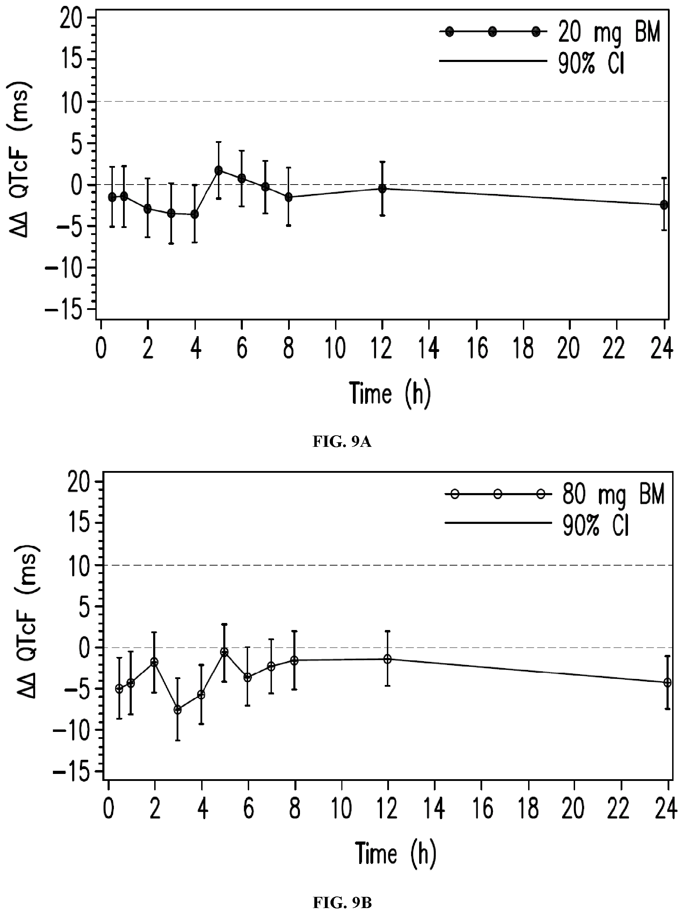

**FIGS. 9A-B** - Placebo-Corrected Changes from Baseline in QTcF in Healthy Volunteers Administered Bardoxolone Methyl (BM). Results from a multiple-dose, randomized, double-blind, placebo-controlled thorough QT study in healthy volunteers (RTA402-C-1006). QTcF interval changes in subjects administered bardoxolone methyl (20 mg or 80 mg - FIG. 9A and FIG. 9B, respectively) are shown relative to changes in patients receiving placebo treatment for six consecutive days. Data are mean values ± 90% CI, assessed 0-24 hours post-dose on Study Day 6, where the upper limit of the 90% CI is equivalent to the 1-sided, upper 95% confidence limit. The 10 ms threshold reference line is relevant to the upper confidence limits.

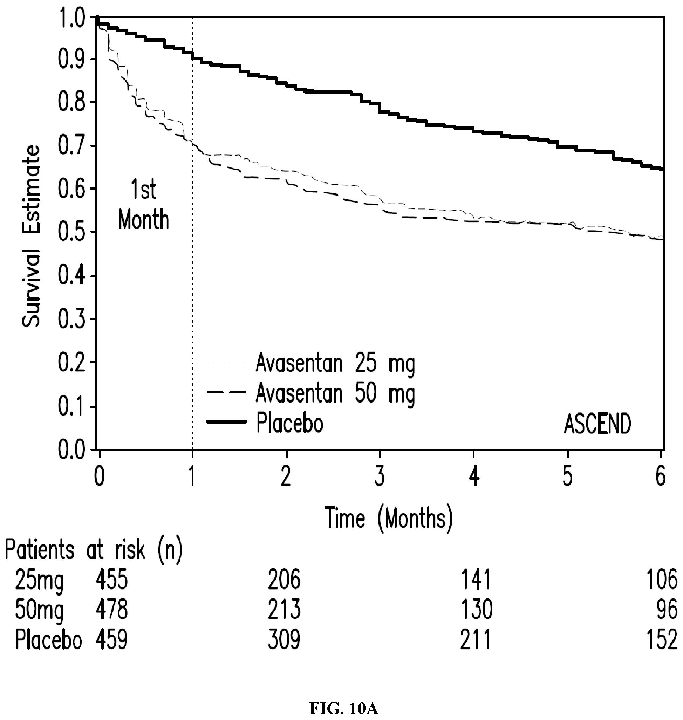

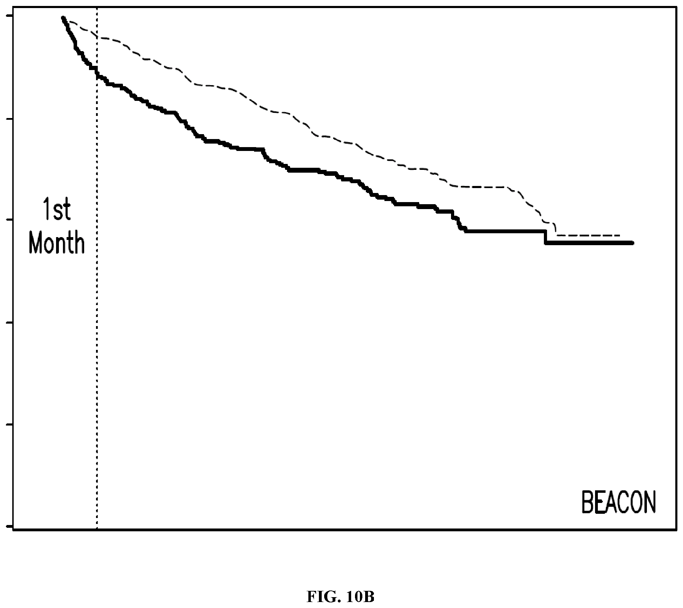

**FIGS. 10A-B** - Kaplan-Meier Plots for Fluid Overload Events in ASCEND (FIG. 10A) and Heart Failure Events in BEACON (ITT Population; FIG. 10B). Time-to-first event analysis for avosentan-induced fluid overload events in ASCEND and heart failure in BEACON. Avosentan-induced fluid overload events in ASCEND were taken from the adverse event reports of the local investigators. Individual signs and symptoms on the adverse event forms indicating fluid overload included: heart failure, edema, fluid overload, fluid retention, hypervolemia, dyspnea, pleural and pericardial effusions, ascites, weight increase, pulmonary rales, and pulmonary edema. Analysis includes only heart failure events occurring on or prior to study drug termination date (October 18, 2012) that were positively adjudicated by an independent Event Adjudication Committee, as outlined in the BEACON EAC Charter.

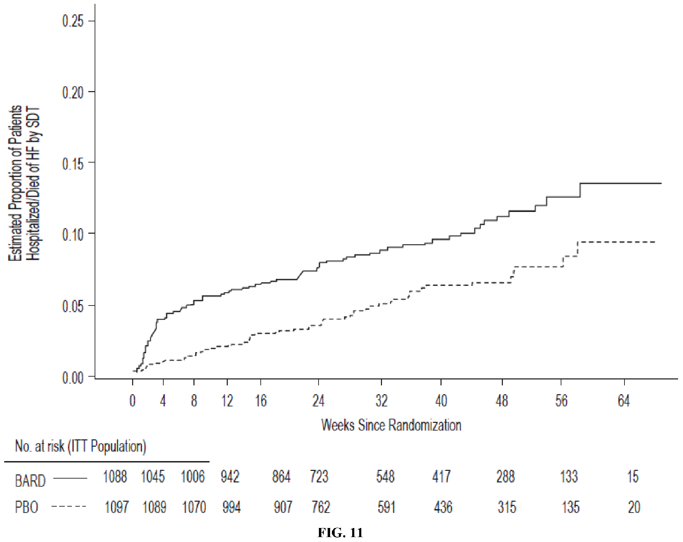

**FIG. 11** - Time to First Hospitalization for Heart Failure or Death Due to Heart Failure Event in BEACON (ITT Population). Analysis includes only heart failure (HF) events occurring on or prior to study drug termination date (SDT) (October 18, 2012) that were positively adjudicated by an independent Event Adjudication Committee, as outlined in the BEACON EAC Charter. Top line is bardoxolone methyl (BARD); bottom line is placebo (PBO).

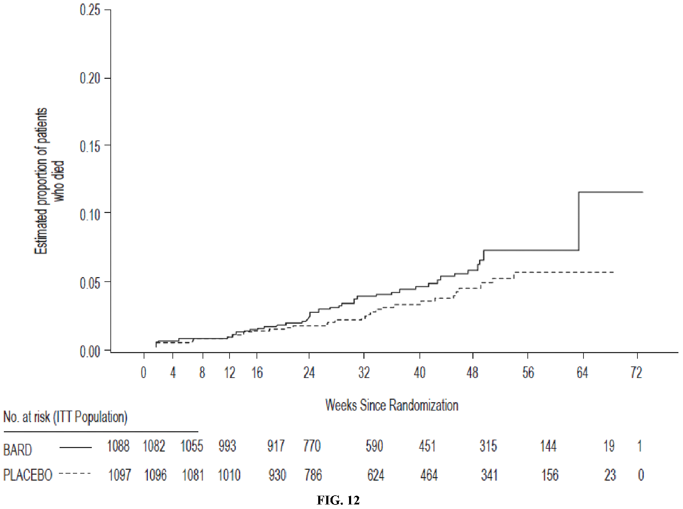

**FIG. 12** - Overall Survival of Bardoxolone Methyl vs. Placebo Patients in BEACON. Results from a randomized, double-blind, placebo-controlled phase 3 study in T2D patients with Stage 4 CKD (BEACON, RTA402-C-0903). Patients were administered placebo or 20 mg of bardoxolone methyl once daily. Analysis includes all deaths occurring prior to database lock (March 4, 2013). Top line is bardoxolone methyl (BARD); bottom line is placebo.

### DESCRIPTION OF ILLUSTRATIVE EMBODIMENTS

In one aspect, the present invention provides new methods for treating or preventing Alport syndrome or a symptom thereof or preventing the onset of symptoms of Alport syndrome in patients using bardoxolone methyl and analogs thereof. These and other aspects of the invention are described in greater detail below.

### I. Characteristics of Patients Who Should Be Excluded from Treatment with Bardoxolone Methyl

Several clinical studies have shown that treatment with bardoxolone methyl improved markers of renal function (including estimated glomerular filtration rate, or eGFR), insulin resistance, and endothelial dysfunction (Pergola *et al.,* 2011). These observations led to the initiation of a large Phase 3 trial (BEACON) of bardoxolone methyl in patients with stage 4 CKD and type 2 diabetes. The primary endpoint in the BEACON trial was a composite of progression to end-stage renal disease (ESRD) and all-cause mortality. This trial was terminated due to excess severe adverse events and mortality in the group of patients treated with bardoxolone methyl.

As discussed below, subsequent analysis of the data from the BEACON trial showed that most of the severe adverse events and mortality involved heart failure and were highly correlated with the presence of one or more risk factors including: (a) elevated baseline levels of B-type natriuretic peptide (BNP; *e.g.,* >200 pg/mL); (b) baseline eGFR <20; (c) history of left-sided heart disease; (d) high baseline albumin-to-creatinine ratio (ACR; *e.g.,* >300 mg/g as defined by dipstick proteinuria of 3+); and (e) advanced age *(e.g.,* >75 years). The analysis indicated that heart failure events were likely related to the development of acute fluid overload in the first three to four weeks of bardoxolone methyl treatment and that this was potentially due to inhibition of endothelin-1 signaling in the kidney. A previous trial of an endothelin receptor antagonist in stage 4 CKD patients was terminated due to a pattern of adverse events and mortality very similar to that found in the BEACON trial. Subsequent non-clinical studies confirmed that bardoxolone methyl, at physiologically relevant concentrations, inhibits endothelin-1 expression in renal proximal tubule epithelial cells and inhibits endothelin receptor expression in human mesangial and endothelial cells. Accordingly, patients at risk of adverse events from inhibition of endothelin signaling should be excluded from future clinical use of bardoxolone methyl.

The present invention concerns new methods of treating Alport syndrome that include modification of the glomerular basement membrane as a significant contributing factor. It also concerns the preparation of pharmaceutical compositions for the treatment of such disorders. In some embodiments of the present invention, patients for treatment are selected on the basis of several criteria: (1) diagnosis of a disorder that involves endothelial dysfunction as a significant contributing factor; (2) lack of elevated levels of B-type natriuretic peptide (BNP; *e.g.,* BNP titers must be <200 pg/mL); (3) lack of chronic kidney disease *(e.g.,* eGFR > 60) or lack of advanced chronic kidney disease *(e.g.,* eGFR > 45); (4) lack of a history of left-sided myocardial disease; and (5) lack of a high ACR *(e.g.,* ACR below 300 mg/g). In some embodiments of the invention, patients with a diagnosis of type 2 diabetes are excluded. In some embodiments of the invention, patients with a diagnosis of cancer are excluded. In some embodiments, patients of advanced age *(e.g.,* >75 years) are excluded. In some embodiments, patients are closely monitored for rapid weight gain suggestive of fluid overload. For example, patients may be instructed to weigh themselves daily for the first four weeks of treatment and contact the prescribing physician if increases of greater than five pounds are observed.

### A. BEACON Study

### 1. Design of Study

Study 402-C-0903, titled "Bardoxolone Methyl Evaluation in Patients with Chronic Kidney Disease and Type 2 Diabetes: The Occurrence of Renal Events" (BEACON) was a phase 3, randomized, double-blind, placebo-controlled, parallel-group, multinational, multicenter study designed to compare the efficacy and safety of bardoxolone methyl (BARD) to placebo (PBO) in patients with stage 4 chronic kidney disease and type 2 diabetes. A total of 2,185 patients were randomized 1:1 to once-daily administration of bardoxolone methyl (20 mg) or placebo. The primary efficacy endpoint of the study was the time-to-first event in the composite endpoint defined as end-stage renal disease (ESRD; need for chronic dialysis, renal transplantation, or renal death) or cardiovascular (CV) death. The study had three secondary efficacy endpoints: (1) change in estimated glomerular filtration rate (eGFR); (2) time-to-first hospitalization for heart failure or death due to heart failure; and (3) time-to-first event of the composite endpoint consisting of non-fatal myocardial infarction, non-fatal stroke, hospitalization for heart failure, or cardiovascular death.

A subset of the BEACON patients consented to additional 24-hour assessments including ambulatory blood pressure monitoring (ABPM) and 24-hour urine collections. An independent Events Adjudication Committee (EAC), blinded to study treatment assignment, evaluated whether renal events, cardiovascular events, and neurological events met the pre-specified definitions of the primary and secondary endpoints. An IDMC, consisting of external clinical experts supported by an independent statistical group, reviewed unblinded safety data throughout the study and made recommendations as appropriate.

### 2. Demographics and Baseline Characteristics of the Population

Table 1 presents summary statistics on select demographic and baseline characteristics of patients enrolled in BEACON. Demographic characteristics were comparable across the two treatment groups. In all treatment groups combined, the average age was 68.5 years and 57% of the patients were male. The bardoxolone methyl arm had slightly more patients in the age subgroup ≥75 years than the placebo arm (27% in bardoxolone methyl arm versus 24% in the placebo arm). Mean weight and BMI across both treatment groups was 95.2 kg and 33.8 kg/m², respectively. Baseline kidney function was generally similar in the two treatment groups; mean baseline eGFR, as measured by the 4-variable Modified Diet in Renal Disease (MDRD) equation, was 22.5 mL/min/1.73 m² and the geometric mean albumin/creatinine ratio (ACR) was 215.5 mg/g for the combined treatment groups.

**Table 1. Select Demographics and Baseline Characteristics of Bardoxolone Methyl (BARD) versus Placebo (PBO) Patients in BEACON (ITT Population)**

| | | **BARD** | **PBO** | **Total** |
|---|---|---|---|---|
| | | **N = 1088** | **N = 1097** | **N = 2185** |
| **Sex, n (%)** | | | | |
| | Male | 626 (58) | 625 (57) | 1251 (57) |
| | Female | 462 (42) | 472(43) | 934 (43) |
| **Age at informed consent (years)** | | | | |
| | n | 1088 | 1097 | 2185 |
| | Mean (SD) | 68.9 (9.7) | 68.2 (9.4) | 68.5 (9.6) |
| | Range (min, max) | 32, 92 | 29, 93 | 29, 93 |
| **Age subgroup, n (%)** | | | | |
| | <75 | 786 (72) | 829 (76) | 1615 (74) |
| | ≥75 | 302 (27) | 268 (24) | 570 (26) |
| **Weight (kg)** | | | | |
| | n | 1087 | 1097 | 2184 |
| | Mean (SD) | 95.1 (22.0) | 95.3 (21.1) | 95.2 (21.5) |
| | Range (min, max) | 46, 194 | 45, 186 | 45, 194 |
| **BMI (kg/m²)** | | | | |
| | n | 1087 | 1097 | 2184 |
| | Mean (SD) | 33.7 (7.1) | 33.9 (7.2) | 33.8 (7.1) |
| | Range (min, max) | 19, 93 | 19, 64 | 19, 93 |
| **eGFR (mL/min/1.73 m²) mean (SD)** | | | | |
| | n | 1088 | 1097 | 2185 |
| | Mean (SD) | 22.4 (4.3) | 22.5 (4.6) | 22.5 (4.5) |
| | Range (min, max) | 13, 34 | 13, 58 | 13, 58 |
| **eGFR MDRD subgroup, n (%)** | | | | |
| | 15 - <20 | 325 (30) | 347 (32) | 672 (31) |
| | 20 - <25 | 399 (37) | 366 (33) | 765 (35) |
| | 25 - <30 | 311 (29) | 318 (29) | 629 (29) |
| **ACR (mg/g) geometric mean** | | | | |
| | n | 1088 | 1097 | 2185 |
| | Geometric mean | 210.4 | 220.7 | 215.5 |
| | (95% CI) | (188, 236) | (196, 249) | (198, 234) |
| | Range (min, max) | <1, 4581 | <1, 79466 | <1, 79466 |
| **ACR subgroup, n (%)** | | | | |
| | <30 | 200 (18) | 211 (19) | 411 (19) |
| | 30-300 | 348 (32) | 308 (28) | 656 (30) |
| | >300 | 540 (50) | 578 (53) | 1118 (51) |

| | | | | |
|---|---|---|---|---|
| Patients were administered placebo or 20 mg of bardoxolone methyl once daily. | | | | |

### B. BEACON Results

### 1. Effect of Bardoxolone Methyl on eGFR

The mean eGFR values for bardoxolone methyl-treated and placebo-treated patients are shown in FIG. 2. On average, bardoxolone methyl patients had expected increases in eGFR that occurred by Week 4 of treatment and remained above baseline through Week 48. In contrast, placebo-treated patients on average had unchanged or slight decreases from baseline. The proportion of patients with eGFR declines was markedly reduced in bardoxolone methyl- versus placebo-treated patients (FIGS. 3A-B). The eGFR trajectories and the proportions of decliners observed in BEACON after one year of treatment were consistent with modeled expectations and results from the BEAM study (RTA402-C-0804). As shown in Table 2, the number of patients who experienced a renal and urinary disorder serious adverse event (SAE) was lower in the bardoxolone methyl group than in the placebo group (52 *vs.* 71, respectively). Additionally, and as discussed in the following section, slightly fewer ESRD events were observed in the bardoxolone methyl group than in the placebo group. Collectively, these data suggest that bardoxolone methyl treatment did not worsen renal status acutely or over time.

**Table 2. Incidence of Treatment-Emergent Serious Adverse Events in BEACON within Each Primary System Organ Class (Safety Population)**

| | **Placebo** | **Bardoxolone methyl** |
|---|---|---|
| **MedDRA System Organ Class** | **N = 1093** | **N = 1092** |
| | **n (%)** | **n (%)** |
| Patients with any serious adverse event | 295 (27) | 363 (33) |
| Number of serious adverse events | 557 | 717 |
| | | |
| Cardiac disorders | 84 (8) | 124 (11) |
| Infections and infestations | 63 (6) | 79 (7) |
| Renal and urinary disorders | 71 (6) | 52 (5) |
| Metabolism and nutrition disorders | 42 (4) | 51 (5) |
| Gastrointestinal disorders | 39 (4) | 46 (4) |
| Respiratory, thoracic and mediastinal disorders | 32 (3) | 43 (4) |
| Nervous system disorders | 35 (3) | 37 (3) |
| General disorders and administration site conditions | 20 (2) | 29 (3) |
| Vascular disorders | 18 (2) | 20 (2) |
| Injury, poisoning and procedural complications | 17 (2) | 19 (2) |
| Musculoskeletal and connective tissue disorders | 13 (1) | 21 (2) |
| Blood and lymphatic system disorders | 11 (1) | 20 (2) |
| Neoplasms benign, malignant and unspecified (incl. cysts and polyps) | 10 (1) | 11 (1) |
| Hepatobiliary disorders | 8 (1) | 4 (<1) |
| Psychiatric disorders | 3 (<1) | 3 (<1) |
| Eye disorders | 2 (<1) | 3 (<1) |
| Investigations | 2 (<1) | 3 (<1) |
| Reproductive system and breast disorders | 3 (<1) | 2 (<1) |
| Skin and subcutaneous tissue disorders | 1 (<1) | 4 (<1) |
| Ear and labyrinth disorders | 1 (<1) | 3 (<1) |
| Endocrine disorders | 1 (<1) | 1 (<1) |
| Immune system disorders | 0 | 2 (<1) |
| Surgical and medical procedures | 0 | 2 (<1) |

| | | |
|---|---|---|
| Table includes only serious adverse events with onset more than 30 days after a patient's last dose of study drug. Column header counts and denominators are the number of patients in the safety population. Each patient is counted at most once in each System Organ Class and Preferred Term. | | |

### 2. Primary Composite Outcome in BEACON

Table 3 provides a summary of adjudicated primary endpoints that occurred on or before the date of study termination (October 18, 2012). Despite the slight reduction in the number of ESRD events in the bardoxolone methyl vs. placebo treatment groups, the number of composite primary endpoints was equal in the two treatment groups (HR = 0.98) due to a slight increase in cardiovascular death events, as depicted in plots of time-to-first composite primary event analysis (FIG. 4).

**Table 3. Adjudicated Primary Endpoints in Bardoxolone Methyl (BARD) vs. Placebo (PBO) Patients in BEACON (ITT Population)**

| | | **PBO** | **BARD** | **Hazard ratio (95% CI)^{a}** | **p-value^{b}** |
|---|---|---|---|---|---|
| | | **N = 1097** | **N = 1088** | | |
| | | **n (%)** | **n (%)** | | |
| **Composite primary efficacy outcome** | | 69 (6) | 69 (6) | 0.98 (0.70, 1.37) | 0.92 |
| **Patient's first event** | | | | | |
| | End stage renal disease (ESRD) | 51 (5) | 43 (4) | | |
| | Chronic dialysis | 47 (4) | 40 (4) | | |
| | Renal transplant | 3 (<1) | 1 (<1) | | |
| | Renal death | 1 (<1) | 2 (<1) | | |
| | CV death | 18 (2) | 26 (2) | | |

| | | | | | |
|---|---|---|---|---|---|
| ^{a} Hazard ratio (bardoxolone methyl/placebo) and 95% confidence interval (CI) were estimated using a Cox proportional hazards model with treatment group, continuous baseline eGFR, and continuous baseline log ACR as covariates. Breslow's method of handling ties in event time was used. ^{b} Treatment group comparisons used SAS's Type 3 chi-square test and two-sided p-value associated with the treatment group variable in the Cox proportional hazards model. | | | | | |

### C. Effects of Bardoxolone Methyl on Heart Failure and Blood Pressure

### 1. Adjudicated Heart Failure in BEACON

The data in Table 4 present a post-hoc analysis of demographic and select laboratory parameters of BEACON patients stratified by treatment group and occurrence of an adjudicated heart failure event. The number of patients with heart failure includes all events through last date of contact (ITT Population).

Comparison of baseline characteristics of patients with adjudicated heart failure events revealed that both bardoxolone methyl-treated and placebo-treated patients with heart failure were more likely to have had a prior history of cardiovascular disease and heart failure and had higher baseline values for B-type natriuretic peptide (BNP) and QTc interval with Fredericia correction (QTcF). Even though the risk for heart failure was higher in the bardoxolone methyl-treated patients, these data suggest that development of heart failure in both groups appeared to be associated with traditional risk factors for heart failure. Baseline ACR was significantly higher in bardoxolone methyl-treated patients with heart failure events than those without. Also of note, the mean baseline level of BNP in patients who experienced heart failure in both treatment groups was meaningfully elevated and suggested that these patients were likely retaining fluid and in sub-clinical heart failure prior to randomization.

**Table 4. Select Demographic and Baseline Characteristics for Bardoxolone Methyl vs. Placebo Patients Stratified by Heart Failure Status**

| **Patients** | **With Heart Failure** | | **Without Heart Failure** | | **Total** | |
|---|---|---|---|---|---|---|
| | **BARD** (N = 103) | **PBO** (N = 57) | **BARD** (N = 985) | **PBO** (N = 1040) | **BARD** (N = 1088) | **PBO** (N = 1097) |
| **Age (years),** Mean ± SD | 70.3 ± 9 | 69.2 ± 8.2 | 68.7 ± 9.8 | 68.1 ± 9.5 | 68.9 ± 9.7 | 68.2 ± 9.4 |
| **History of CVD,** N (%) | 80 (78)^{a} | 47 (82)^{b} | 529 (54) | 572 (55) | 609 (56) | 619 (56) |
| **History of HF,** N (%) | 36 (35)^{a} | 21 (37)^{b} | 130 (13) | 133 (13) | 166 (15) | 154 (14) |
| **History of MI,** N (%) | 33 (32)^{a} | 22 (39)^{b} | 185 (19) | 188 (18) | 218 (20) | 210 (19) |
| **History of A-FIB,** N (%) | 4 (4) | 3 (5) | 46 (5) | 40 (4) | 50 (5) | 43 (4) |
| **Concomitant Med Use,** N (%) | | | | | | |
| ACEi/ARB | 35 (34)^{a} | 16 (28)^{b} | 659 (67) | 701 (67) | 694 (64) | 717 (65) |
| Diuretic | 39 (38)^{a} | 15 (26)^{b} | 528 (54) | 586 (56) | 567 (52) | 601 (55) |
| Beta-Blocker | 38 (37)^{a} | 23 (40) | 482 (49) | 506 (49) | 520 (48) | 529 (48) |
| Statin | 57 (55) | 26 (46)^{b} | 640 (65) | 721 (69) | 697 (64) | 747 (68) |
| Calcium Channel Blocker | 25 (24)^{a} | 17 (30)^{b} | 406 (41) | 467 (45) | 431 (40) | 484 (44) |
| **eGFR (mL/min/1.73 m²),** | 21.7 ± 4.6 | 22.2 ± 4.7 | 22.5 ± 4.2 | 22.5 ± 4.6 | 22.4 ± 4.3 | 22.5 ± 4.6 |
| Mean ± SD | | | | | | |
| **ACR (mg/g),** Geo Mean | 353.9^{a} | 302.0 | 199.3 | 216.9 | 210.4 | 220.7 |
| **SBP (mmHg),** Mean ± SD | 139.5 ± 13.3 | 142.3 ± 11.2 | 139.5 ± 11.6 | 139.6 ± 11.8 | 139.5 ± 11.7 | 139.8 ± 11.8 |
| **DBP (mmHg),** Mean ± SD | 66.4 ± 9.1^{a} | 69.1 ± 8.8 | 70.4 ± 8.7 | 70.8 ± 8.6 | 70.1 ± 8.8 | 70.7 ± 8.7 |
| **BNP (pg/mL)** | | | | | | |
| Mean ± SD | 526.0 ± 549.4^{a} | 429.8 ± 434.3^{b} | 223.1 ± 257.5 | 232.3 ± 347.1 | 251.2 ± 309.1 | 242.7 ± 354.7 |
| >100, N (%) | 78 (76)^{a} | 43 (75)^{b} | 547 (56) | 544 (52) | 625 (57) | 587 (54) |
| **QTcF (ms)** | | | | | | |
| Mean ± SD | 447.9 ± 31.2^{a,c} | 432.5 ± 27.6^{b} | 425.3 ± 27.8 | 424.7 ± 27.9 | 427.4 ± 28.9 | 425.1 ± 28 |
| >450, N (%) | 40 (39)^{a} | 14 (25) | 170 (17) | 167 (16) | 210 (19) | 181 (16) |

| | | | | | | |
|---|---|---|---|---|---|---|
| a *p* < 0.05 for BARD patients with HF vs. BARD patients without HF b *p* < 0.05 for PBO patients with HF *vs.* PBO patients without HF c *p* < 0.05 for BARD vs. PBO patients with HF | | | | | | |

### 2. Assessment of Clinical Parameters Associated with BNP Increases

As a surrogate of fluid retention, a post-hoc analysis was performed on a subset of patients for whom BNP data were available at baseline and Week 24. Patients in the bardoxolone methyl arm experienced a significantly greater increase in BNP than patients in the placebo arm (Mean ± SD: 225 ± 598 *vs.* 34 ± 209 pg/mL, *p* < 0.01). Also noted was a higher proportion of bardoxolone methyl- vs. placebo-treated patients with increases in BNP at Week 24 (Table 5).

BNP increases at Week 24 did not appear to be related to baseline BNP, baseline eGFR, changes in eGFR, or changes in ACR. However, in bardoxolone methyl-treated patients only, baseline ACR was significantly correlated with Week 24 changes from baseline in BNP, suggesting that the propensity for fluid retention may be associated with baseline severity of renal dysfunction, as defined by albuminuria status, and not with the general changes in renal function, as assessed by eGFR (Table 6).

Further, these data suggest that increases in eGFR, which are glomerular in origin, are distinct anatomically, as sodium and water regulation occurs in the renal tubules.

**Table 5. Analysis of BNP and eGFR Values of Bardoxolone Methyl vs. Placebo Patients Stratified by Changes from Baseline in BNP at Week 24**

| **WK24 BNP Change** | **Treatment** | **N** | **Median BL BNP** | **Mean BL eGFR** | **Mean WK24 ΔeGFR** |
|---|---|---|---|---|---|
| **<25%** | PBO | 131 | 119.0 | 23.5 | -0.6 |
| **Increase** | BARD | 84 | 187.0 | 22.3 | 6.1 |
| **25% to 100%** | PBO | 48 | 102.5 | 22.0 | 0.4 |
| **Increase** | BARD | 45 | 119.0 | 22.7 | 5.5 |
| **≥100%** | PBO | 37 | 143.5 | 23.1 | 0.1 |
| **Increase** | BARD | 82 | 155.0 | 21.9 | 7.6 |

| | | | | | |
|---|---|---|---|---|---|
| Post-hoc analysis of changes in BNP in BEACON at Week 24. | | | | | |

**Table 6. Correlations between Changes from Baseline in BNP at Week 24 and Baseline ACR in Bardoxolone Methyl vs. Placebo Patients in BEACON**

| **Treatment** | **N** | **Correlation Coefficient** | **P-value** |
|---|---|---|---|
| **PBO** | 216 | 0.05 | 0.5 |
| **BARD** | 211 | 0.20 | <0.01 |

| | | | |
|---|---|---|---|
| Post-hoc analysis of changes in BNP in BEACON at Week 24. Only patients with baseline and Week 24 BNP values included in analysis. | | | |

### 3. Serum Electrolytes

No clinically meaningful changes were noted in serum potassium or serum sodium for the subset of patients with 24-hr urine collections (Table 7). The change in serum magnesium levels in bardoxolone methyl-treated patients was consistent with changes observed in prior studies (FIG. 5).

**Table 7. Week 4 Changes from Baseline in Serum Electrolytes in Bardoxolone Methyl vs. Placebo 24-hour ABPM Sub-Study Patients**

| | | **Serum Potassium (mmol/L)** | | | **Serum Sodium (mmol/L)** | | | **Serum Magnesium (mEq/L)** | | |
|---|---|---|---|---|---|---|---|---|---|---|
| | | **BL** | **WK4** | **WK4 Δ** | **BL** | **WK4** | **WK4 Δ** | **BL** | **WK4** | **WK4 Δ** |
| **PBO** | n | 88 | 87 | 87 | 88 | 87 | 87 | 88 | 87 | 87 |
| | Mean ± SE | 4.8 ± 0.1 | 4.7 ± 0.1 | -0.10 + 0.04* | 140.2 ± 0.2 | 139.7 ± 0.3 | -0.3 ± 0.2 | 1.72 ± 0.03 | 1.69 ± 0.03 | -0.03 ± 0.02 |
| **BARD** | n | 83 | 77 | 77 | 83 | 77 | 77 | 83 | 77 | 77 |
| | Mean ± SE | 4.7 ± 0.1 | 4.8 ± 0.1 | 0.10 + 0.05*^{†} | 140.1 ± 0.3 | 140.3 ± 0.3 | 0.2 ± 0.3 | 1.74 ± 0.02 | 1.53 ± 0.03 | -0.21 ± 0.02*^{†} |

| | | | | | | | | | | |
|---|---|---|---|---|---|---|---|---|---|---|
| Data include only BEACON patients enrolled in the 24-hour ABPM sub-study. Changes in serum electrolyte values only calculated for patients with baseline and Week 4 data. * *p <* 0.05 for Week 4 versus baseline values within each treatment group; † *p <* 0.05 for Week 4 changes in BARD vs. PBO patients. | | | | | | | | | | |

### 4. 24-hour Urine Collections

A subset of patients consented to additional 24-hr assessments (sub-study) of ambulatory blood pressure monitoring (ABPM) and 24-hr urine collection at selected visits. Urinary sodium excretion data from BEACON sub-study patients revealed a clinically meaningful reduction in urine volume and excretion of sodium at Week 4 relative to baseline in the bardoxolone methyl-treated patients (Table 8). These decreases were significantly different from Week 4 changes in urine volume and urinary sodium observed in placebo-treated patients. Also of note, reductions in serum magnesium were not associated with renal loss of magnesium.

Additionally, in a pharmacokinetic study in patients with type 2 diabetes and stage 3b/4 CKD administered bardoxolone methyl for eight weeks (402-C-1102), patients with stage 4 CKD had significantly greater reductions of urinary sodium and water excretion than stage 3b CKD patients (Table 9).

**Table 8. Week 4 Changes from Baseline in 24-hour Urine Volume, Urinary Sodium, and Urinary Potassium in Bardoxolone Methyl vs. Placebo 24-hour ABPM Sub-Study Patients**

| | | **Urine Volume (mL)** | | | **Urinary Sodium (mmol/24 h)** | | | **Urinary Potassium (mmol/24 h)** | | | **Urinary Magnesium (mmol/24 h)** | | |
|---|---|---|---|---|---|---|---|---|---|---|---|---|---|
| | | BL | WK4 | WK4 Δ | BL | WK4 | WK4 Δ | BL | WK4 | WK4 Δ | BL | WK4 | WK4 Δ |
| **PBO** | n | 87 | 72 | 71 | 81 | 68 | 62 | 81 | 68 | 62 | 59 | 53 | 46 |
| | Mean ± SE | 2053 ± 82 | 1928 ± 89 | -110 ± 71 | 160 ± 8 | 145 ± 8 | -11 ± 9 | 55 ± 3 | 52 ± 3 | -3 ± 3 | 7.5 ± 0.5 | 6.0 ± 0.5 | -0.6 ± 0.4 |
| **BARD** | n | 82 | 64 | 63 | 77 | 61 | 57 | 77 | 61 | 57 | 56 | 43 | 40 |
| | Mean ± SE | 2024 ± 83 | 1792 ± 84 | -247 ± 71* | 164 ± 9 | 140 ± 9 | -27 ± 9* | 60 ± 3 | 52 ± 2 | -7 ± 3* | 7.0 ± 0.4 | 6.0 ± 0.4 | -0.9 ± 0.5 |

| | | | | | | | | | | | | | |
|---|---|---|---|---|---|---|---|---|---|---|---|---|---|
| Data include only BEACON patients enrolled in the 24-hour ABPM sub-study. Changes at Week 4 only calculated for patients with baseline and Week 4 data. * *p* < 0.05 for Week 4 versus baseline values within each treatment group; † *p <* 0.05 for Week 4 changes in BARD versus PBO patients. | | | | | | | | | | | | | |

**Table 9. Week 8 Changes from Baseline in 24-h Urine Volume and 24-h Urinary Sodium Bardoxolone Methyl-treated Patients Grouped by CKD Severity (from a Patient Pharmacokinetic Study)**

| **CKD Stage** | | **Urine Volume (mL)** | | **Urinary Sodium (mmol/24 h)** | |
|---|---|---|---|---|---|
| | N | WK8 Δ | p-value | WK8 Δ | p-value |
| Stage 3b | 9 | 355 | 0.04 | -12 | 0.02 |
| Stage 4 | 6 | -610 | | -89 | |

| | | | | | |
|---|---|---|---|---|---|
| Patients were treated with 20 mg bardoxolone methyl once daily for 56 consecutive days; post-treatment follow-up visit occurred on Study Day 84. Data are means. Data include patients with baseline and Week 8 data. | | | | | |

### 5. Hospital Records from EAC Adjudication Packets

The first scheduled post-baseline assessment in BEACON was at Week 4. Since many of the heart failure events occurred prior to Week 4, the clinical database provides limited information to characterize these patients. Post-hoc review of the EAC case packets for heart failure cases that occurred prior to Week 4 was performed to assess clinical, vitals, laboratory, and imaging data collected at the time of the first heart failure event (Tables 10 and 11).

Examination of these records revealed common reports of rapid weight gain immediately after randomization, dyspnea and orthopnea, peripheral edema, central/pulmonary edema on imaging, elevated blood pressure and heart rate, and preserved ejection fraction. The data suggest that heart failure was caused by rapid fluid retention concurrent with preserved ejection fraction and elevated blood pressure. The preserved ejection fraction is consistent with clinical characteristics of heart failure caused by diastolic dysfunction stemming from ventricular stiffening and impaired diastolic relaxation. This collection of signs and symptoms differs in clinical characteristics from heart failure with reduced ejection fraction, which occurs because of weakened cardiac pump function or contractile impairment (Vasan *et al.,* 1999). Therefore, rapid fluid accumulation in patients with stuff ventricles and minimal renal reserve likely resulted in increased fluid back-up into the lungs and the noted clinical presentation.

Baseline central laboratory values from the clinical database were compared to local laboratory values obtained on admission for heart failure that were included in the EAC packets. Unchanged serum creatinine, sodium, and potassium concentrations in bardoxolone methyl-treated patients with heart failure events that occurred within the first four weeks after randomization (Table 11) suggest that heart failure was not associated with acute renal function decline or acute kidney injury. Overall, the clinical data suggest that the etiology of heart failure is not caused by a direct renal or cardiotoxic effect, but is more likely to be due to sodium and fluid retention.

**Table 10. Post-Hoc Analysis of Cardiovascular Parameters of Bardoxolone Methyl vs. Placebo Patients with Heart Failure Events Occurring Within First Four Weeks of Treatment**

| | | **LVEF** | **SBP (mmHg)** | | | **DBP (mmHg)** | | | **Heart Rate (bpm)** | | |
|---|---|---|---|---|---|---|---|---|---|---|---|
| | | **HF** | **BL** | **HF** | **Δ** | **BL** | **HF** | **Δ** | **BL** | **HF** | **Δ** |
| **PBO** | n | 4 | 8 | 6 | 6 | 8 | 6 | 6 | 8 | 5 | 5 |
| | Mean ± SE | 49% ± 6% | 141 ± 5 | 148 ± 11 | 4.7 ± 7.2 | 65 ± 3 | 65 ± 5 | 1.2 ± 3.6 | 70 ± 3 | 65 ± 3 | -3.6 ± 2.9 |
| **BARD** | n | 23 | 42 | 33 | 33 | 42 | 34 | 34 | 42 | 32 | 32 |
| | Mean ± SE | 52% ± 2% | 142 ± 2 | 154 ± 4 | 10.5 ± 3.1 | 67 ± 2 | 75 ± 2 | 7.9 ± 2.1 | 67 ± 1 | 81 ± 3 | 14.5 ± 2.7 |

| | | | | | | | | | | | |
|---|---|---|---|---|---|---|---|---|---|---|---|
| Post-hoc analyses of heart failure cases in BEACON. Vital signs at baseline calculated from the average of three standard cuff measurements. Vital signs from HF hospitalization gathered from admission notes included in EAC Adjudication packets and represent singular assessments using different BP monitoring equipment. LVEF only assessed during HF hospitalization. Timing of HF admission calculated from event start date and treatment start date and varied from Weeks 0-4 for each patient. | | | | | | | | | | | |

**Table 11. Post-Hoc Analysis of Serum Electrolytes of Bardoxolone Methyl vs. Placebo Patients with Heart Failure Events Occurring Within First Four Weeks of Treatment**

| | | **Serum Creatinine (mg/dL)** | | | **Serum Sodium (mmol/L)** | | | **Serum Potassium (mmol/L)** | | |
|---|---|---|---|---|---|---|---|---|---|---|
| | | **BL** | **HF** | **Δ** | **BL** | **HF** | **Δ** | **BL** | **HF** | **Δ** |
| **PBO** | n | 8 | 8 | 8 | 8 | 8 | 8 | 8 | 8 | 8 |
| | Mean ± SE | 3.4 ± 0.2 | 3.3 ± 0.2 | -0.1 ± 0.2 | 140.0 ± 1.0 | 137.0 ± 1.0 | -2.5 ± 0.6 | 4.5 ± 0.2 | 4.4 ± 0.1 | -0.1 ± 0.2 |
| **BARD** | n | 42 | 38 | 38 | 42 | 30 | 30 | 42 | 34 | 34 |
| | Mean ± SE | 2.8 ± 0.1 | 2.7 ± 0.1 | -0.1 ± 0.1 | 140.0 ± 0.0 | 139.0 ± 1.0 | -1.0 ± 0.5 | 4.7 ± 0.1 | 4.8 ± 0.1 | 0.1 ± 0.1 |

| | | | | | | | | | | |
|---|---|---|---|---|---|---|---|---|---|---|
| Post-hoc analyses of heart failure cases in BEACON. Baseline clinical chemistries assessed at central laboratory. Clinical chemistries from HF hospitalization gathered from hospital notes included in EAC Adjudication packets and represent assessments made at different local laboratories. | | | | | | | | | | |

### 6. Blood Pressure in BEACON

Mean changes from baseline in systolic and diastolic blood pressures for bardoxolone methyl-treated and placebo-treated patients, based on the average of triplicate standardized blood pressure cuff measurements collected at each visit, are shown in FIGS. 6A-B. Blood pressure was increased in the bardoxolone methyl group relative to the placebo group, with mean increases of 1.9 mmHg in systolic and 1.4 mmHg in diastolic blood pressures noted in the bardoxolone methyl group by Week 4 (the first post-randomization assessment). The increases in systolic blood pressure (SBP) appeared to diminish by Week 32, while diastolic blood pressure (DBP) increases were sustained.

The Week 4 SBP and DBP increases in bardoxolone methyl-treated patients relative to placebo-treated patients were more apparent in the ABPM measurements (FIGS. 7A-B). This difference in magnitude could be due to the different techniques that were used or to differences in baseline characteristics in the ABPM sub-study patients. Patients in the ABPM sub-study had a higher baseline ACR than the entire population. Regardless, the data demonstrate that bardoxolone methyl increased blood pressure in the BEACON patient population.

### 7. Blood Pressure Changes in Prior CKD Studies

In an open label, dose-ranging study in type 2 diabetic patients with stage 3b-4 CKD (402-C-0902), no dose-related trend in blood pressure changes or change at any individual dose level was noted following 85 consecutive days of treatment at doses ranging from 2.5 to 30 mg of bardoxolone methyl (amorphous dispersion formulation, as used in BEACON). Post-hoc analysis of blood pressure data stratified by CKD stage suggests that bardoxolone methyl-treated patients with stage 4 CKD tended to have increases in blood pressure relative to baseline levels, with the effect most appreciable in the three highest dose groups, whereas bardoxolone methyl-treated patients with stage 3b CKD had no apparent change (Table 12). Although sample sizes in the dose groups stratified by CKD stage are small, these data suggest that the effect of bardoxolone methyl treatment on blood pressure may be related to CKD stage.

Blood pressure values from a phase 2b study with bardoxolone methyl (BEAM, 402-C-0804), which used an earlier crystalline formulation of the drug and employed a titration design, were highly variable and despite noted increases in some bardoxolone methyl treatment groups, no clear dose-related trend was observed in blood pressure.

**Table 12. Changes from Baseline in Systolic and Diastolic Blood Pressure in Patients with Type 2 Diabetes and Stage 3b-4 CKD Stratified by Baseline CKD Stage Dosed with Bardoxolone Methyl**

| **Dose (mg)** | **CKD Stage** | **N** | **ΔSBP** | **ΔDBP** |
|---|---|---|---|---|
| 2.5 | 3b/4 | 14 | 0.1 ± 4.2 | 0.2 ± 1.8 |
| | 3b | 10 | 0 ± 4.4 | 1 ± 2 |
| | 4 | 4 | 0.3 ± 11 | -1.5 ± 3.9 |
| 5 | 3b/4 | 24 | -1.5 ± 2.3 | -1.4 ± 1.5 |
| | 3b | 19 | -2.1 ± 2 | -1.3 ± 1.4 |
| | 4 | 5 | 0.5 ± 9.1 | -1.4 ± 5.6 |
| 10 | 3b/4 | 24 | -2.4 ± 3.1 | 0.3 ± 1.3 |
| | 3b | 20 | -4.2± 3.4 | -0.3 ± 1.3 |
| | 4 | 4 | 6.1 ± 6.7 | 3.6 ± 4.5 |
| 15 | 3b/4 | 48 | 1.1 ± 2.3 | -1 ± 1.2 |
| | 3b | 26 | -2.2 ± 3.3 | -1.3 ± 1.5 |
| | 4 | 22 | 5 ± 2.8 | -0.6 ± 1.9 |
| 30 | 3b/4 | 12 | 7.2 ± 6.2 | 3.2 ± 2.2 |
| | 3b | 3 | -0.4 ± 13.8 | -1.8 ± 3.9 |
| | 4 | 9 | 9.7 ± 7.3 | 4.7 ± 2.5 |

| | | | | |
|---|---|---|---|---|
| Patients were administered 2.5, 5, 10, 15, or 30 mg doses of bardoxolone methyl once daily for 85 days. | | | | |

### 8. Blood Pressure and QTcF in Healthy Volunteers

Intensive blood pressure monitoring was employed in a separate Thorough QT Study, which was conducted in healthy volunteers. In both bardoxolone methyl-treated groups, one given the therapeutic dose, 20 mg, which was also studied in BEACON, and one given the supratherapeutic dose of 80 mg, the change in blood pressure did not differ from changes observed in placebo-treated patients (FIGS. 8A-D) after 6 days of once daily administration. Bardoxolone methyl did not increase QTcF as assessed by placebo-corrected QTcF changes (ΔΔQTcF) after 6 days of treatment at 20 or 80 mg (FIGS. 9A-B).

Bardoxolone methyl has also been tested in non-CKD disease settings. In early clinical studies of bardoxolone methyl in oncology patients (RTA 402-C-0501, RTA 402-C-0702), after 21 consecutive days of treatment at doses that ranged from 5 to 1300 mg/day (crystalline formulation), no mean change in blood pressure was observed across all treatment groups. Similarly, in a randomized, placebo-controlled study in patients with hepatic dysfunction (RTA 402-C-0701), 14 consecutive days of bardoxolone methyl treatment at doses of 5 and 25 mg/day (crystalline formulation) resulted in mean decreases in systolic and diastolic blood pressure (Table 13).

Collectively, these data suggest that bardoxolone methyl does not prolong the QT interval and does not cause blood pressure increases in patients who do not have baseline cardiovascular morbidity or stage 4 CKD.

**Table 13. Changes from Baseline in Blood Pressure in Patients with Hepatic Dysfunction Treated with Bardoxolone Methyl**

| | | **Mean ΔSBP** ± **SE (mmHg)** | | **Mean ΔDBP ± SE (mmHg)** | |
|---|---|---|---|---|---|
| **Dose** | N | D7 | D14 | D7 | D14 |
| **PBO** | 4 | -10 ± 8.5 | -1.3 ± 5.5 | -4.0 ± 2.0 | 0.0 ± 3.1 |
| **5 mg** | 6 | -12.8 ± 5.2 | -8.8 ± 5.1 | -2.0 ± 2.3 | -1.7 ± 3.2 |
| **25 mg** | 6 | -11.5 ± 5.2 | -1.2 ± 3.6 | -4.0 ± 2.8 | -1.5 ± 4.1 |

### 9. Summary and Analysis of Heart Failure

Comparison of baseline characteristics of patients with heart failure events revealed that while the risk for heart failure was higher in the bardoxolone methyl-treated patients, both bardoxolone methyl-treated and placebo-treated patients with heart failure were more likely to have had a prior history of cardiovascular disease and heart failure and on average, had higher baseline ACR, BNP, and QTcF. Thus, development of heart failure in these patients was likely associated with traditional risk factors for heart failure. Additionally, many of the patients with heart failure were in subclinical heart failure prior to randomization, as indicated by their high baseline BNP levels.

As a surrogate of fluid retention after randomization, post-hoc analysis was performed on a subset of patients for whom BNP data were available, and increases were significantly greater in bardoxolone methyl-treated patients vs. placebo-treated patients at Week 24, with the BNP increases in bardoxolone methyl-treated patients directly correlated with baseline ACR. Urinary sodium excretion data from BEACON ABPM sub-study patients revealed a clinically meaningful reduction in urine volume and excretion of sodium at Week 4 relative to baseline in the bardoxolone methyl-treated patients only. In another study, urinary sodium levels and water excretion were reduced in stage 4 CKD patients but not in stage 3b CKD patients. Together, these data suggest that bardoxolone methyl differentially affects sodium and water handling, with retention of these more pronounced in patients with stage 4 CKD.

Consistent with this phenotype for fluid retention, post-hoc review of the narrative descriptions for heart failure events provided in hospital admission notes, together with anecdotal reports from investigators, indicates that heart failure events in bardoxolone methyl-treated patients were often preceded by rapid fluid weight gain and were not associated with acute decompensation of the kidneys or heart.

Blood pressure changes, indicative of overall volume status, were also increased in the bardoxolone methyl group relative to the placebo group as measured by standardized blood pressure cuff monitoring in BEACON. Pre-specified blood pressure analysis in healthy volunteer studies demonstrated no changes in either systolic or diastolic blood pressure. While the intent-to-treat (ITT) analyses of phase 2 CKD studies conducted with bardoxolone methyl showed no clear changes in blood pressure, post-hoc analyses of these studies suggest that increases in both systolic and diastolic blood pressure are dependent on CKD stage. Taken together, these data suggest that the effects of bardoxolone methyl treatment on blood pressure may be associated with CKD disease severity.

Thus, the urinary electrolyte, BNP, and blood pressure data collectively support that bardoxolone methyl treatment can differentially affect volume status, having no clinically detectable effect in healthy volunteers or early-stage CKD patients, while likely promoting fluid retention in patients with more advanced renal dysfunction and with traditional risk factors associated with heart failure at baseline. The increases in eGFR are likely due to glomerular effects whereas effects on sodium and water regulation are tubular in origin. As eGFR change was not correlated with heart failure, the data suggest that effects on eGFR and sodium and water regulation are anatomically and pharmacologically distinct.

The increased risk for heart failure and related adverse events with bardoxolone methyl treatment was not observed in prior studies (Table 14). However, since prior studies of bardoxolone methyl enrolled 10-fold fewer patients, the increased risk, if present, may have been undetectable. Moreover, BEACON limited enrollment to patients with stage 4 CKD, a population known to be at higher risk for cardiovascular events relative to patients with stage 3b CKD. Thus, the advanced nature of renal disease and significant cardiovascular risk burden of the BEACON population (manifested, among other markers, by low baseline eGFR, high baseline ACR, and high baseline BNP levels) were likely important factors in the observed pattern of cardiovascular events.

To examine further the relationship between key endpoints in BEACON and clinically meaningful thresholds of traditional risk factors of fluid overload, an additional post-hoc analysis was performed. Various eligibility criteria related to these risk factors were applied to exclude patients at most risk and explore the resulting outcomes from BEACON. Combinations of select criteria, including exclusion of patients with eGFR of 20 mL/min/1.73 m² or less, markedly elevated levels of proteinuria, and either age over 75 or BNP greater than 200 pg/mL abrogate the observed imbalances (Table 15). Applying these same criteria to SAEs also markedly improves or abrogates the noted imbalances (Table 16). Taken together, these findings suggest utility of these and other renal and cardiovascular risk markers in future selection criteria for clinical studies with bardoxolone methyl.

**Table 14. Frequency of Treatment-Emergent Adverse Events Related to Heart Failure¹ by Primary System Organ Class (SOC) Observed in Prior Chronic Kidney Disease Studies with Bardoxolone Methyl**

| | **Study** | | **0804 (BEAM)** | | | | **0902** | | | | |
|---|---|---|---|---|---|---|---|---|---|---|---|
| | | | **BARD (Crystalline)** | | | | **BARD (SDD)** | | | | |
| | **SOC** | **Preferred Term** | **PBO (N = 57)** | **25 mg (N = 57)** | **75 mg (N = 57)** | **150 mg (N = 56)** | **2.5 mg (N = 14)** | **5 mg (N = 25)** | **10 mg (N = 28)** | **15 mg (N = 50)** | **30 mg (N = 14)** |
| **AEs** | **Metab** | Oedema peripheral | 3 (5) | 3 (5) | 1 (2) | 3 (5) | 0 | 0 | 0 | 0 | 1 (7) |
| | | Fluid overload | 0 | 3 (5) | 2 (4) | 0 | - | - | - | - | - |
| | **Genrl** | Oedema peripheral | 11 (19) | 11 (19) | 10 (18) | 11 (20) | 0 | 3 (12) | 5 (18) | 3 (6) | 3 (21) |
| | | Generalised oedema | 0 | 2 (4) | 0 | 0 | - | - | - | - | - |
| | **Resp** | Dyspnoea | 5 (9) | 2 (4) | 6 (11) | 4 (7) | 0 | 0 | 0 | 0 | 1 (7) |
| | | Dyspnoea exertional | 0 | 1 (2) | 0 | 3 (5) | 1 (7) | 0 | 0 | 0 | 0 |
| | | Orthopnoea | 1 (2) | 0 | 0 | 0 | - | - | - | - | - |
| | | Pulmonary oedema | 0 | 0 | 1 (2) | 0 | - | - | - | - | - |
| | **Inv** | Ejection fraction decreased | 0 | 1 (2) | 0 | 0 | - | - | - | - | - |
| | **Card** | Oedema peripheral | 1 (2) | 4 (7) | 3 (5) | 4 (7) | 0 | 0 | 1 (4) | 1 (2) | 0 |
| | | Cardiac failure congestive | 3 (5) | 2 (4) | 3 (5) | 3 (5) | 0 | 0 | 1 (4) | 0 | 1 (7) |
| | | Dyspnoea paroxysmal nocturnal | 0 | 0 | 1 (2) | 0 | - | - | - | - | - |
| **SAEs** | **Card** | Cardiac failure congestive | 3 (5) | 2 (4) | 2 (4) | 2 (4) | 0 | 0 | 1 (4) | 0 | 1 (7) |
| | **Genrl** | Oedema peripheral | 0 | 0 | 0 | 1 (2) | - | - | - | - | - |
| | **Metab** | Fluid overload | 0 | 1 (2) | 1 (2) | 0 | - | - | - | - | - |
| | **Resp** | Dyspnoea | 1 (2) | 0 | 0 | 0 | - | - | - | - | - |
| | | Pulmonary oedema | 0 | 0 | 1 (2) | 0 | - | - | - | - | - |

| | | | | | | | | | | | |
|---|---|---|---|---|---|---|---|---|---|---|---|
| In 402-C-0804, patients were administered 25, 75, 150 mg of bardoxolone methyl (crystalline formulation) or placebo once daily for 52 weeks. In RTA402-C-0903, patients were administered 2.5, 5, 10, 15, or 30 mg doses of bardoxolone methyl (SDD formulation) once daily for 85 days. 1 Adverse events with preferred terms matching Standardized MedDRA Queries for cardiac failure outlined in the BEACON EAC Charter (Submission Serial 133, dated February 2, 2012). | | | | | | | | | | | |

**Table 15. Effect of Excluding Patients with Select Baseline Characteristics on Primary Endpoints, Heart Failure, and All-Cause Mortality in BEACON**

| **Event** | **Observed** | | **Eligibility Criteria (N)** | | | | | | |
|---|---|---|---|---|---|---|---|---|---|
| | | **N** | **BL BNP ≤ 200** | **No h/o HF** | **BL ACR ≤ 1000** | **BL eGFR > 20** | **Age ≤ 75** | **BL ACR ≤ 1000, eGFR > 20, Age ≤ 75** | **BL ACR ≤ 300, eGFR > 20, BNP ≤ 200** |
| **Heart Failure** | BARD | 103 | 22 | 67 | 63 | 56 | 75 | 19 | 5 |
| | PBO | 57 | 16 | 36 | 40 | 37 | 45 | 20 | 3 |
| **All-Cause Death** | BARD | 44 | 14 | 35 | 32 | 27 | 20 | 11 | 5 |
| | PBO | 31 | 8 | 24 | 21 | 18 | 23 | 11 | 4 |
| **ESRD** | BARD | 47 | 12 | 35 | 21 | 18 | 38 | 9 | 1 |
| | PBO | 55 | 22 | 44 | 27 | 14 | 46 | 6 | 1 |
| **Randomized Patients** | BARD | 1088 | 559 | 922 | 798 | 735 | 786 | 368 | 209 |
| | PBO | 1097 | 593 | 943 | 792 | 718 | 829 | 400 | 217 |

| | | | | | | | | | |
|---|---|---|---|---|---|---|---|---|---|
| Post-hoc analysis of outcomes in BEACON. Observed totals for number of patients with heart failure, all-cause and cardiovascular deaths, and ESRD includes all events through last date of contact (ITT Population). | | | | | | | | | |

**Table 16. Effect of Excluding Patients with Select Baseline Characteristics on Treatment-Emergent Serious Adverse Events by Primary SOC in BEACON (ITT Population)**

| **Primary SOC** | **All Patients** | | **BL ACR ≤ 1000, eGFR > 20, Age ≤ 75** | | **BL ACR ≤ 300, eGFR > 20, BNP ≤ 200** | |
|---|---|---|---|---|---|---|
| **Treatment** | **PBO (N = 1097)** | **BARD (N = 1088)** | **PBO (N = 400)** | **BARD (N = 368)** | **PBO (N = 217)** | **BARD (N = 209)** |
| Blood and lymphatic system disorders | 11 (1) | 20 (2) | 3 (<1) | 4 (<1) | 2 (<1) | 0 |
| Cardiac disorders | 84 (8) | 124 (11) | 32 (3) | 35 (3) | 10 (1) | 16 (1) |
| Ear and labyrinth disorders | 1 (<1) | 3 (<1) | 1 (<1) | 1 (<1) | 0 | 1 (<1) |
| Endocrine disorders | 1 (<1) | 1 (<1) | 1 (<1) | 1 (<1) | 1 (<1) | 1 (<1) |
| Eye disorders | 2 (<1) | 3 (<1) | 1 (<1) | 1 (<1) | 1 (<1) | 0 |
| Gastrointestinal disorders | 39 (4) | 46 (4) | 13 (1) | 10 (1) | 8 (1) | 7 (1) |
| General disorders and administration site conditions | 20 (2) | 29 (3) | 3 (<1) | 2 (<1) | 2 (<1) | 3 (<1) |
| Hepatobiliary disorders | 8 (1) | 4 (<1) | 2 (<1) | 1 (<1) | 0 | 1 (<1) |
| Immune system disorders | 0 | 2 (<1) | 0 | 0 | 0 | 0 |
| Infections and infestations | 63 (6) | 79 (7) | 20 (2) | 20 (2) | 12 (1) | 9 (1) |
| Injury, poisoning and procedural complications | 17 (2) | 19 (2) | 3 (<1) | 4 (<1) | 0 | 2 (<1) |
| Investigations | 2 (<1) | 3 (<1) | 1 (<1) | 2 (<1) | 0 | 0 |
| Metabolism and nutrition disorders | 42 (4) | 51 (5) | 11 (1) | 14 (1) | 9 (1) | 5 (<1) |
| Musculoskeletal and connective tissue disorders | 13 (1) | 21 (2) | 6 (1) | 9 (1) | 3 (<1) | 6 (1) |
| Neoplasms benign, malignant and unspecified | 10 (1) | 11 (1) | 6 (1) | 3 (<1) | 2 (<1) | 1 (<1) |
| Nervous system disorders | 35 (3) | 37 (3) | 13 (1) | 6 (1) | 9 (1) | 4 (<1) |
| Psychiatric disorders | 3 (<1) | 3 (<1) | 1 (<1) | 2 (<1) | 1 (<1) | 1 (<1) |
| Renal and urinary disorders | 71 (6) | 52 (5) | 14 (1) | 9 (1) | 2 (<1) | 4 (<1) |
| Reproductive system and breast disorders | 3 (<1) | 2 (<1) | 0 | 0 | 0 | 0 |
| Respiratory, thoracic and mediastinal disorders | 32 (3) | 43 (4) | 11 (1) | 15 (1) | 7 (1) | 6 (1) |
| Skin and subcutaneous tissue disorders | 1 (<1) | 4 (<1) | 1 (<1) | 1 (<1) | 1 (<1) | 1 (<1) |
| Surgical and medical procedures | 0 | 2 (<1) | 0 | 1 (<1) | 0 | 1 (<1) |
| Vascular disorders | 18 (2) | 20 (2) | 5 (<1) | 4 (<1) | 2 (<1) | 2 (<1) |

| | | | | | | |
|---|---|---|---|---|---|---|
| Post-hoc analyses of treatment-emergent serious adverse events in BEACON. Event totals include only SAEs with onset no more than 30 days after a patient's last dose of study drug. | | | | | | |

### D. Potential Mechanisms of Fluid Overload in BEACON

Data presented in prior sections suggest that bardoxolone methyl promotes fluid retention in a subset of patients who are at most risk of developing heart failure independent of drug administration. The data suggest that the effects are not associated with acute or chronic renal or cardiac toxicity. Therefore, a comprehensive list of well-established renal mechanisms that affect volume status (Table 17) was explored to determine if any of the etiologies matched the clinical phenotype observed with bardoxolone methyl.

Initial investigation focused on possible activation of the renin-angiotensin-aldosterone system. Activation of this pathway reduces serum potassium due to increased renal excretion. However, bardoxolone methyl did not affect serum potassium and slightly reduced urinary potassium in the BEACON sub-study (Table 7).

Another potential mechanism that was investigated was whether transtubular ion gradient changes may have resulted in sodium and consequent water resorption, since bardoxolone methyl affects serum magnesium and other electrolytes. However, this mechanism also involves potassium regulation, and baseline serum magnesium did not appear to be associated with fluid retention or heart failure hospitalizations.

After other etiologies were excluded for reasons listed in Table 16, suppression of endothelin signaling was the primary remaining potential mechanism of volume regulation that was consistent with the bardoxolone methyl treatment effect in BEACON. Therefore, an extensive investigation of modulation of the endothelin pathway as a potential explanation for fluid retention observed in the BEACON study was conducted.

**Table 17. Established Renal Mechanisms Affecting Volume Status**

| **Mechanism** | **Na⁺ Retention** | **K⁺ Retention** | **Effect on GFR** | **Comments** |
|---|---|---|---|---|
| **Bardoxolone Methyl** | ↑ | None | ↑ | -↑ Na⁺ retention independent of K⁺ in Stage 4 CKD patients, ↑ GFR |
| **Endothelin** | ↓ | None | ↓ | -Suppression of endothelin fits BARD pattern |
| **Endothelial Nitric Oxide (NO)** | ↓ | None | ↑ | -NO ↓ Na⁺ reabsorption and ↑ GFR |
| | | | | -BARD ↑ both Na⁺ and GFR |
| | | | | -BARD has been shown *in vitro* and *in vivo* to increase bioavailable endothelial NO, but Na⁺ effect is likely independent of NO and GFR changes |
| **Antidiuretic Hormone (ADH)** | ↑ | ↑ | ↓ at ↑ levels of ADH | -ADH TNa⁺ and K⁺ while ↓GFR |
| | | | | -BARD does not affect K⁺ and ↑ GFR |
| **Transtubular ion gradients** | ↑ with | ↑ | No direct effect | -Ion gradients have dual effect on Na⁺ and K⁺; Cl^{,} HCO₃⁻ gradient often generated as HCO₃⁻ absorption dependent on Na⁺ absorption |
| | ↑ GFR | | | |
| | | | | -BARD does not affect K⁺ or HCO₃⁻ |
| **Renin-Angiotensin-Aldosterone (RAAS)** | ↑ | ↓ | ↑ | -RAAS signaling ↑ K urinary excretion and ↓ serum levels |
| | | | | -BARD does not affect K⁺ levels and has been shown to ↓ AII levels in CKD patients and suppress AII signaling *in vitro* and *in vivo* |
| **Pressure Natriuresis** | ↓ | ↓ | Slight ↑ | -Volume expansion leads to ↑ medullary plasma flow and ↓ hypertonicity; ↓ water absorption in the loop of Henle with ↓ of Na⁺ and K⁺ |
| | | | | -BARD-mediated magnitude of volume expansion unlikely sufficient to promote this effect; BARD ↑ Na⁺ and does not affect K⁺ |
| **Prostaglandins (PGE₂, PGI₂)** | **↓** | Slight ↓ | ↑ | -PGs ↑GFR and ↑Na⁺ urine excretion |
| | | | | -BARD ↑ Na⁺ retention, not excretion |
| **Natriuretic peptides** | ↓ | Slight ↓ | ↑ | -Natriuretic peptides have divergent effects on Na⁺ and GFR with slight effect on K⁺ |
| | | | | -BNP and other natriuretic peptides ↑ Na⁺ urine excretion |
| | | | | -BARD ↑ Na⁺ retention, not excretion |
| | | | | -BARD does no interfere with natriuretic peptides, as GFR would likely ↓ |
| **Peritubular factors** | ↑ with | ↑ with | None | -Na⁺ and K⁺ move with GFR |
| | ↑ GFR | ↑ GFR | | -BARD does not affect K⁺ |

| | | | | |
|---|---|---|---|---|
| Mechanisms and characteristics of fluid retention. | | | | |

### 1. Modulation of the Endothelin System

The most directly analogous clinical data for comparison of the effects of known endothelin pathway modulators to the BEACON study are those with the endothelin receptor antagonist (ERA) avosentan. Avosentan was studied in stage 3-4 CKD patients with diabetic nephropathy in the ASCEND study, a large outcomes study to assess the time to first doubling of serum creatinine, ESRD, or death (Mann *et al.,* 2010). While the baseline eGFR in this study was slightly above the mean baseline eGFR in BEACON, patients in the ASCEND study had a mean ACR that was approximately seven-fold higher than BEACON (Table 18). Therefore, the overall cardiovascular risk profile was likely similar between the two studies.

As in BEACON, the ASCEND study was terminated prematurely due to an early imbalance in heart failure hospitalization and fluid overload events. Importantly, avosentan-induced fluid overload-related adverse events, including serious and non-serious, were increased only within the first month of treatment (FIGS. 10A-B).

Examination of key endpoints in the ASCEND study reveals an approximate threefold increase in risk of congestive heart failure (CHF) with a modest, non-significant increase in death. Additionally, a small, numerical reduction in ESRD events was also observed. The BEACON study demonstrated similar findings, albeit with a lower incidence of heart failure events. Nonetheless, the two studies showed striking similarities in clinical presentation and timing of heart failure, as well as influences on other key endpoints (Table 19).

**Table 18. Select Demographic and Baseline Characteristics for Patients in ASCEND* and BEACON (ITT Population)**

| **BL Characteristic** | **ASCEND** | | | **BEACON** | |
|---|---|---|---|---|---|
| | **PBO (N = 459)** | **Avosentan 25 mg (N = 455)** | **Avosentan 50 mg (N = 478)** | **PBO (N = 1097)** | **BARD 20 mg (N = 1088)** |
| **Age** | 61 ± 9 | 61 ± 9 | 61 ± 9 | 68 ± 9 | 69 ± 10 |
| **History of CHF (% of patients)** | 13.5% | 14.5% | 14.4% | 15% | 14% |
| **Systolic Blood Pressure (mmHg)** | 135 ± 15 | 137 ± 14 | 137 ± 14 | 140 ± 12 | 140 ± 12 |
| **BMI (kg/m²)** | 30 ± 6 | 30 ± 6 | 30 ± 7 | 34 ± 7 | 34 ± 7 |
| **eGFR (mL/min/1.73 m²)** | 33 ± 11 | 34 ± 11 | 33 ± 11 | 22 ± 5 | 22 ± 4 |
| **Median ACR (mg/g)** | 1540 | 1416 | 1474 | 221 | 210 |
| **ACEi / ARB (% of patients)** | 100% | 100% | 100% | 84% | 85% |
| **Diuretics (% of patients)** | 65% | 64% | 65% | 64% | 64% |

| | | | | | |
|---|---|---|---|---|---|
| *Results from a randomized, double-blind, placebo-controlled trial of 1392 patients with type 2 diabetes and overt nephropathy receiving avosentan (25 or 50 mg) or placebo in addition to continued angiotensin-converting enzyme inhibition and/or angiotensin receptor blockade (ASCEND). | | | | | |

**Table 19. Occurrence of Death, End Stage Renal Disease, or Heart Failure in ASCEND and BEACON (ITT Population)**

| | **ASCEND** | | | **BEACON** | |
|---|---|---|---|---|---|
| **Event** | **PBO** (N = 459) | **Avosentan 25 mg** (N = 455) | **Avosentan 50 mg** (N = 478) | **PBO** (N = 1097) | **BARD 20 mg** (N = 1088) |
| **CHF** | 2.2% | 5.9%* | 6.1%* | 5.0% | 8.8%* |
| **Death** | 2.6% | 3.6% | 4.6% | 2.8% | 4.0% |
| **ESRD** | 6.5% | 4.4% | 5.0% | 4.6% | 4.0% |

| | | | | | |
|---|---|---|---|---|---|
| Occurrence of adjudicated CHF, death, and ESRD events in ASCEND and BEACON. In ASCEND, for an event to be qualified as CHF, the patient had to have typical signs and/or symptoms of heart failure and receive new therapy for CHF and be admitted to the hospital for at least 24 hours; ESRD was defined as need for dialysis or renal transplantation or an eGFR < 15 mL/min/1.73 m². Percentages for BEACON include all CHF and ESRD events through last date of contact and total number of deaths at the time of database lock (March 21, 2013). ESRD in BEACON was defined as need for chronic dialysis, renal transplantation, or renal death; additional details and definitions for heart failure are outlined in the BEACON EAC Charter. * *p* < 0.05 *vs.* placebo. | | | | | |

### 2. Mechanism of Endothelin Receptor Antagonist-Induced Fluid Overload

The role of endothelin in fluid overload has been extensively investigated. Through the use of knock-out models in mice, investigators have demonstrated that acute disruption of the endothelin pathway followed by a salt challenge promotes fluid overload. Specific knock-out of endothelin-1 (ET-1), endothelin receptor type A (ETA), endothelin receptor type B (ETB), or the combination of ETA and ETB have all been shown to promote fluid overload in animals with a clinical phenotype consistent with ERA-mediated fluid overload in patients. These effects are caused by acute activation of the epithelial sodium channel (ENaC), which is expressed in the collecting ducts of the kidney, where it reabsorbs sodium and promotes fluid retention (Vachiery and Davenport, 2009).

### 3. Relationship between Plasma and Urinary Endothelin-1 in Humans

An assessment of plasma and urinary levels of endothelin-1 (ET-1) in humans with eGFR values ranging from stage 5 CKD to supra-normal (8 to 131 mL/min/1.73 m²) has been previously reported (Dhaun *et al.,* 2009). Plasma levels significantly and inversely correlated with eGFR, but due to the modest slope of the curve, meaningful differences of ET-1 were not readily apparent across the large eGFR range assessed. As a surrogate for kidney production of ET-1, the organ where the most ET-1 is produced, fractional excretion of ET-1 was calculated by assessing the plasma and urinary levels of ET-1. From eGFRs >100 to approximately 30 mL/min/1.73 m², urinary levels were relatively unchanged (FIG. 17). However, ET-1 levels appear to increase exponentially with decreasing eGFR in patients with stage 4 and 5 CKD. These data suggest that renal ET-1 is primarily dysregulated in patients with advanced (stage 4 and 5) CKD. Based on these published data, the inventors hypothesized that the differential effects on fluid handling by bardoxolone methyl, if due to endothelin modulation, could be due to the disparate endogenous production of ET-1 in the kidney, which is meaningfully increased in stage 4 and 5 CKD patients.

### 4. Bardoxolone Methyl Modulates Endothelin Signaling

As described above, bardoxolone methyl reduces ET-1 expression in human cell lines, including mesangial cells found in the kidney as well as endothelial cell. Furthermore, *in vitro* and *in vivo* data suggest that bardoxolone methyl and analogs modulate the endothelin pathway to promote a vasodilatory phenotype by suppressing the vasoconstrictive ET_{A} receptor and restoring normal levels of the vasodilatory ET_{B} receptor. Thus, the potent activation of Nrf2-related genes with bardoxolone methyl is associated with suppression of pathological endothelin signaling and facilitates vasodilation by modulating expression of ET receptors.

### E. Rationale for BEACON Termination

### 1. Adjudicated Heart Failure

Hospitalizations for heart failure or death due to heart failure were among the cardiovascular events adjudicated by the EAC. An imbalance in adjudicated heart failure and related events was the major finding that contributed to the early termination of BEACON. Additionally, heart failure-related AEs, such as edema, contributed to a higher discontinuation rate than expected. The overall imbalance in time-to-first adjudicated heart failure appeared to result from the large contribution of events occurring within the first three to four weeks after initiation of treatment. The Kaplan-Meyer analysis shows that after this initial period the event rates between the treatment arms appear to maintain parallel trajectories. The pattern reflected in FIG. 11 suggests an acute, physiologic effect that precipitated hospitalization for heart failure versus a cumulative toxic effect.

### 2. Mortality

At the time of the termination of the study, more deaths had occurred in the bardoxolone methyl group than in the placebo group, and the relationship between mortality and heart failure was unclear. A majority of the fatal outcomes (49 of the 75 deaths) occurring prior to clinical database lock (March 4, 2013) were confirmed as being cardiovascular in nature (29 bardoxolone methyl patients *vs.* 20 placebo patients). Most of the cardiovascular deaths were classified as "cardiac death - not otherwise specified," based on pre-specified definitions outlined in the BEACON EAC charter. On final analysis, the Kaplan-Meier analysis for overall survival showed no apparent separation until approximately Week 24 (FIG. 12). There were three fatal heart failure events, all in bardoxolone methyl-treated patients. In addition, as reflected in Table 16, the percentage of deaths occurring in patients that were over 75 years old was higher in bardoxolone methyl-treated patients compared to placebo-treated patients. Notably, if patients over 75 years old are excluded, the numbers of fatal events in the bardoxolone methyl arm compared to the placebo arm are 20 and 23, respectively.

### 3. Summary of Other Safety Data from BEACON

In addition to the effects of bardoxolone methyl treatment on eGFR and renal SAEs, the number of hepatobiliary SAEs was reduced in the bardoxolone methyl group relative to the placebo group (4 versus 8, respectively; Table 2), and no Hy's Law cases were observed. The number of neoplasm-related SAEs was also balanced across both groups. Lastly, bardoxolone methyl treatment was not associated with QTc prolongation, as assessed by ECG assessments at Week 24 (Table 20).

**Table 20. Change from Baseline in QTcF at Week 24 in Bardoxolone Methyl versus Placebo Patients in BEACON (Safety Population)**

| | | **Observed** | | **Change from baseline** | |
|---|---|---|---|---|---|
| **Timepoint/ QTcF interval (msec)** | | **Placebo N = 1093** | **Bardoxolone methyl N = 1092** | **Placebo N = 1093** | **Bardoxolone methyl N = 1092** |
| | n | 719 | 639 | 719 | 637 |
| | Mean (SD) | 428.8 (29.2) | 425.8 (26.5) | 3.6 (16.4) | -0.9 (19.2) |
| | Range (min, max) | 362, 559 | 355, 518 | -59, 82 | -88, 69 |
| | Quartiles (25th, median, 75th) | 408, 426, 445 | 407, 425, 443 | -7, 3, 13 | -13, -1, 10 |

| | | | | | |
|---|---|---|---|---|---|
| Data includes only ECG assessments collected on or before a patient's last dose of study drug. Visits are derived relative to a patient's first dose of study drug. | | | | | |

### F. BEACON Conclusions

In summary, interrogation of data from studies conducted with bardoxolone methyl revealed that the drug can differentially regulate fluid retention, with no clinically detectable effect in healthy volunteers or early-stage CKD patients, while likely pharmacologically promoting fluid retention in patients with advanced renal dysfunction. Since the development of heart failure in both bardoxolone methyl- and placebo-treated patients was associated with traditional risk factors for heart failure, this pharmacological effect in patients with baseline cardiac dysfunction may explain the increased risk for heart failure with bardoxolone methyl treatment in BEACON. These data suggest that decreasing the overall risk for heart failure in future clinical studies by selecting a patient population with lower baseline risk for heart failure should avoid increases in heart failure associated with bardoxolone methyl treatment. Importantly, the available data show that fluid overload in BEACON was not caused by a direct renal or cardiac toxicity. The clinical phenotype of fluid overload is similar to that observed with ERAs in advanced CKD patients, and preclinical data demonstrate that bardoxolone methyl modulates the endothelin pathway. As acute disruption of the endothelin pathway in advanced CKD patients is known to activate a specific sodium channel (ENaC) that can promote acute sodium and volume retention (Schneider, 2007), these mechanistic data, along with the clinical profile of bardoxolone methyl patients with heart failure, provide a reasonable hypothesis to the mechanism of fluid retention in BEACON. Because compromised renal function may be an important factor that contributes to a patient's inability to compensate for short-term fluid overload, and because relatively limited numbers of patients with earlier stages of CKD have been treated to date, exclusion of patients with CKD (*e.g.,* patients with an eGFR < 60) from treatment with BARD and other AIMs may be prudent and may be included as an element of the present invention.

### II. Compounds for the Treatment of Alport Syndrome or Symptoms thereof or the Prevention of Symptoms of Alport Syndrome

In one aspect of the present disclosure, there are provided methods of treating or reducing the symptoms of Alport syndrome in a patient comprising administering to the patient a therapeutically effective amount of bardoxolone methyl, an analog thereof, or a composition comprising either bardoxolone methyl or an analog thereof. Analogs of bardoxolone methyl include compounds of the formula: 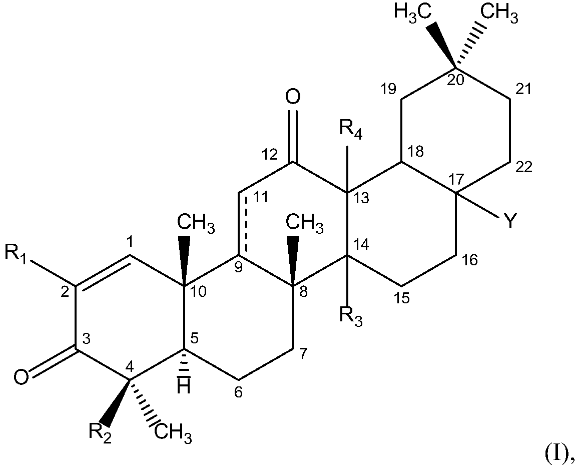 wherein:
R₁ is -CN, halo, -CF₃, or -C(O)Rₐ, wherein Rₐ is -OH, alkoxy_{(C1-4)}, -NH₂, alkylamino_{(C1-4)}, or -NH-S(O)₂-alkyl_{(C1-4)};
R₂ is hydrogen or methyl;
R₃ and R₄ are each independently hydrogen, hydroxy, methyl or as defined below when either of these groups is taken together with group R_{c}; and
Y is:
   -H, -OH, -SH, -CN, -F, -CF₃, -NH₂ or -NCO;
   alkyl_{(C≤8)}, cycloalkyl_{(C≤8)}, alkenyl_{(C≤8)}, alkynyl_{(C≤8)}, aryl_{(C≤12)}, aralkyl_{(C≤12)}, heteroaryl_{(C≤8)}, heterocycloalkyl_{(C≤12)}, alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)} arylamino_{(C≤8)}, aralkylamino_{(C≤8)}, alkylthio_{(C≤8)}, acylthio_{(C≤8)}, alkylsulfonylamino_{(C≤8)}, cycloalkylsulfonylamino_{(C≤8)}, or substituted versions of any of these groups;
   -alkanediyl_{(C≤8)}-R_{b}, -alkenediyl_{(C≤8)}-R_{b}, or a substituted version of any of these groups, wherein R_{b} is:
      hydrogen, hydroxy, halo, amino or mercapto; or
      heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)} arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
   -(CH₂)ₘC(O)R_{c}, wherein m is 0-6 and R_{c} is:
      hydrogen, hydroxy, halo, amino, -NHOH, or mercapto; or
      alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)} arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      R_{c} and R₃, taken together, are -O- or -NR₄-, wherein R_{d} is hydrogen or alkyl_{(C≤8)}; or
      R_{c} and R₄, taken together, are -O- or -NR₄-, wherein R_{d} is hydrogen or alkyl_{(C≤8)}; or
   -NHC(O)Rₑ, wherein Rₑ is:
      hydrogen, hydroxy, amino; or
      alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)} arylamino_{(C≤8)}, or a substituted version of any of these groups;
or a pharmaceutically acceptable salt thereof.

These compounds are known as antioxidant inflammation modulators. These compounds have shown the ability to activate Nrf2, as measured by elevated expression of one or more Nrf2 target genes (*e.g.,* NQO1 or HO-1; Dinkova-Kostova *et al.,* 2005). Further, these compounds are capable of indirect and direct inhibition of pro-inflammatory transcription factors including NF-κB and STAT3 (Ahmad *et al.,* 2006; Ahmad *et al.,* 2008). In some aspects, there are provided methods of preventing progression of Alport syndrome or a symptom thereof in a subject or patient in need thereof comprising administering to the subject or patient bardoxolone methyl or an analog thereof in an amount sufficient to prevent progression of Alport syndrome or a symptom thereof in the subject or patient. Additionally, one or more of the compounds described herein may be used in methods to prevent the onset of one or more symptoms of Alport syndrome or prevent the progression of Alport syndrome.

Triterpenoids, biosynthesized in plants by the cyclization of squalene, are used for medicinal purposes in many Asian countries; and some, such as ursolic and oleanolic acid, are known to be anti-inflammatory and anti-carcinogenic (Huang *et al.,* 1994; Nishino *et al.,* 1988). However, the biological activity of these naturally-occurring molecules is relatively weak, and therefore the synthesis of new analogs to enhance their potency was undertaken (Honda *et al.,* 1997; Honda *et al.,* 1998). An ongoing effort for the improvement of anti-inflammatory and antiproliferative activity of oleanolic and ursolic acid analogs led to the discovery of 2-cyano-3,12-dioxooleane-1,9(11)-dien-28-oic acid (CDDO) and related compounds (Honda *et al.,* 1997, 1998, 1999, 2000a, 2000b, 2002; Suh *et al.,* 1998; 1999; 2003; Place *et al.,* 2003; Liby *et al.,* 2005). Several potent derivatives of oleanolic acid were identified, including methyl-2-cyano-3,12-dioxooleana-1,9-dien-28-oic acid (CDDO-Me; RTA 402; bardoxolone methyl). RTA 402, an antioxidant inflammation modulator (AIM), suppresses the induction of several important inflammatory mediators, such as iNOS, COX-2, TNFα, and IFNy, in activated macrophages, thereby restoring redox homeostasis in inflamed tissues. RTA 402 has also been reported to activate the Keap1/Nrf2/ARE signaling pathway resulting in the production of several anti-inflammatory and antioxidant proteins, such as heme oxygenase-1 (HO-1). It induces the cytoprotective transcription factor Nrf2 and suppresses the activities of the pro-oxidant and pro-inflammatory transcription factors NF-κB and STAT3. *In vivo,* RTA 402 has demonstrated significant single agent anti-inflammatory activity in several animal models of inflammation such as renal damage in the cisplatin model and acute renal injury in the ischemia-reperfusion model. In addition, significant reductions in serum creatinine have been observed in patients treated with RTA 402.

Accordingly, in pathologies involving oxidative stress alone or oxidative stress exacerbated by inflammation, treatment may comprise administering to a subject or patient a therapeutically effective amount of a compound of this invention, such as those described above or throughout this specification. Treatment may be administered preventively in advance of a predictable state of oxidative stress (*e.g*., organ transplantation or the administration of therapy to a cancer patient), or it may be administered therapeutically in settings involving established oxidative stress and inflammation.

Non-limiting examples of triterpenoids that may be used in accordance with the methods of this invention are shown here.

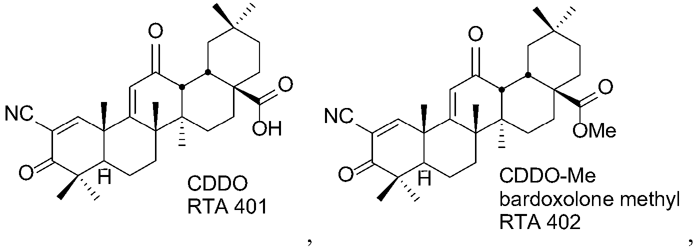

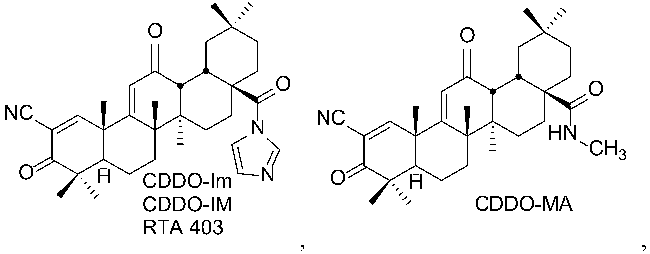

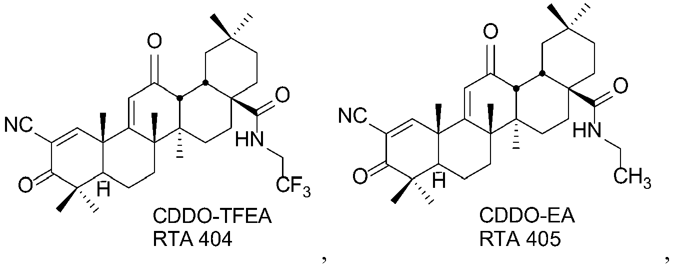

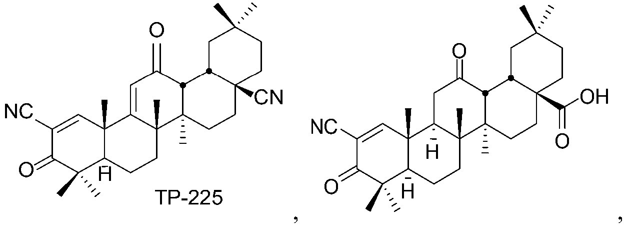

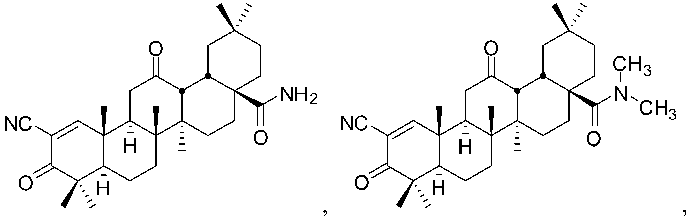

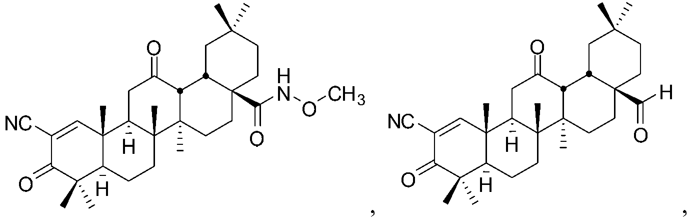

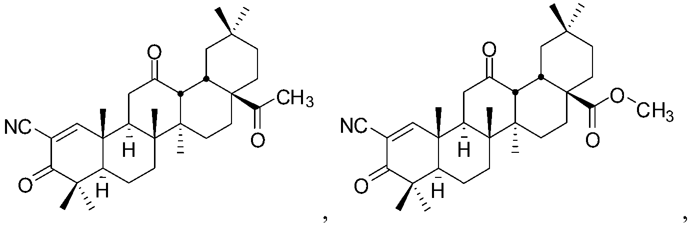

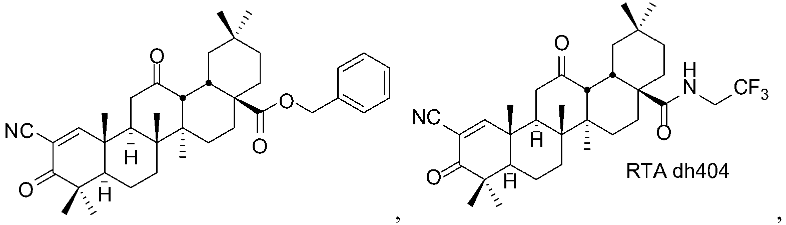

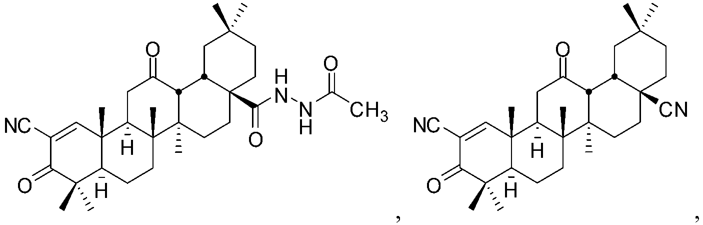

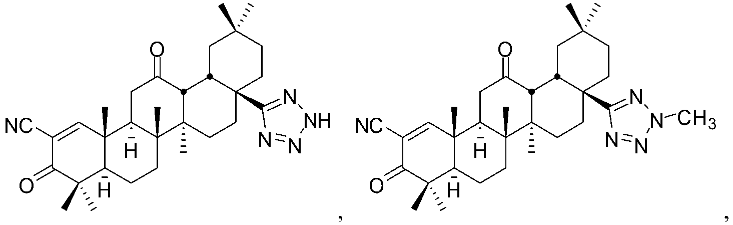

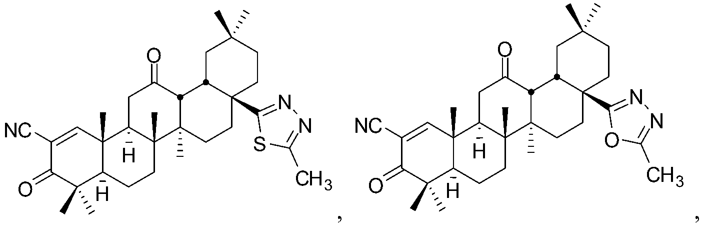

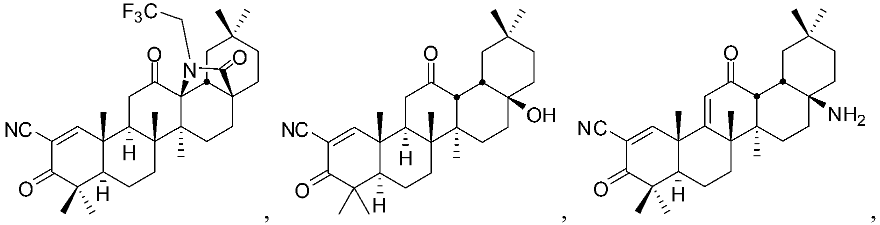

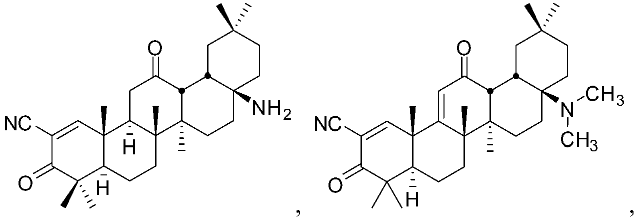

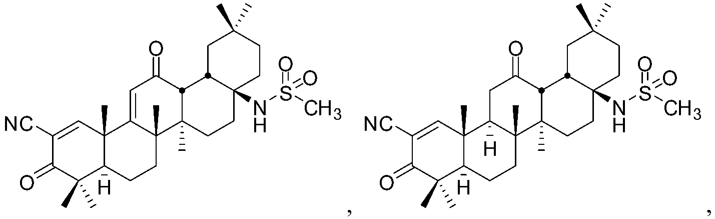

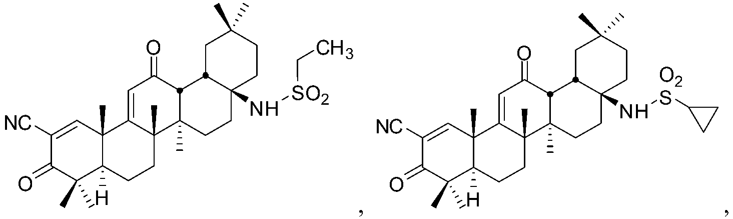

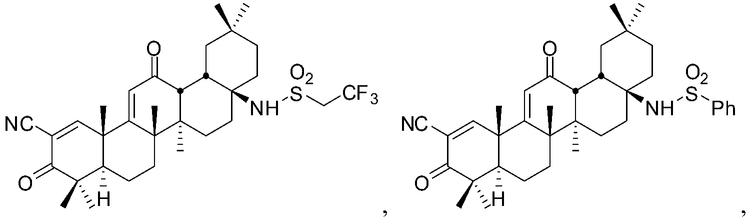

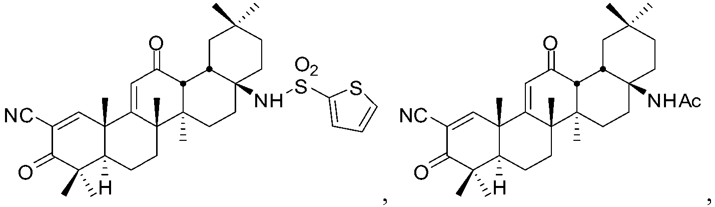

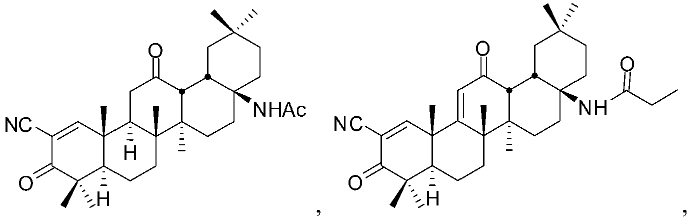

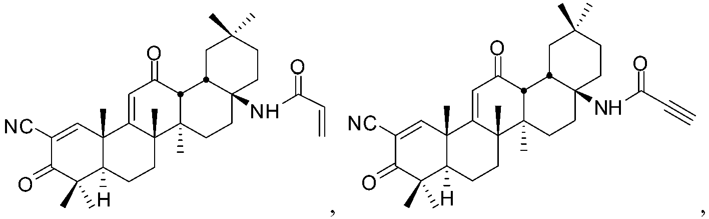

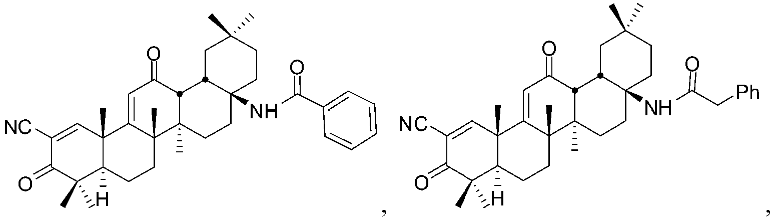

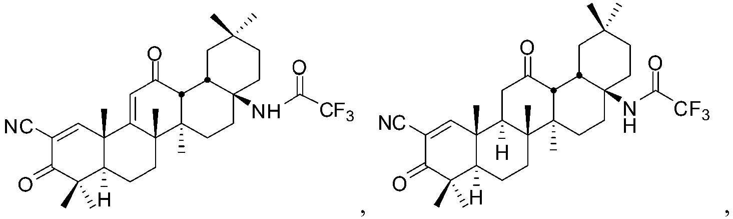

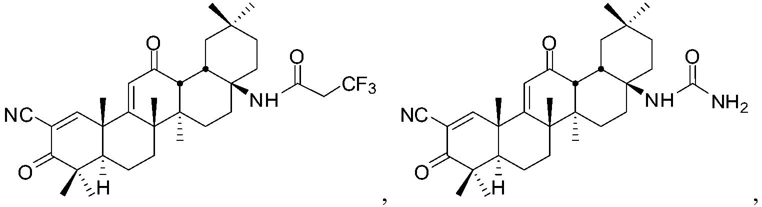

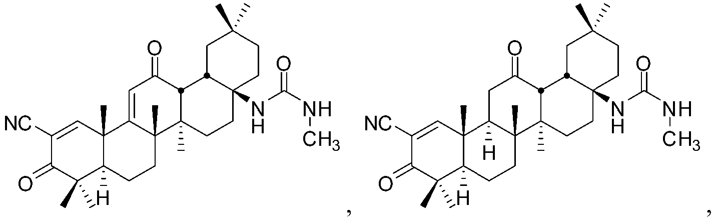

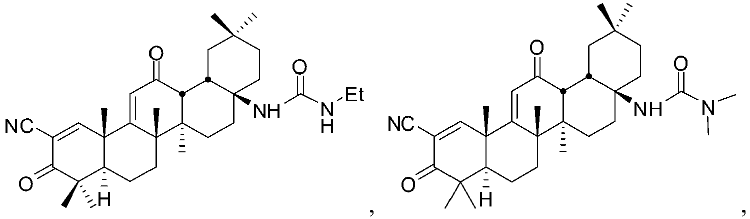

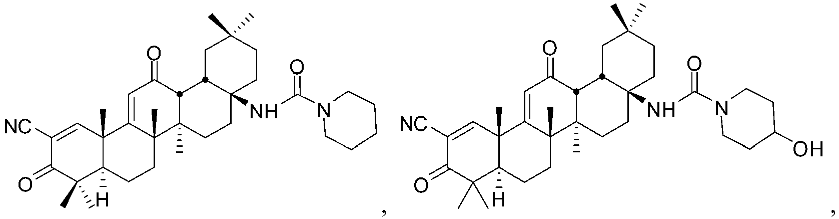

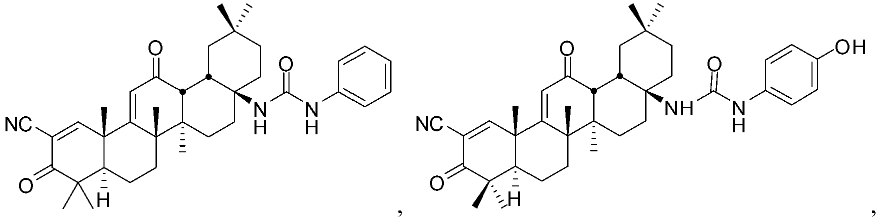

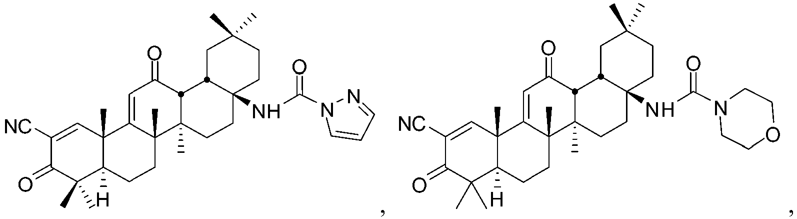

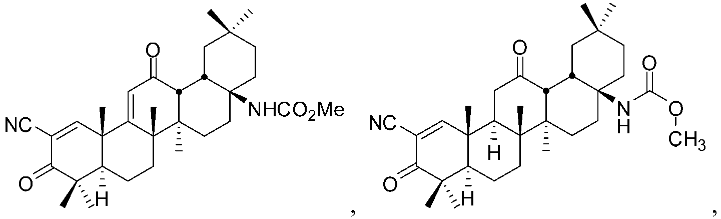

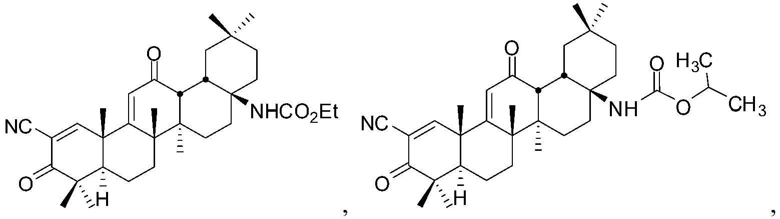

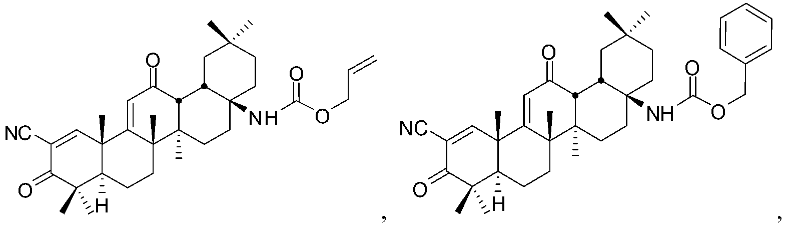

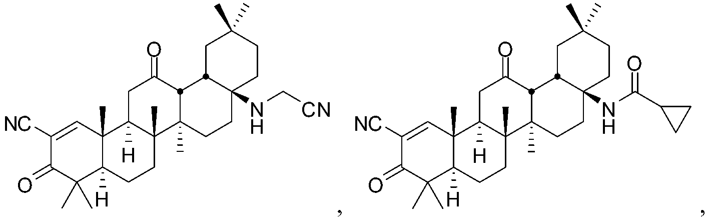

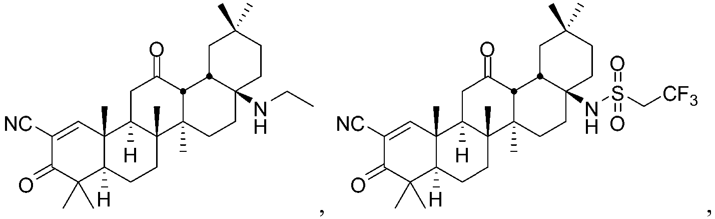

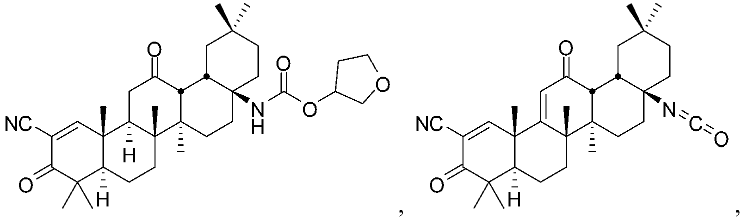

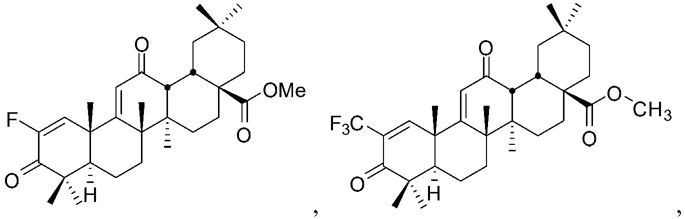

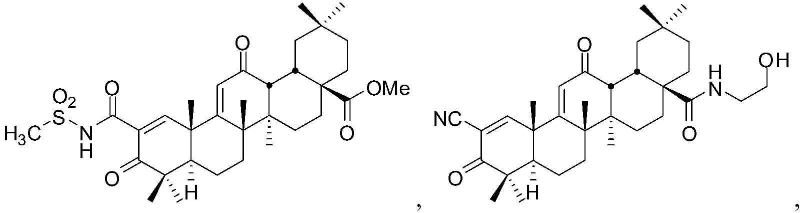

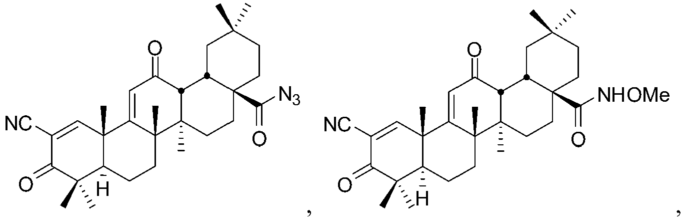

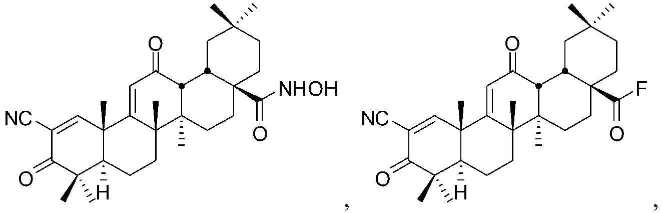

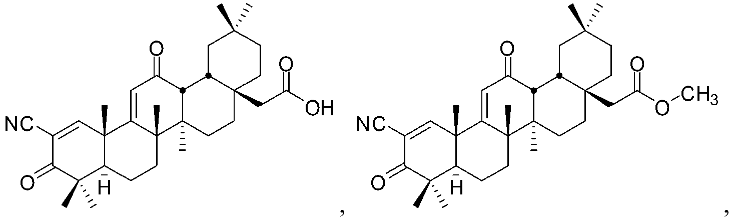

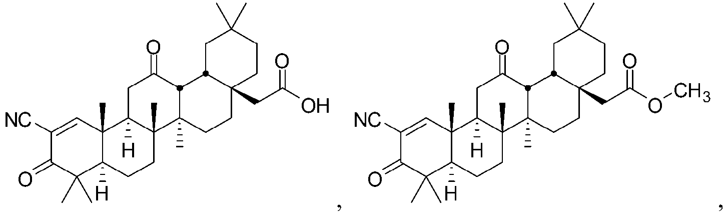

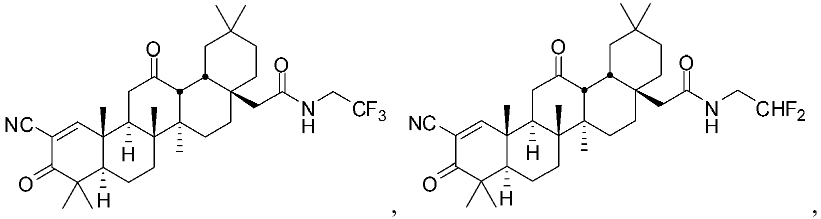

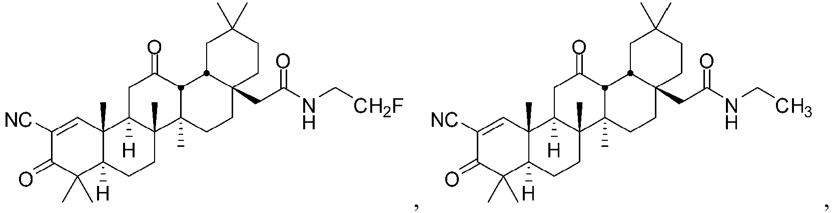

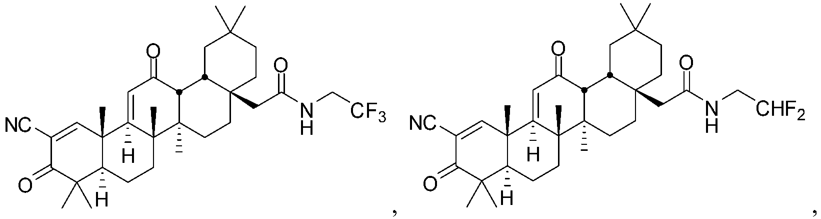

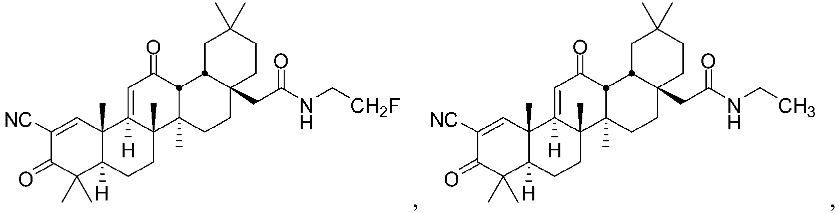

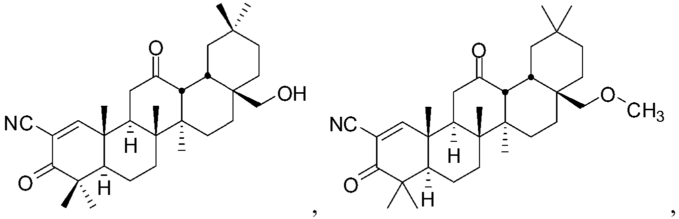

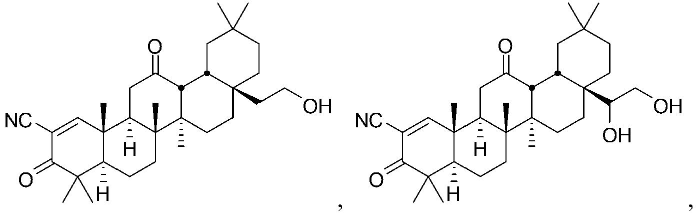

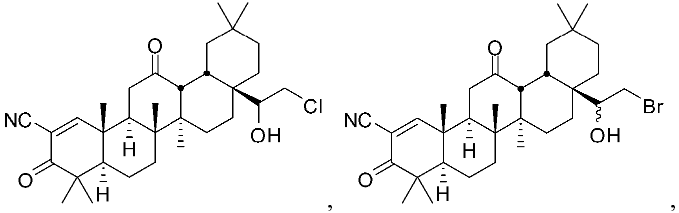

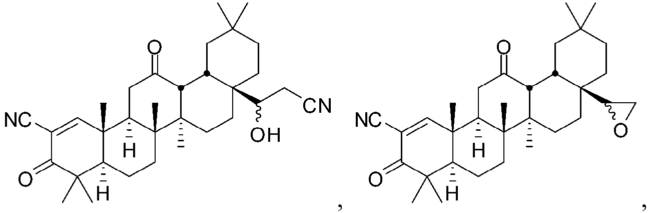

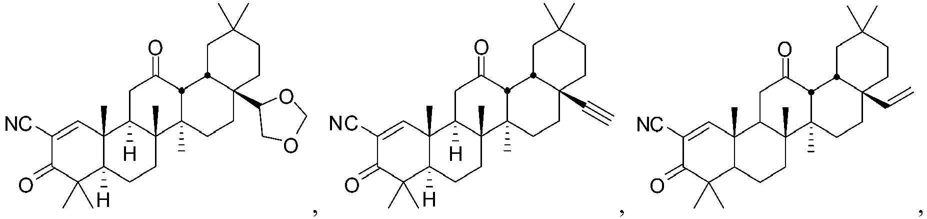

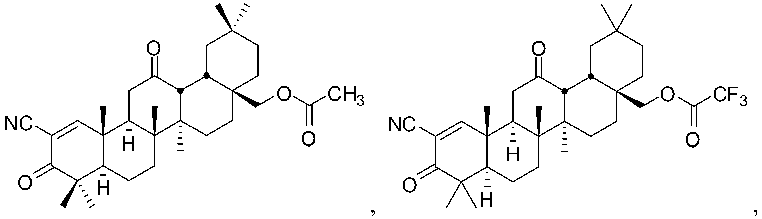

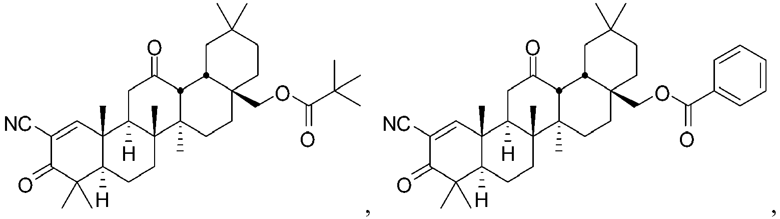

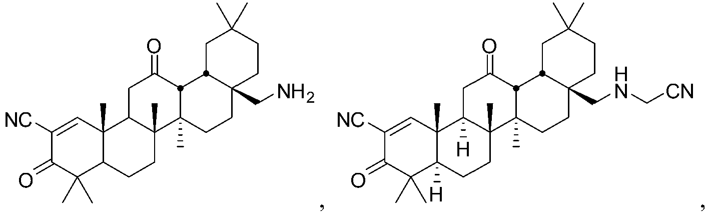

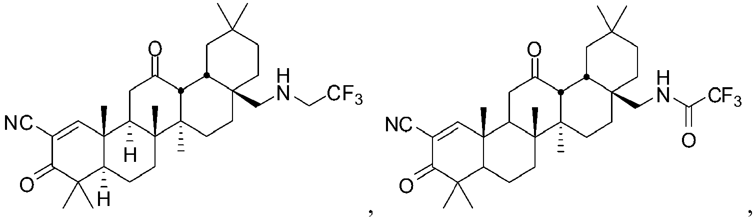

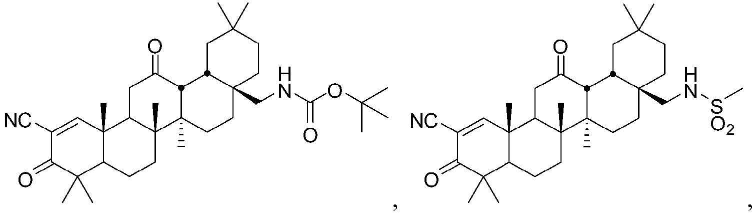

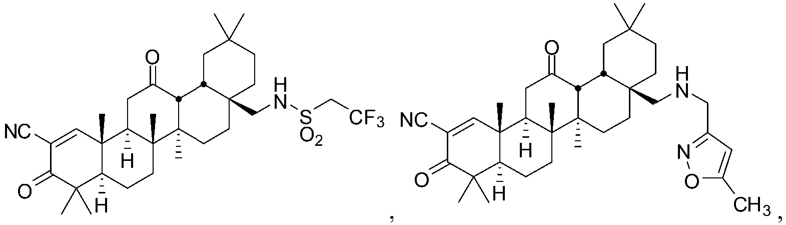

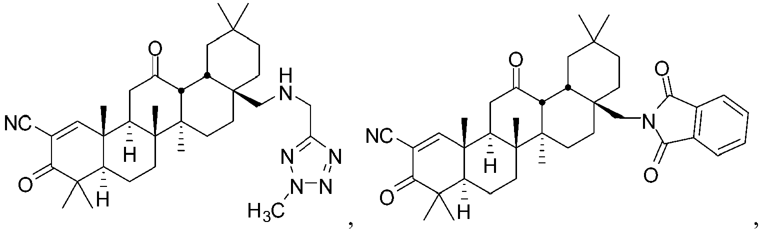

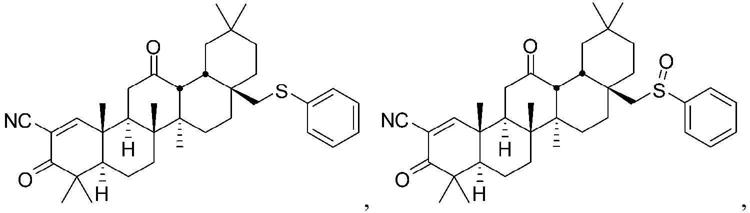

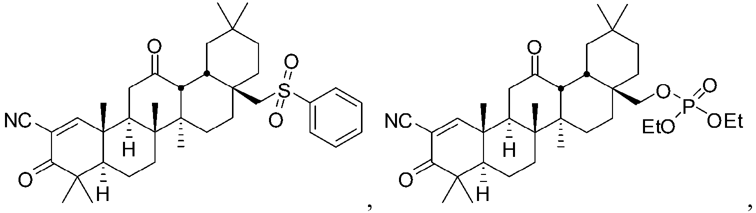

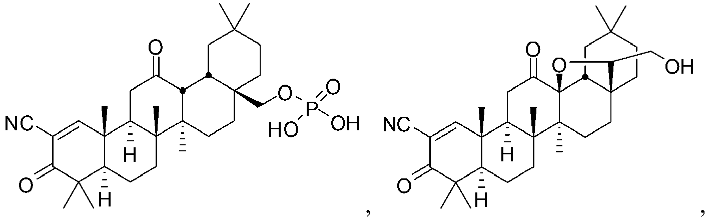

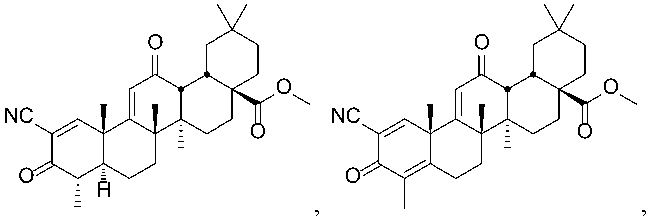

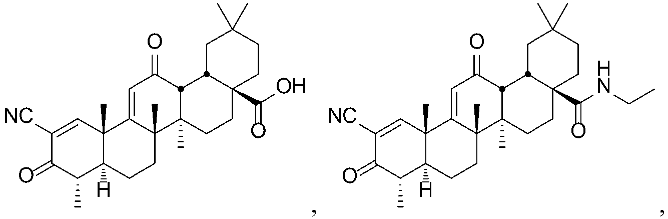

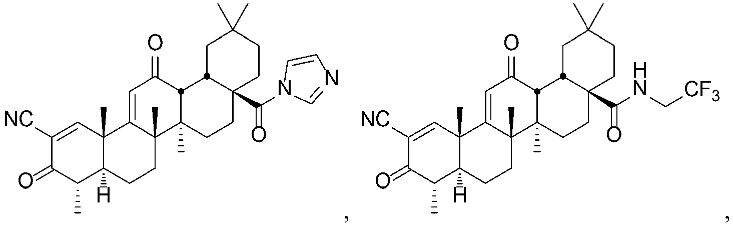

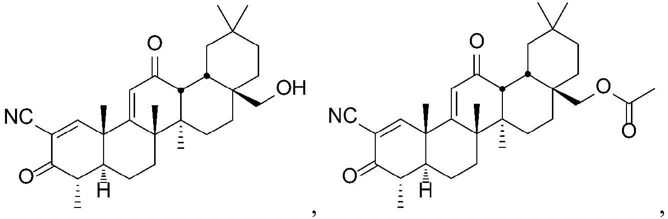

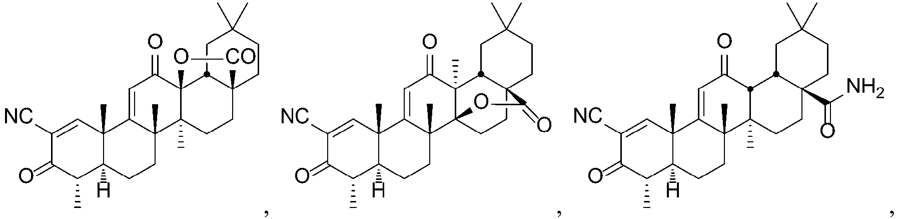

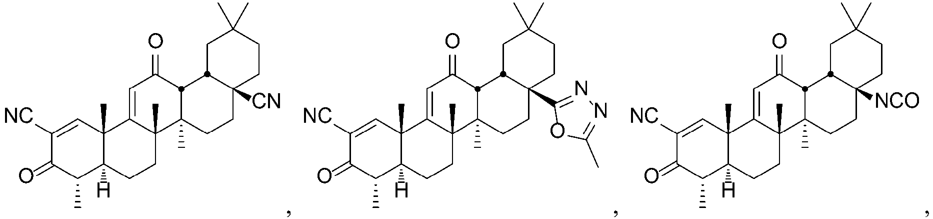

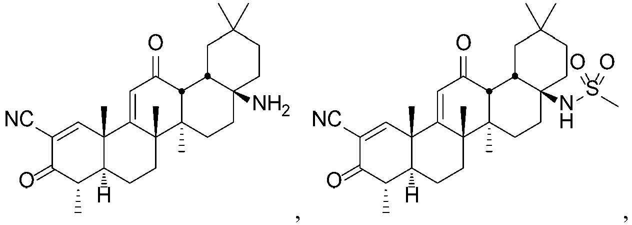

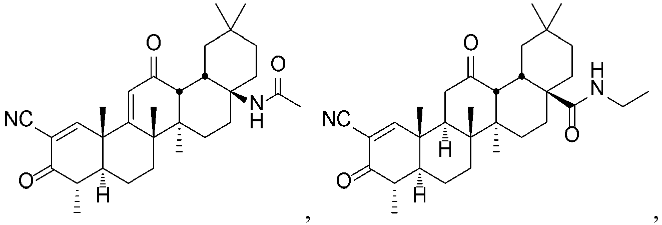

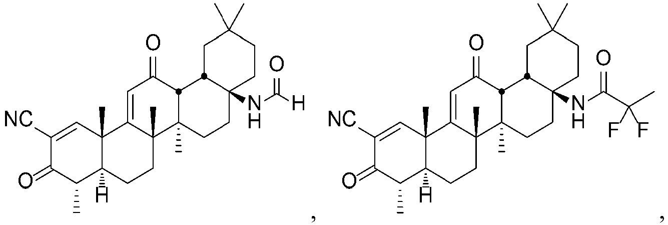

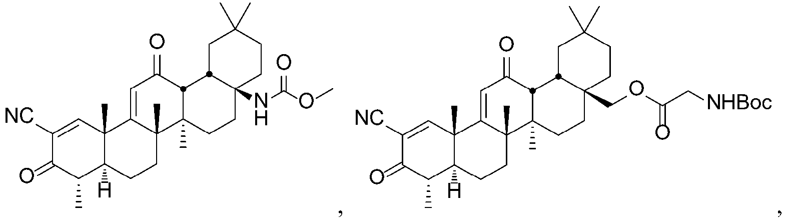

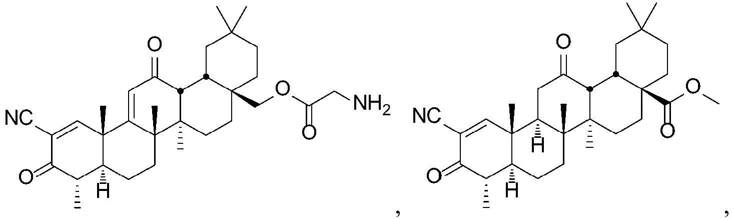

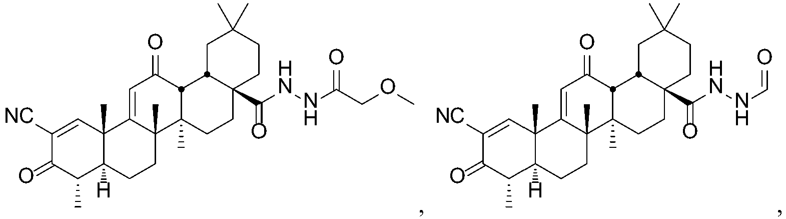

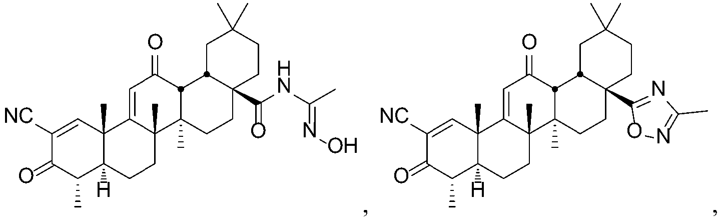

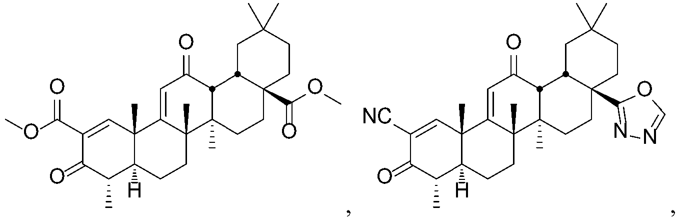

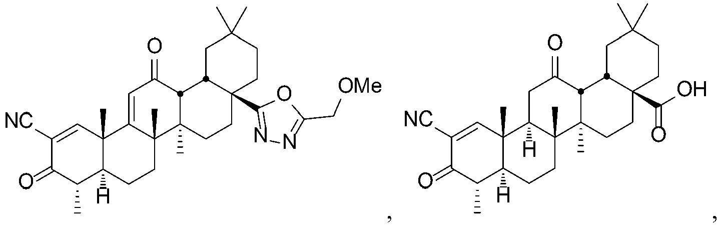

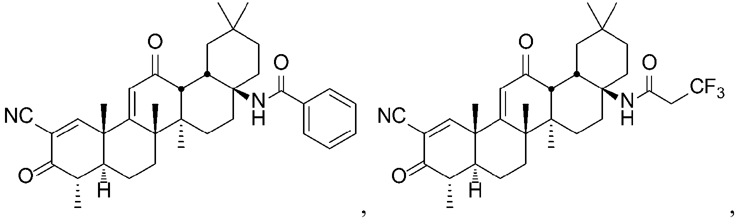

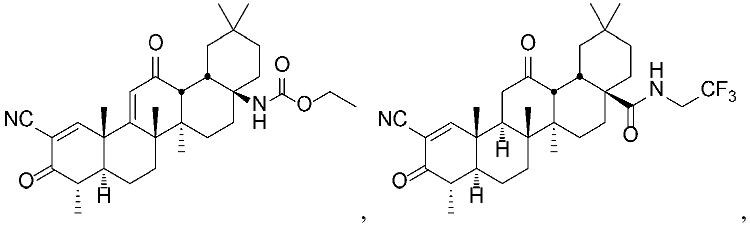

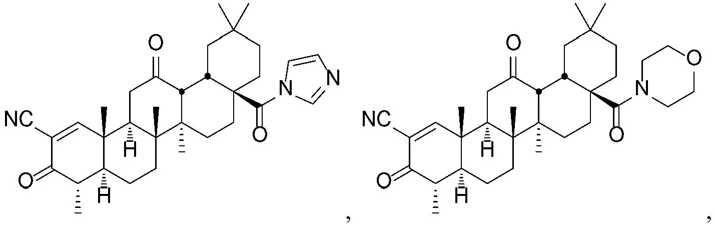

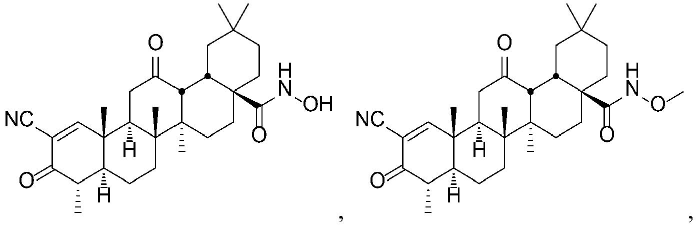

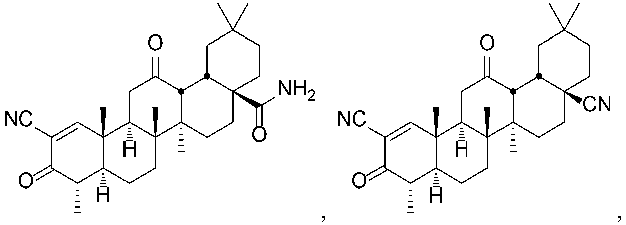

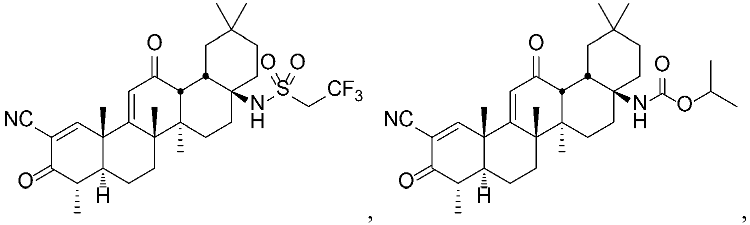

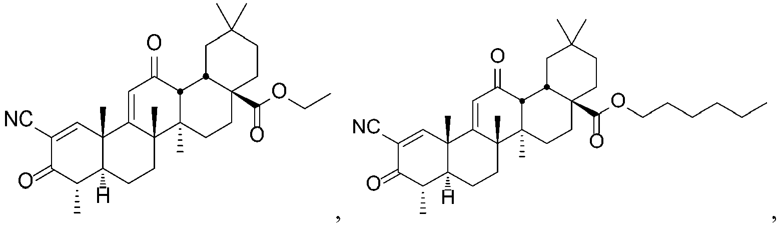

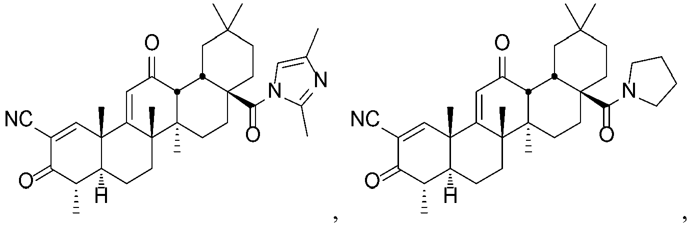

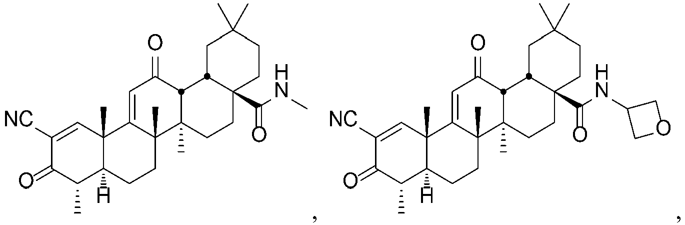

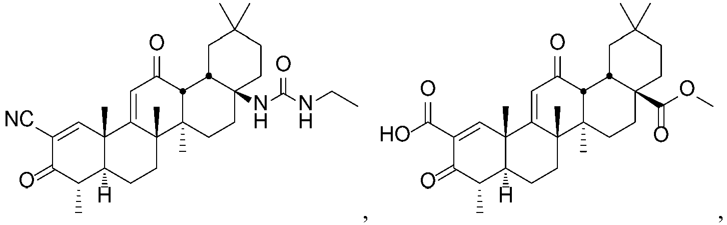

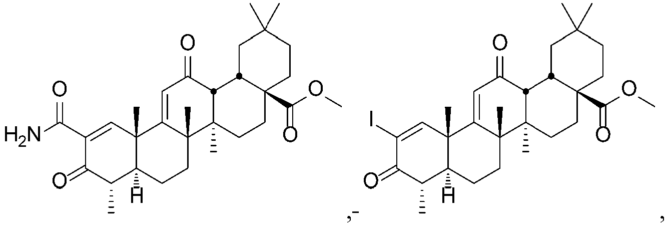

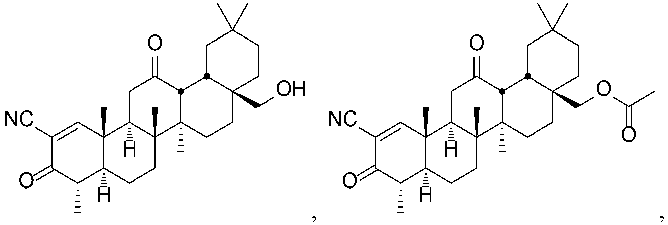

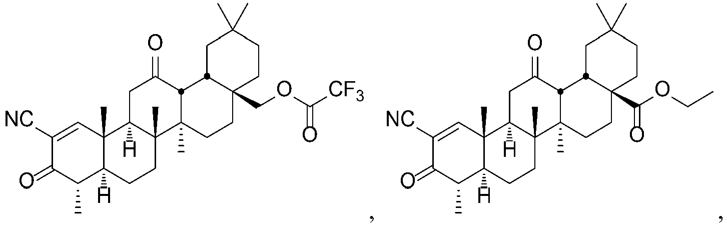

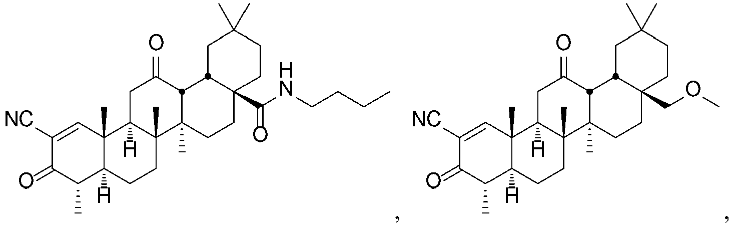

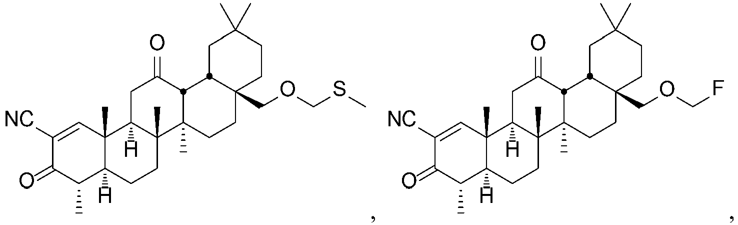

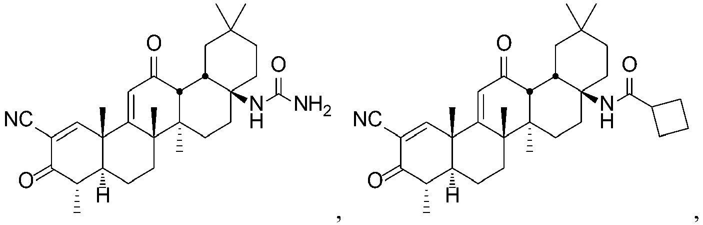

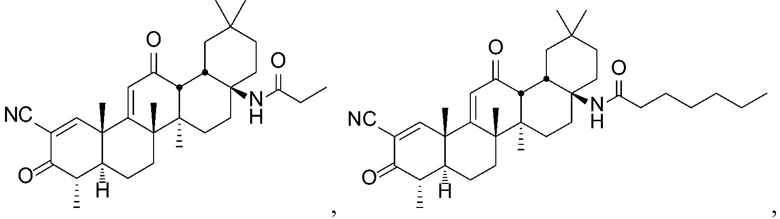

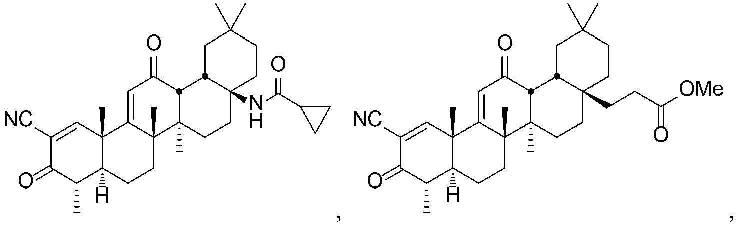

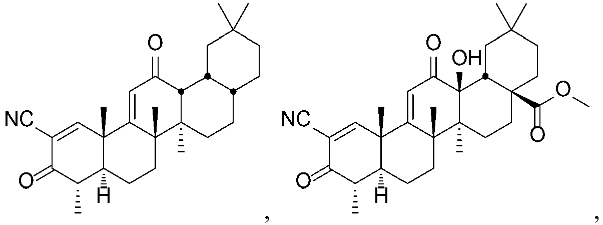

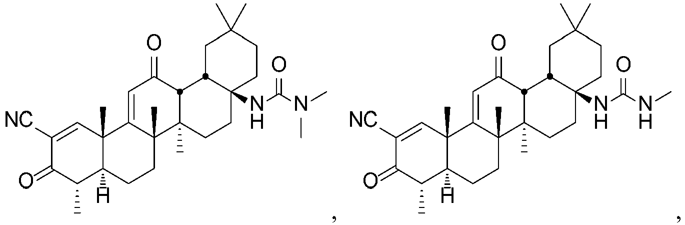

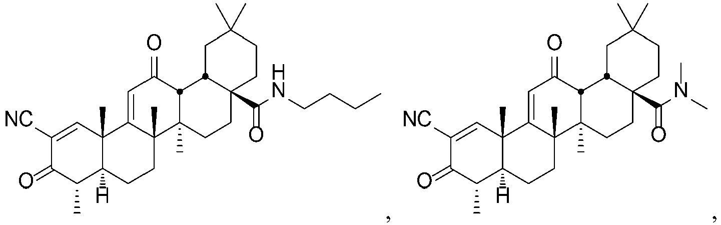

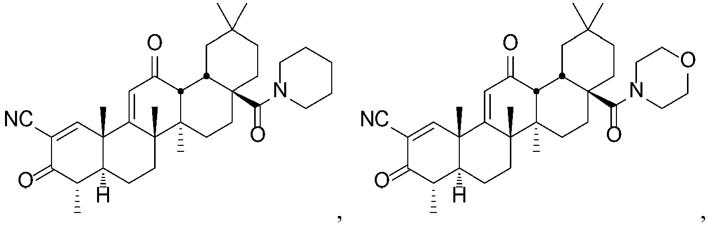

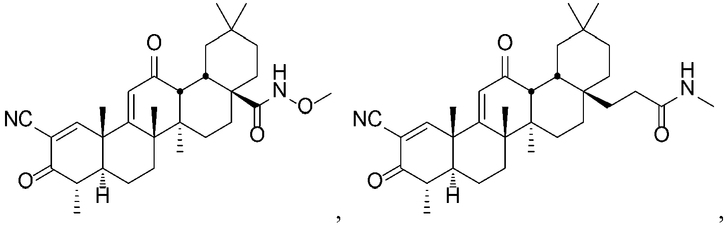

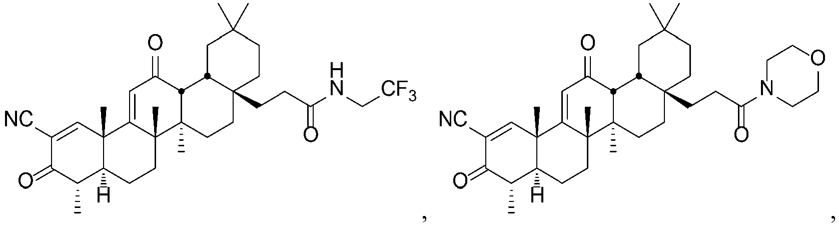

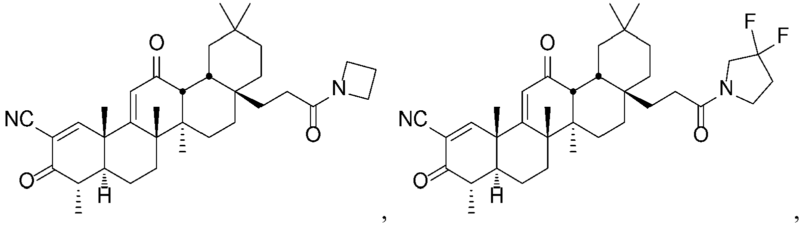

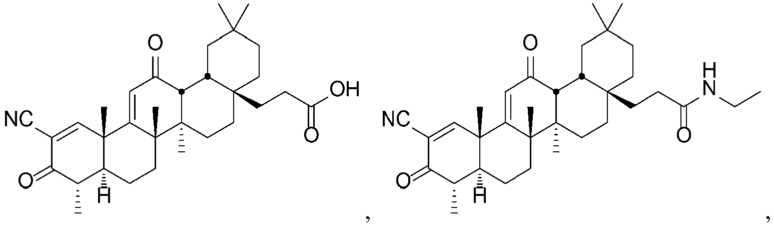

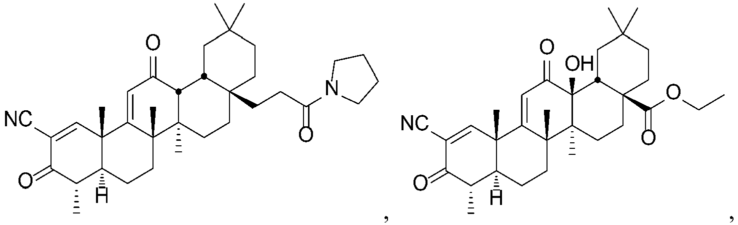

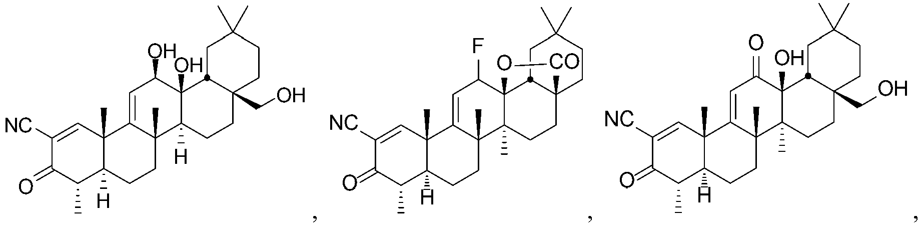

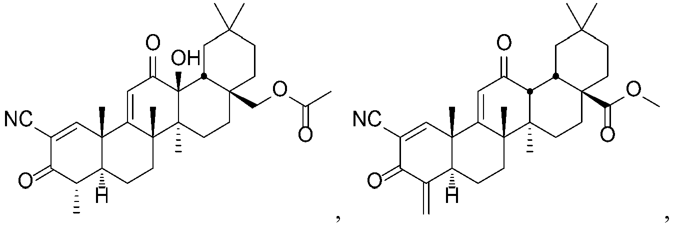

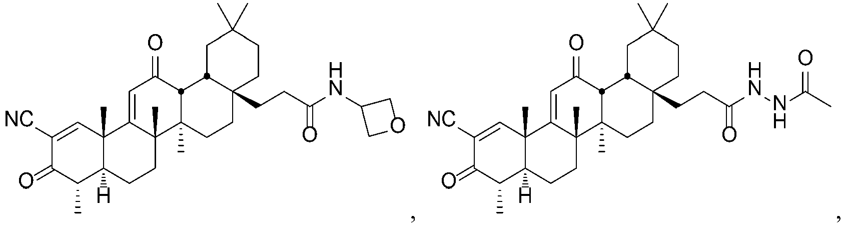

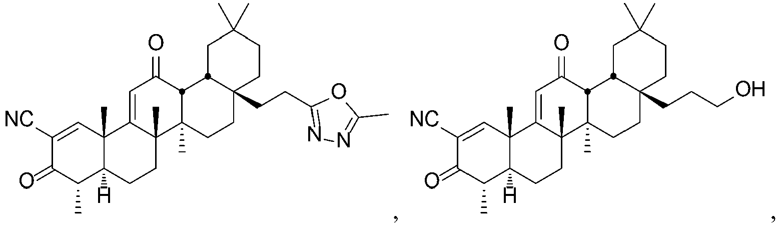

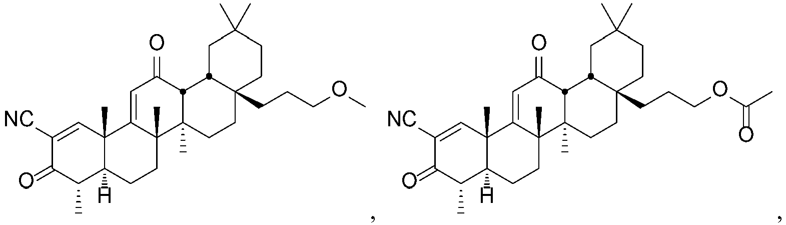

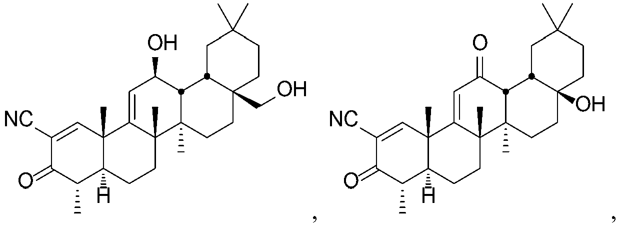

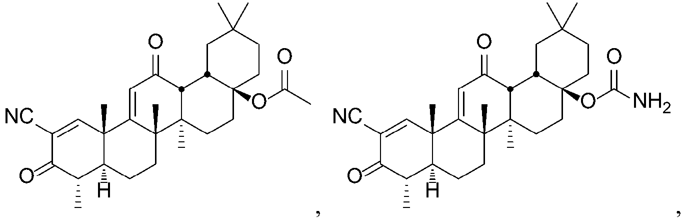

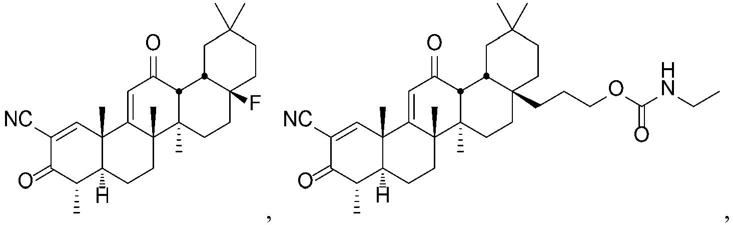

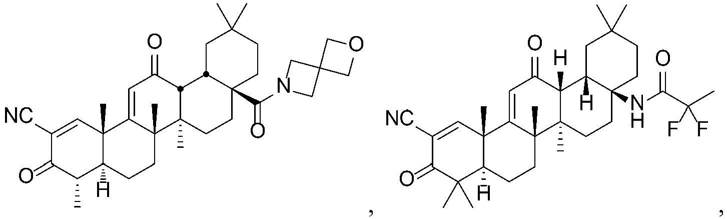

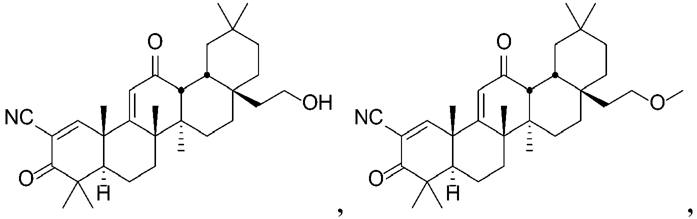

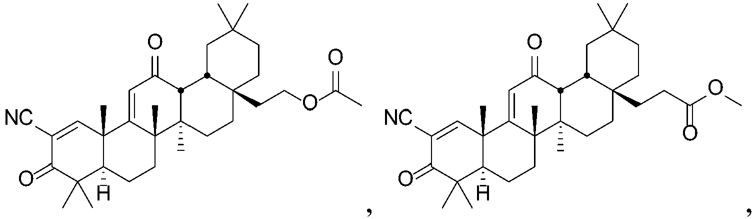

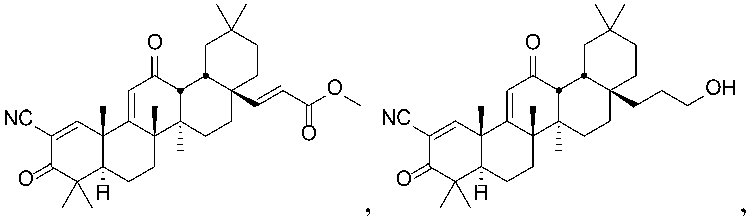

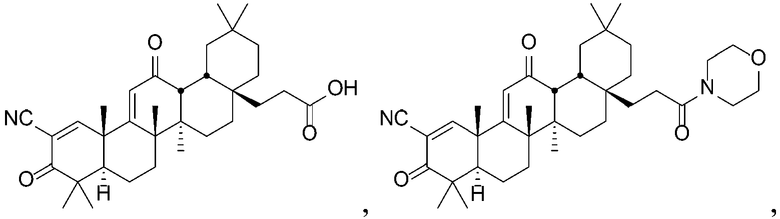

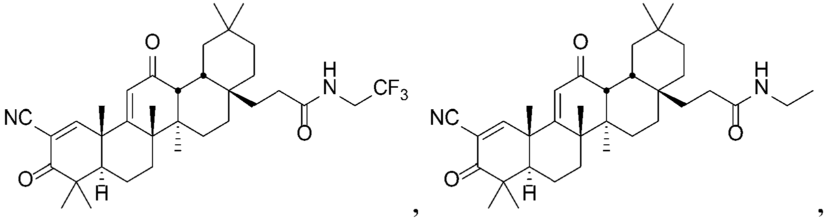

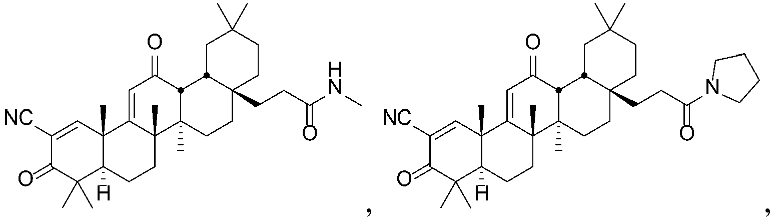

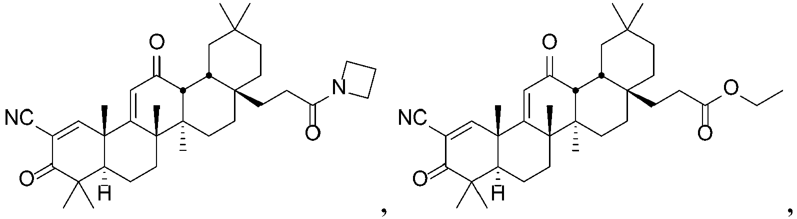

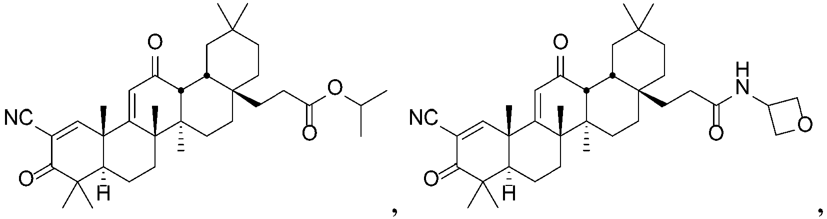

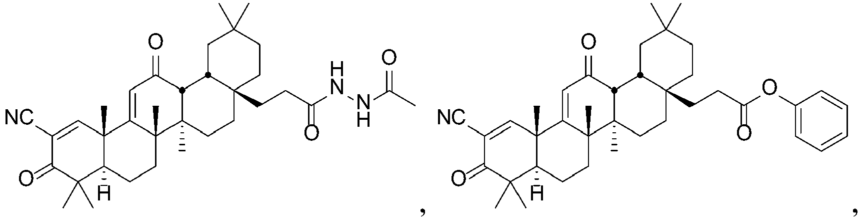

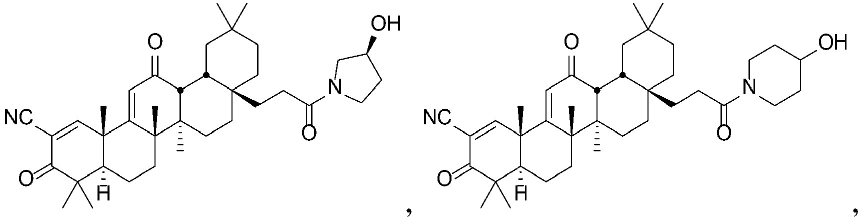

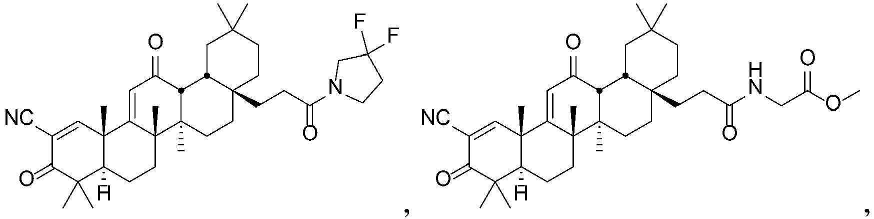

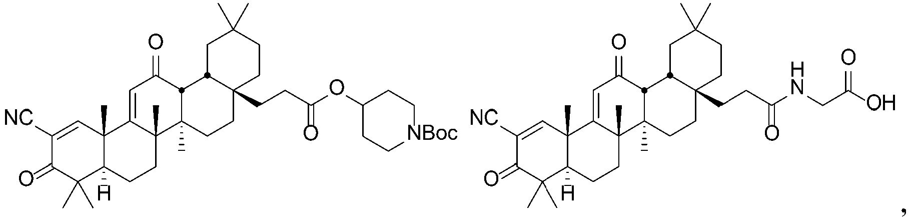

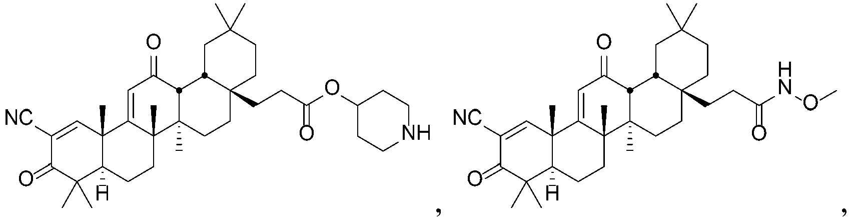

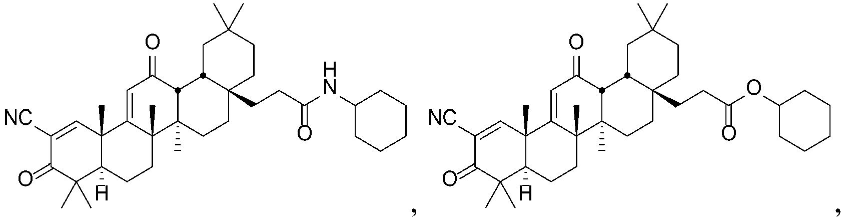

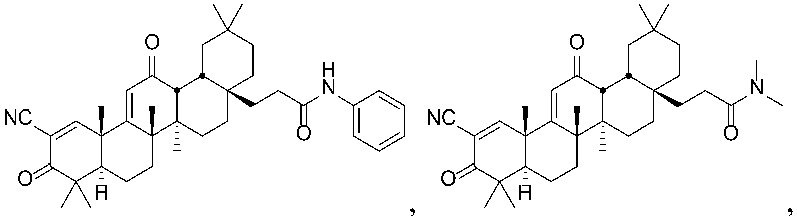

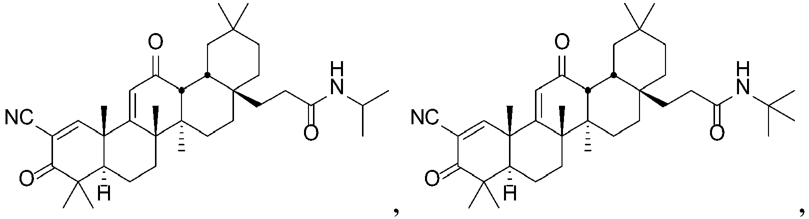

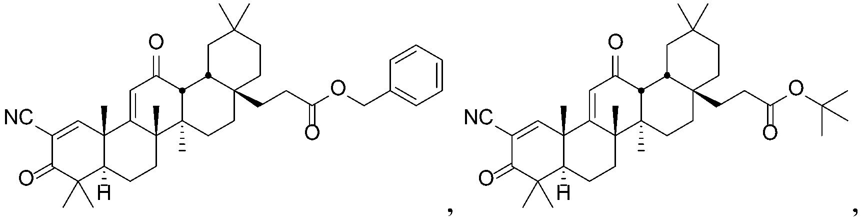

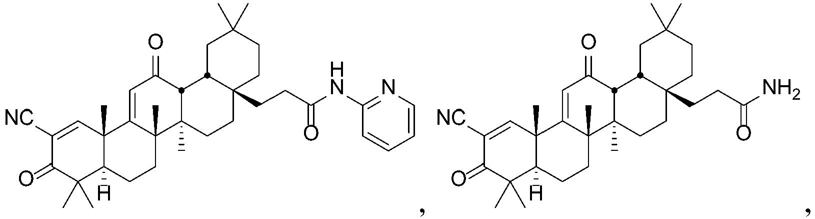

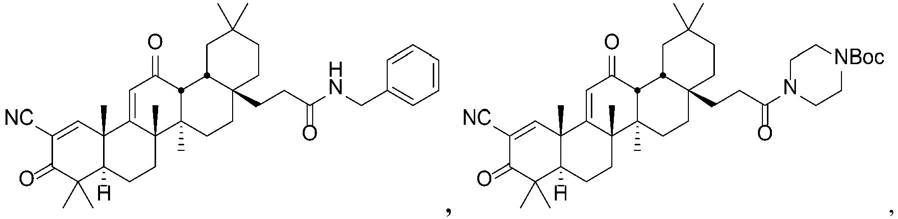

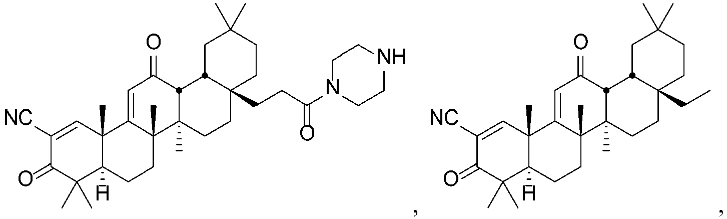

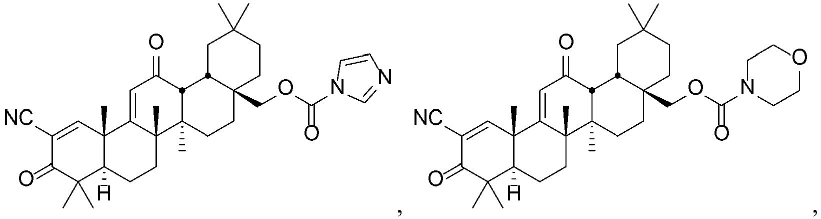

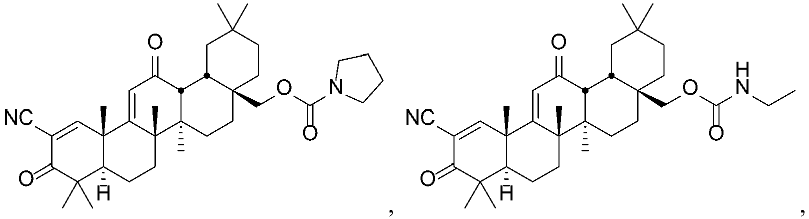

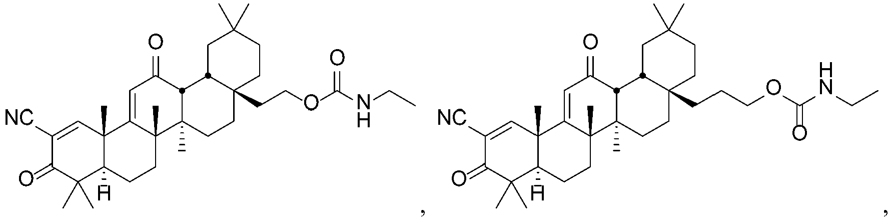

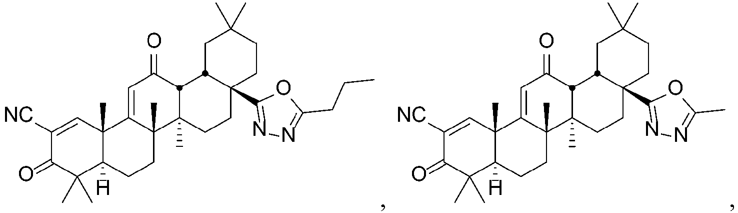

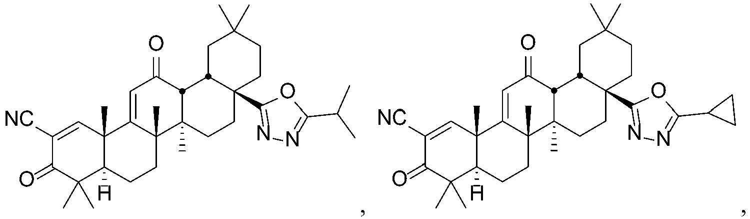

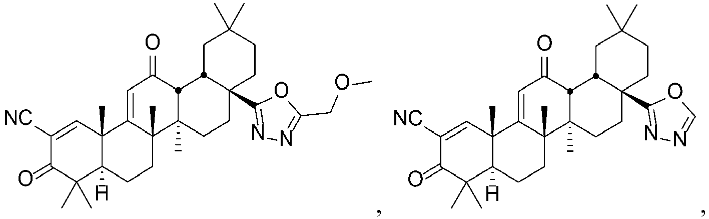

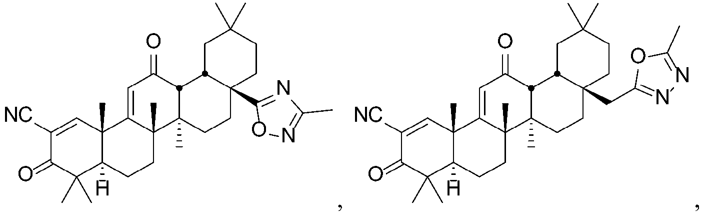

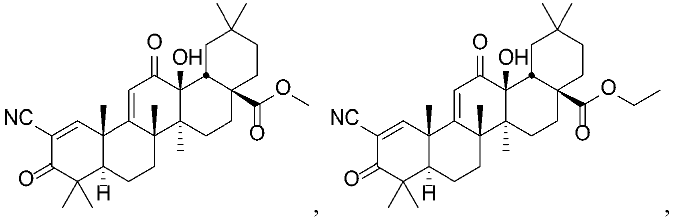

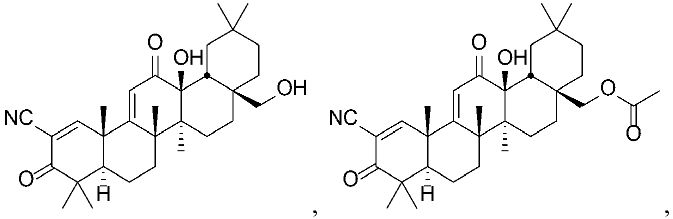

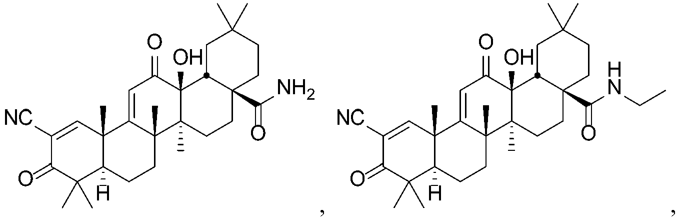

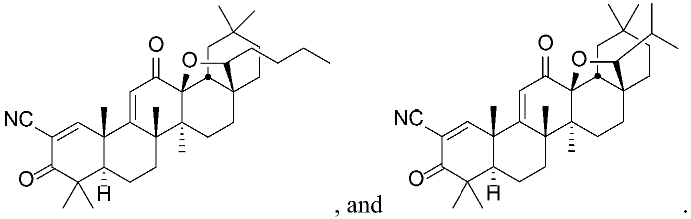

Table 21 summarizes *in vitro* results for several of these compounds in which RAW264.7 macrophages were pre-treated with DMSO or drugs at various concentrations (nM) for 2 hours, and then treated with 20 ng/mL IFNγ for 24 hours. NO concentration in the media was determined using a Griess reagent system; cell viability was determined using WST-1 reagent. NQO1 CD represents the concentration required to induce a two-fold increase in the expression of NQO1, an Nrf2-regulated antioxidant enzyme, in Hepalclc7 murine hepatoma cells (Dinkova-Kostova *et al.,* 2005). All these results are orders of magnitude more active than, for example, the parent oleanolic acid molecule. In part because induction of antioxidant pathways resulting from Nrf2 activation provides important protective effects against oxidative stress and inflammation, analogs of RTA 402 may therefore also be used to for the treatment and/or Alport syndrome or symptoms thereof or prevent the onset of symptoms of Alport syndrome.

**Table 21. Suppression of IFNy-induced NO production.**

| **Working ID** | **RAW264.7 (20 ng/mL IFNy)** | | **Hepalc1c7 cells** |
|---|---|---|---|
| | **NO IC₅₀** | **WST-1 IC₅₀** | **NQO1 CD** |
| RTA 401 | ~10 nM | > 200 nM | 2.3 nM |
| RTA 402 | 2.2 nM | 80 nM | 1.0 nM |
| RTA 403 | ~0.6 nM | 100 nM | 3.3 nM |
| RTA 404 | 5.8 nM | 100 nM | n/a |
| RTA 405 | 6 nM | ~200 nM | n/a |
| TP-225 | ~0.4 nM | 75 nM | 0.28 nM |

Without being bound by theory, the potency of the compounds of the present invention, *e.g*., RTA 402, is largely derived from the addition of α,β-unsaturated carbonyl groups. In *in vitro* assays, most activity of the compounds can be abrogated by the introduction of dithiothreitol (DTT), N-acetyl cysteine (NAC), or glutathione (GSH); thiol containing moieties that interact with α,β-unsaturated carbonyl groups (Wang *et al.,* 2000; Ikeda *et al.,* 2003; 2004; Shishodia *et al.,* 2006). Biochemical assays have established that RTA 402 directly interacts with a critical cysteine residue (C179) on IKKβ (see below) and inhibits its activity (Shishodia *et al.,* 2006; Ahmad *et al.,* 2006). IKKβ controls activation of NF-κB through the "classical" pathway which involves phosphorylation-induced degradation of IκB resulting in release of NF-κB dimers to the nucleus. In macrophages, this pathway is responsible for the production of many pro-inflammatory molecules in response to TNFα and other pro-inflammatory stimuli.

RTA 402 also inhibits the JAK/STAT signaling pathway at multiple levels. JAK proteins are recruited to transmembrane receptors (*e.g.*, IL-6R) upon activation by ligands such as interferons and interleukins. JAKs then phosphorylate the intracellular portion of the receptor causing recruitment of STAT transcription factors. The STATs are then phosphorylated by JAKs, form dimers, and translocate to the nucleus where they activate transcription of several genes involved in inflammation. RTA 402 inhibits constitutive and IL-6-induced STAT3 phosphorylation and dimer formation and directly binds to cysteine residues in STAT3 (C259) and in the kinase domain of JAK1 (C1077). Biochemical assays have also established that the triterpenoids directly interact with critical cysteine residues on Keap1 (Dinkova-Kostova *et al.,* 2005). Keap1 is an actin-tethered protein that keeps the transcription factor Nrf2 sequestered in the cytoplasm under normal conditions (Kobayashi and Yamamoto, 2005). Oxidative stress results in oxidation of the regulatory cysteine residues on Keap1 and causes the release of Nrf2. Nrf2 then translocates to the nucleus and binds to antioxidant response elements (AREs) resulting in transcriptional activation of many antioxidant and anti-inflammatory genes. Another target of the Keapl/Nrf2/ARE pathway is heme oxygenase 1 (HO-1). HO-1 breaks down heme into bilirubin and carbon monoxide and plays many antioxidant and anti-inflammatory roles (Maines and Gibbs, 2005). HO-1 has recently been shown to be potently induced by the triterpenoids (Liby *et al.,* 2005), including RTA 402. RTA 402 and many structural analogs have also been shown to be potent inducers of the expression of other Phase 2 proteins (Yates *et al.,* 2007). RTA 402 is a potent inhibitor of NF-κB activation. Furthermore, RTA 402 activates the Keap1/Nrf2/ARE pathway and induces expression of HO-1.

Compounds employed may be made using the methods described by Honda *et al.* (2000a); Honda *et al.* (2000b); Honda *et al.* (2002); and U.S. Patent Application Publications 2009/0326063, 2010/0056777, 2010/0048892, 2010/0048911, 2010/0041904, 2003/0232786, 2008/0261985 and 2010/0048887, all of which are incorporated by reference herein. These methods can be further modified and optimized using the principles and techniques of organic chemistry as applied by a person skilled in the art. Such principles and techniques are taught, for example, in Smith, March's Advanced Organic Chemistry: Reactions, Mechanisms, and Structure (2013), which is incorporated by reference herein. In addition, the synthetic methods may be further modified and optimized for preparative, pilot- or large-scale production, either batch of continuous, using the principles and techniques of process chemistry as applied by a person skilled in the art. Such principles and techniques are taught, for example, in Anderson, Practical Process Research & Development - A Guide for Organic Chemists (2012), which is incorporated by reference herein.

Compounds of the present invention may contain one or more asymmetrically-substituted carbon or nitrogen atoms, and may be isolated in optically active or racemic form. Thus, all chiral, diastereomeric, racemic form, epimeric form, and all geometric isomeric forms of a chemical formula are intended, unless the specific stereochemistry or isomeric form is specifically indicated. Compounds may occur as racemates and racemic mixtures, single enantiomers, diastereomeric mixtures and individual diastereomers. In some embodiments, a single diastereomer is obtained. The chiral centers of the compounds of the present invention can have the S or the R configuration.

Chemical formulas used to represent compounds of the present invention will typically only show one of possibly several different tautomers. For example, many types of ketone groups are known to exist in equilibrium with corresponding enol groups. Similarly, many types of imine groups exist in equilibrium with enamine groups. Regardless of which tautomer is depicted for a given compound, and regardless of which one is most prevalent, all tautomers of a given chemical formula are intended.

In addition, atoms making up the compounds of the present invention are intended to include all isotopic forms of such atoms. Isotopes, as used herein, include those atoms having the same atomic number but different mass numbers. By way of general example and without limitation, isotopes of hydrogen include tritium and deuterium, and isotopes of carbon include ¹³C and ¹⁴C.

Polymorphic forms of the compounds of the present invention, e.g., Forms A and B of CDDO-Me, may be used in accordance with the methods of this inventions. Form B displays a bioavailability that is surprisingly better than that of Form A. Specifically the bioavailability of Form B was higher than that of Form A CDDO-Me in monkeys when the monkeys received equivalent dosages of the two forms orally, in gelatin capsules. See U.S. Patent Application Publication 2009/0048204, which is incorporated by reference herein in its entirety.

"Form A" of CDDO-Me (RTA 402) is unsolvated (non-hydrous) and can be characterized by a distinctive crystal structure, with a space group of P4₃ 2₁2 (no. 96) unit cell dimensions of a = 14.2 Å, b = 14.2 Å and c = 81.6 A, and by a packing structure, whereby three molecules are packed in helical fashion down the crystallographic b axis. In some embodiments, Form A can also be characterized by X-ray powder diffraction (XRPD) pattern (CuKα) comprising significant diffraction peaks at about 8.8, 12.9, 13.4, 14.2 and 17.4 °θ. In some variations, the X-ray powder diffraction of Form A is substantially as shown in FIG. 1A or FIG. 1B.

Unlike Form A, "Form B" of CDDO-Me is in a single phase but lacks such a defined crystal structure. Samples of Form B show no long-range molecular correlation, *i.e.,* above roughly 20 Å. Moreover, thermal analysis of Form B samples reveals a glass transition temperature (T_{g}) in a range from about 120°C to about 130°C. In contrast, a disordered nanocrystalline material does not display a T_{g} but instead only a melting temperature (Tₘ), above which crystalline structure becomes a liquid. Form B is typified by an XRPD spectrum (FIG. 1C) differing from that of Form A (FIG. 1A or FIG. 1B). Since it does not have a defined crystal structure, Form B likewise lacks distinct XRPD peaks, such as those that typify Form A, and instead is characterized by a general "halo" XRPD pattern. In particular, the non-crystalline Form B falls into the category of "X-ray amorphous" solids because its XRPD pattern exhibits three or fewer primary diffraction halos. Within this category, Form B is a "glassy" material.

Form A and Form B of CDDO-Me are readily prepared from a variety of solutions of the compound. For example, Form B can be prepared by fast evaporation or slow evaporation in MTBE, THF, toluene, or ethyl acetate. Form A can be prepared in several ways, including *via* fast evaporation, slow evaporation, or slow cooling of a CDDO-Me solution in ethanol or methanol. Preparations of CDDO-Me in acetone can produce either Form A, using fast evaporation, or Form B, using slow evaporation.

Various means of characterization can be used together to distinguish Form A and Form B CDDO-Me from each other and from other forms of CDDO-Me. Illustrative of the techniques suitable for this purpose are solid state Nuclear Magnetic Resonance (NMR), X-ray powder diffraction (compare FIGS. 1A & B with FIG. 1C), X-ray crystallography, differential scanning calorimetry (DSC), dynamic vapor sorption/desorption (DVS), Karl Fischer analysis (KF), hot stage microscopy, modulated differential screening calorimetry, FT-IR, and Raman spectroscopy. In particular, analysis of the XRPD and DSC data can distinguish Form A, Form B, and a hemibenzenate form of CDDO-Me. See U.S. Patent Application Publication 2009/0048204, which is incorporated by reference herein in its entirety.

Additional details regarding polymorphic forms of CDDO-Me are described in U.S. Patent Application Publication 2009/0048204, PCT Publication WO 2009/023232 and PCT Publication WO 2010/093944, which are all incorporated herein by reference in their entireties.

Non-limiting specific formulations of the compounds disclosed herein include CDDO-Me polymer dispersions. See, for example, PCT Publication WO 2010/093944, which is incorporated herein by reference in its entirety. Some of the formulations reported therein exhibit higher bioavailability than either the micronized Form A or nanocrystalline Form A formulations. Additionally, the polymer dispersion based formulations demonstrate further surprising improvements in oral bioavailability relative to the micronized Form B formulations. For example, the methacrylic acid copolymer, Type C and HPMC-P formulations showed the greatest bioavailability in the subject monkeys.

Compounds employed in methods of the invention may also exist in prodrug form. Since prodrugs enhance numerous desirable qualities of pharmaceuticals, e.g., solubility, bioavailability, manufacturing, *etc.,* the compounds employed in some methods of the invention may, if desired, be delivered in prodrug form. Thus, the invention contemplates prodrugs of compounds of the present invention as well as methods of delivering prodrugs. Prodrugs of the compounds employed in the invention may be prepared by modifying functional groups present in the compound in such a way that the modifications are cleaved, either in routine manipulation or *in vivo,* to the parent compound. Accordingly, prodrugs include, for example, compounds described herein in which a hydroxy, amino, or carboxy group is bonded to any group that, when the prodrug is administered to a subject or patient, cleaves to form a hydroxy, amino, or carboxylic acid, respectively.

It should be recognized that the particular anion or cation forming a part of any salt of this invention is not critical, so long as the salt, as a whole, is pharmacologically acceptable. Examples of pharmaceutically acceptable salts and their methods of preparation and use are presented in Handbook of Pharmaceutical Salts: Properties, and Use (2002), which is incorporated herein by reference.

In some embodiments, the compounds employed in the methods described in the present invention have the advantage that they may be more efficacious than, be less toxic than, be longer acting than, be more potent than, produce fewer side effects than, be more easily absorbed than, and/or have a better pharmacokinetic profile *(e.g.,* higher oral bioavailability and/or lower clearance) than, and/or have other useful pharmacological, physical, or chemical properties over, compounds known in the prior art, whether for use in the indications stated herein or otherwise.

### III. Pharmaceutical Formulations and Routes of Administration

Administration of the compounds of the present invention to a patient will follow general protocols for the administration of pharmaceuticals, taking into account the toxicity, if any, of the drug. It is expected that the treatment cycles would be repeated as necessary.

The compounds of the present invention may be administered by a variety of methods, *e.g.,* orally or by injection (*e.g.,* subcutaneous, intravenous, intraperitoneal, *etc*.)*.* Depending on the route of administration, the active compounds may be coated by a material to protect the compound from the action of acids and other natural conditions which may inactivate the compound. They may also be administered by continuous perfusion/infusion of a disease or wound site. Specific examples of formulations, including a polymer-based dispersion of CDDO-Me that showed improved oral bioavailability, are provided in U.S. Patent Application Publication No. 2009/0048204, which is incorporated herein by reference in its entirety. It will be recognized by those skilled in the art that other methods of manufacture may be used to produce dispersions of the present invention with equivalent properties and utility (*see,* Repka *et al.,* 2002 and references cited therein). Such alternative methods include but are not limited to solvent evaporation, extrusion, such as hot melt extrusion, and other techniques.

To administer the active compound by other than parenteral administration, it may be necessary to coat the compound with, or co-administer the compound with, a material to prevent its inactivation. For example, the active compound may be administered to a patient in an appropriate carrier, for example, liposomes, or a diluent. Pharmaceutically acceptable diluents include saline and aqueous buffer solutions. Liposomes include water-in-oil-in-water CGF emulsions as well as conventional liposomes.

The therapeutic compound may also be administered parenterally, intraperitoneally, intraspinally, or intracerebrally. Dispersions may be prepared in, *e.g.,* glycerol, liquid polyethylene glycols, mixtures thereof, and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The carrier may be a solvent or dispersion medium containing, for example, water, ethanol, polyol (such as, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, sodium chloride, or polyalcohols such as mannitol and sorbitol, in the composition. Prolonged absorption of the injectable compositions can be brought about by including in the composition an agent which delays absorption, for example, aluminum monostearate or gelatin.

Sterile injectable solutions can be prepared by incorporating the therapeutic compound in the required amount in an appropriate solvent with one or a combination of ingredients enumerated above, as required, followed by filtered sterilization. Generally, dispersions are prepared by incorporating the therapeutic compound into a sterile carrier which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (*i.e.,* the therapeutic compound) plus any additional desired ingredient from a previously sterile-filtered solution thereof.

The therapeutic compound can be orally administered, for example, with an inert diluent or an assimilable edible carrier. The therapeutic compound and other ingredients may also be enclosed in a hard or soft shell gelatin capsule, compressed into tablets, or incorporated directly into the subject's or patient's diet. For oral therapeutic administration, the therapeutic compound may be incorporated with excipients and used in the form of ingestible tablets, buccal tablets, troches, capsules, elixirs, suspensions, syrups, wafers, and the like. The percentage of the therapeutic compound in the compositions and preparations may, of course, be varied. The amount of the therapeutic compound in such therapeutically useful compositions is such that a suitable dosage will be obtained.

It is especially advantageous to formulate parenteral compositions in dosage unit form for ease of administration and uniformity of dosage. Dosage unit form as used herein refers to physically discrete units suited as unitary dosages for the subjects or patients to be treated; each unit containing a predetermined quantity of therapeutic compound calculated to produce the desired therapeutic effect in association with the required pharmaceutical carrier. The specification for the dosage unit forms of the invention are dictated by and directly dependent on (a) the unique characteristics of the therapeutic compound and the particular therapeutic effect to be achieved, and (b) the limitations inherent in the art of compounding such a therapeutic compound for the treatment of a selected condition in a patient.

The therapeutic compound may also be administered topically to the skin, eye, or mucosa. Alternatively, if local delivery to the lungs is desired the therapeutic compound may be administered by inhalation in a dry-powder or aerosol formulation.

The therapeutic compound may be formulated in a biocompatible matrix for use in a drug-eluting stent.

In some embodiments, the effective dose range for the therapeutic compound can be extrapolated from effective doses determined in animal studies for a variety of different animals. In general a human equivalent dose (HED) in mg/kg can be calculated in accordance with the following formula (*see, e.g.,* Reagan-Shaw *et al., FASEB J.,* 22(3):659-661, 2008, which is incorporated herein by reference): $HED \left(mg/kg\right) = Animal dose \left(mg/kg\right) \times \left(Animal {K}_{m}/Human {K}_{m}\right)$

Use of the Kₘ factors in conversion results in more accurate HED values, which are based on body surface area (BSA) rather than only on body mass. Kₘ values for humans and various animals are well known. For example, the Kₘ for an average 60 kg human (with a BSA of 1.6 m²) is 37, whereas a 20 kg child (BSA 0.8 m²) would have a Kₘ of 25. Kₘ for some relevant animal models are also well known, including: mice Kₘ of 3 (given a weight of 0.02 kg and BSA of 0.007); hamster Kₘ of 5 (given a weight of 0.08 kg and BSA of 0.02); rat Kₘ of 6 (given a weight of 0.15 kg and BSA of 0.025) and monkey Kₘ of 12 (given a weight of 3 kg and BSA of 0.24).

Precise amounts of the therapeutic composition depend on the judgment of the practitioner and are peculiar to each individual. Nonetheless, a calculated HED dose provides a general guide. Other factors affecting the dose include the physical and clinical state of the patient, the route of administration, the intended goal of treatment and the potency, stability and toxicity of the particular therapeutic formulation.

The actual dosage amount of a compound of the present invention or composition comprising a compound of the present invention administered to a subject or a patient may be determined by physical and physiological factors such as age, sex, body weight, severity of condition, the type of disease being treated, previous or concurrent therapeutic interventions, idiopathy of the subject or the patient and on the route of administration. These factors may be determined by a skilled artisan. The practitioner responsible for administration will typically determine the concentration of active ingredient(s) in a composition and appropriate dose(s) for the individual subject or patient. The dosage may be adjusted by the individual physician in the event of any complication.

In some embodiments, the pharmaceutically effective amount is a daily dose from about 0.1 mg to about 500 mg of the compound. In some variations, the daily dose is from about 1 mg to about 300 mg of the compound. In some variations, the daily dose is from about 10 mg to about 200 mg of the compound. In some variations, the daily dose is about 25 mg of the compound. In other variations, the daily dose is about 75 mg of the compound. In still other variations, the daily dose is about 150 mg of the compound. In further variations, the daily dose is from about 0.1 mg to about 30 mg of the compound. In some variations, the daily dose is from about 0.5 mg to about 20 mg of the compound. In some variations, the daily dose is from about 1 mg to about 15 mg of the compound. In some variations, the daily dose is from about 1 mg to about 10 mg of the compound. In some variations, the daily dose is from about 1 mg to about 5 mg of the compound.

In some embodiments, the pharmaceutically effective amount is a daily dose of 0.01 - 25 mg of compound per kg of body weight. In some variations, the daily dose is 0.05 - 20 mg of compound per kg of body weight. In some variations, the daily dose is 0.1 - 10 mg of compound per kg of body weight. In some variations, the daily dose is 0.1 - 5 mg of compound per kg of body weight. In some variations, the daily dose is 0.1 - 2.5 mg of compound per kg of body weight.

In some embodiments, the pharmaceutically effective amount is a daily dose of 0.1 - 1000 mg of compound per kg of body weight. In some variations, the daily dose is 0.15 - 20 mg of compound per kg of body weight. In some variations, the daily dose is 0.20 - 10 mg of compound per kg of body weight. In some variations, the daily dose is 0.40 - 3 mg of compound per kg of body weight. In some variations, the daily dose is 0.50 - 9 mg of compound per kg of body weight. In some variations, the daily dose is 0.60 - 8 mg of compound per kg of body weight. In some variations, the daily dose is 0.70 - 7 mg of compound per kg of body weight. In some variations, the daily dose is 0.80 - 6 mg of compound per kg of body weight. In some variations, the daily dose is 0.90 - 5 mg of compound per kg of body weight. In some variations, the daily dose is from about 1 mg to about 5 mg of compound per kg of body weight.

An effective amount typically will vary from about 0.001 mg/kg to about 1,000 mg/kg, from about 0.01 mg/kg to about 750 mg/kg, from about 0.1 mg/kg to about 500 mg/kg, from about 0.2 mg/kg to about 250 mg/kg, from about 0.3 mg/kg to about 150 mg/kg, from about 0.3 mg/kg to about 100 mg/kg, from about 0.4 mg/kg to about 75 mg/kg, from about 0.5 mg/kg to about 50 mg/kg, from about 0.6 mg/kg to about 30 mg/kg, from about 0.7 mg/kg to about 25 mg/kg, from about 0.8 mg/kg to about 15 mg/kg, from about 0.9 mg/kg to about 10 mg/kg, from about 1 mg/kg to about 5 mg/kg, from about 100 mg/kg to about 500 mg/kg, from about 1.0 mg/kg to about 250 mg/kg, or from about 10.0 mg/kg to about 150 mg/kg, in one or more dose administrations daily, for one or several days (depending, of course, of the mode of administration and the factors discussed above). Other suitable dose ranges include 1 mg to 10,000 mg per day, 100 mg to 10,000 mg per day, 500 mg to 10,000 mg per day, and 500 mg to 1,000 mg per day. In some particular embodiments, the amount is less than 10,000 mg per day with a range, for example, of 750 mg to 9,000 mg per day.

The effective amount may be less than 1 mg/kg/day, less than 500 mg/kg/day, less than 250 mg/kg/day, less than 100 mg/kg/day, less than 50 mg/kg/day, less than 25 mg/kg/day, less than 10 mg/kg/day, or less than 5 mg/kg/day. It may alternatively be in the range of 1 mg/kg/day to 200 mg/kg/day. For example, regarding treatment of patients with Alport syndrome, the unit dosage may be an amount that reduces urine protein concentration by at least 40% as compared to an untreated subject or patient. In another embodiment, the unit dosage is an amount that reduces urine protein concentration to a level that is within ±10% of the urine protein level of a healthy subject or patient.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 1 mg/kg/body weight to about 5 mg/kg/body weight, a range of about 5 mg/kg/body weight to about 100 mg/kg/body weight, about 5 microgram/kg/body weight to about 500 milligram/kg/body weight, *etc.,* can be administered, based on the numbers described above.

In certain embodiments, a pharmaceutical composition of the present invention may comprise, for example, at least about 0.1% of a compound of the present invention. In other embodiments, the compound of the present invention may comprise between about 2% to about 75% of the weight of the unit, or between about 25% to about 60%, for example, and any range derivable therein.

Single or multiple doses of the agents are contemplated. Desired time intervals for delivery of multiple doses can be determined by one of ordinary skill in the art employing no more than routine experimentation. As an example, subjects or patients may be administered two doses daily at approximately 12 hour intervals. In some embodiments, the agent is administered once a day.

The agent(s) may be administered on a routine schedule. As used herein a routine schedule refers to a predetermined designated period of time. The routine schedule may encompass periods of time which are identical or which differ in length, as long as the schedule is predetermined. For instance, the routine schedule may involve administration twice a day, every day, every two days, every three days, every four days, every five days, every six days, a weekly basis, a monthly basis or any set number of days or weeks therebetween. Alternatively, the predetermined routine schedule may involve administration on a twice daily basis for the first week, followed by a daily basis for several months, *etc.* In other embodiments, the invention provides that the agent(s) may be taken orally and that the timing of which is or is not dependent upon food intake. Thus, for example, the agent can be taken every morning and/or every evening, regardless of when the subject or patient has eaten or will eat.

Non-limiting specific formulations include CDDO-Me polymer dispersions (see U.S. Patent Application Publication No. 2009/0048204, filed August 13, 2008, which is incorporated herein by reference). Some of the formulations reported therein exhibited higher bioavailability than either the micronized Form A or nanocrystalline Form A formulations. Additionally, the polymer dispersion based formulations demonstrated further surprising improvements in oral bioavailability relative to the micronized Form B formulations. For example, the methacrylic acid copolymer, Type C and HPMC-P formulations showed the greatest bioavailability in the subject monkeys.

### IV. Combination Therapy

In addition to being used as a monotherapy, the compounds of the present invention may also find use in combination therapies. Effective combination therapy may be achieved with a single composition or pharmacological formulation that includes both agents, or with two distinct compositions or formulations, administered at the same time, wherein one composition includes a compound of this invention, and the other includes the second agent(s). Alternatively, the therapy may precede or follow the other agent treatment by intervals ranging from minutes to months.

Various combinations may be employed, such as when a compound of the present invention is "A" and "B" represents a secondary agent, non-limiting examples of which are described below:

| | | | | | | | |
|---|---|---|---|---|---|---|---|
| A/B/A | B/A/B | B/B/A | A/A/B | A/B/B | B/A/A | A/B/B/B | B/A/B/B |
| B/B/B/A | B/B/A/B | | A/A/B/B | A/B/A/B | | A/B/B/A | B/B/A/A |
| B/A/B/A | B/A/A/B | | A/A/A/B | B/A/A/A | | A/B/A/A | A/A/B/A |

It is contemplated that other therapeutic agents may be used in conjunction with the treatments of the current invention. In some embodiments, the present invention contemplates the use of one or more other therapies for the treatment of Alport syndrome in conjunction with the compounds described in the methods therein. These therapies include the use of an angiotensin-converting enzyme (ACE) inhibitor, angiotensin receptor blockade (ARB), or an aldosterone antagonist. Some non-limiting examples of ACE inhibitor include Ramipril, enalapril, Lisinopril, benazepril, fosinopril, quinapril, cilazapril, perinopril, or trandolapril. Similarly, some non-limiting examples of angiotensin receptor blockade agent include losartan, candesartan, irbesartan, telmisartan, valsartan, or epresartan. A non-limiting example of an aldosterone antagonist is spirolactone.

### V. Diagnostic Tests

### A. Measurement of B-type Natriuretic Peptide (BNP) Levels

B-type natriuretic peptide (BNP) is a 32-amino acid neurohormone that is synthesized in the ventricular myocardium and released into circulation in response to ventricular dilation and pressure overload. The functions of BNP include natriuresis, vasodilation, inhibition of the renin-angiotensin-aldosterone axis, and inhibition of sympathetic nerve activity. The plasma concentration of BNP is elevated among patients with congestive heart failure (CHF), and increases in proportion to the degree of left ventricular dysfunction and the severity of CHF symptoms.

Numerous methods and devices are well known to the skilled artisan for measuring BNP levels in patient samples, including serum and plasma. With regard to polypeptides, such as BNP, immunoassay devices and methods are often used. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule. *See, e.g.,* U.S. Patents 5,631,170 and 5,955,377. In a specific example, B-type natriuretic peptide (BNP) levels may be determined by the following method(s): protein immunoassays as described in US Patent Publication 2011/0201130, which is incorporated by reference in its entirety herein. Furthermore, a number of commercially available methods exist (*e.g.,* Rawlins *et al.,* 2005, which is incorporated herein by reference in its entirety).

### B. Measurement of Albumin/Creatinine Ratio (ACR)

Conventionally, proteinuria is diagnosed by a simple dipstick test. Traditionally, dipstick protein tests are quantified by measuring the total quantity of protein in a 24-hour urine collection test.

Alternatively the concentration of protein in the urine may be compared to the creatinine level in a spot urine sample. This is termed the protein/creatinine ratio (PCR). The UK Chronic Kidney Disease Guidelines (2005; which are incorporated herein by reference in their entirety) states PCR is a better test than 24-hour urinary protein measurement. Proteinuria is defined as a protein/creatinine ratio greater than 45 mg/mmol (which is equivalent to albumin/creatinine ratio of greater than 30 mg/mmol or approximately 300 mg/g as defined by dipstick proteinuria of 3+) with very high levels of proteinuria being for a PCR greater than 100 mg/mmol.

Protein dipstick measurements should not be confused with the amount of protein detected on a test for microalbuminuria, which denotes values for protein for urine in mg/day versus urine protein dipstick values which denote values for protein in mg/dL. That is, there is a basal level of proteinuria that can occur below 30 mg/day which is considered non-pathological. Values between 30-300 mg/day are termed microalbuminuria which is considered pathologic. Urine protein lab values for microalbumin of >30 mg/day correspond to a detection level within the "trace" to "1+" range of a urine dipstick protein assay. Therefore, positive indication of any protein detected on a urine dipstick assay obviates any need to perform a urine microalbumin test as the upper limit for microalbuminuria has already been exceeded.

### C. Measurement of Estimated Glomerular Filtration Rate (eGFR)

A number of formulae have been devised to estimate GFR values on the basis of serum creatinine levels. A commonly used surrogate marker for estimate of creatinine clearance (eCcᵣ) is the Cockcroft-Gault (CG) formula, which in turn estimates GFR in mL/min. It employs serum creatinine measurements and a patient's weight to predict the creatinine clearance. The formula, as originally published, is: $eCCr = \frac{\left(140-Age\right) \times Mass \left(in kg\right)}{72 \times Serum creatinine \left(in\frac{mg}{dL}\right)}$ This formula expects weight to be measured in kilograms and creatinine to be measured in mg/dL, as is standard in the USA. The resulting value is multiplied by a constant of 0.85 if the patient is female. This formula is useful because the calculations are simple and can often be performed without the aid of a calculator.

When serum creatinine is measured in µmol/L, then: $eCCr = \frac{\left(140-Age\right) \times Mass \left(in kg\right) \times Constant}{Serum creatinine \left(in\frac{μmol}{L}\right)}$ where Constant is 1.23 for men and 1.04 for women.

One interesting feature of the Cockcroft and Gault equation is that it shows how dependent the estimation of C_{Cr} is based on age. The age term is (140 - age). This means that a 20-year-old person (140-20 = 120) will have twice the creatinine clearance as an 80-year-old (140-80 = 60) for the same level of serum creatinine. The CG equation assumes that a woman will have a 15% lower creatinine clearance than a man at the same level of serum creatinine.

Alternatively, eGFR values may be calculated using the Modification of Diet in Renal Disease (MDRD) formula. The 4-variable formula is as follows: $eGFR = 175 \times Standardized serum {creatinine}^{- 1.154} \times {Age}^{- 0.203} \times C$ where C is 1.212 if the patient is a black male, 0.899 if the patient is a black female, and 0.742 if the patient is a non-black female. Serum creatinine values are based on the IDMS-traceable creatinine determination (see below).

Chronic kidney disease is defined as a GFR less than 60 mL/min/1.73 m² that is present for three or more months.

### D. Measurement of Serum Creatinine Levels

A serum creatinine test measures the level of creatinine in the blood and provides an estimate glomerular filtration rate. Serum creatinine values in the BEACON and BEAM trials were based on the isotope dilution mass spectrometry (IDMS)-traceable creatinine determinations. Other commonly used creatinine assay methodologies include (1) alkaline picrate methods (*e.g.*, Jaffe method [classic] and compensated [modified] Jaffe methods), (2) enzymatic methods, (3) high-performance liquid chromatography, (4) gas chromatography, and (5) liquid chromatography. The IDMS method is widely considered to be the most accurate assay (Peake and Whiting, 2006, which is incorporated herein by reference in its entirety).

### VI. Definitions

When used in the context of a chemical group: "hydrogen" means -H; "hydroxy" means -OH; "oxo" means =O; "carbonyl" means -C(=O)-; "carboxy" means -C(=O)OH (also written as -COOH or -CO₂H); "halo" means independently -F, -Cl, -Br or -I; "amino" means -NH₂; "hydroxyamino" means -NHOH; "nitro" means -NO₂; imino means =NH; "cyano" means -CN; "isocyanate" means -N=C=O; "azido" means -N₃; in a monovalent context "phosphate" means -OP(O)(OH)₂ or a deprotonated form thereof; in a divalent context "phosphate" means -OP(O)(OH)O- or a deprotonated form thereof; "mercapto" means -SH; and "thio" means =S; "sulfonyl" means -S(O)₂-; and "sulfinyl" means -S(O)-.

In the context of chemical formulas, the symbol "-" means a single bond, "=" means a double bond, and "≡" means triple bond. The symbol "----" represents an optional bond, which if present is either single or double. The symbol "-̅ -̅ -̅ -̅" represents a single bond or a double bond. Thus, the formula 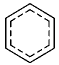 covers, for example, 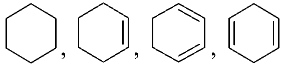 and 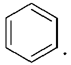 And it is understood that no one such ring atom forms part of more than one double bond. Furthermore, it is noted that the covalent bond symbol "-", when connecting one or two stereogenic atoms, does not indicate any preferred stereochemistry. Instead, it covers all stereoisomers as well as mixtures thereof. The symbol " 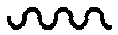 ", when drawn perpendicularly across a bond *(e.g.,* 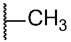 for methyl) indicates a point of attachment of the group. It is noted that the point of attachment is typically only identified in this manner for larger groups in order to assist the reader in unambiguously identifying a point of attachment. The symbol " 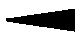 " means a single bond where the group attached to the thick end of the wedge is "out of the page." The symbol " 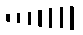 " means a single bond where the group attached to the thick end of the wedge is "into the page". The symbol " 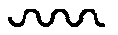 " means a single bond where the geometry around a double bond (e.g., either E or Z) is undefined. Both options, as well as combinations thereof are therefore intended. Any undefined valency on an atom of a structure shown in this application implicitly represents a hydrogen atom bonded to that atom. A bold dot on a carbon atom indicates that the hydrogen attached to that carbon is oriented out of the plane of the paper.

When a variable is depicted as a "floating group" on a ring system, for example, the group "R" in the formula: 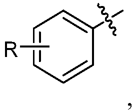 then the variable may replace any hydrogen atom attached to any of the ring atoms, including a depicted, implied, or expressly defined hydrogen, so long as a stable structure is formed. When a variable is depicted as a "floating group" on a fused ring system, as for example the group "R" in the formula: 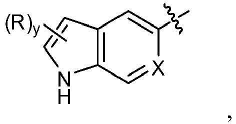 then the variable may replace any hydrogen attached to any of the ring atoms of either of the fused rings unless specified otherwise. Replaceable hydrogens include depicted hydrogens (*e.g.,* the hydrogen attached to the nitrogen in the formula above), implied hydrogens (*e.g.,* a hydrogen of the formula above that is not shown but understood to be present), expressly defined hydrogens, and optional hydrogens whose presence depends on the identity of a ring atom (*e.g.,* a hydrogen attached to group X, when X equals -CH-), so long as a stable structure is formed. In the example depicted, R may reside on either the 5-membered or the 6-membered ring of the fused ring system. In the formula above, the subscript letter "y" immediately following the R enclosed in parentheses, represents a numeric variable. Unless specified otherwise, this variable can be 0, 1, 2, or any integer greater than 2, only limited by the maximum number of replaceable hydrogen atoms of the ring or ring system.

For the chemical groups and compound classes, the number of carbon atoms in the group or class is as indicated as follows: "Cn" defines the exact number (n) of carbon atoms in the group/class. "C≤n" defines the maximum number (n) of carbon atoms that can be in the group/class, with the minimum number as small as possible for the group/class in question. For example, it is understood that the minimum number of carbon atoms in the groups "alkyl_{(C≤8)}", "cycloalkanediyl_{(C≤8)}", "heteroaryl_{(C≤8)}", and "acyl_{(C≤8)}" is one, the minimum number of carbon atoms in the groups "alkenyl_{(C≤8)}", "alkynyl_{(C≤8)}", and "heterocycloalkyl_{(C≤8)}" is two, the minimum number of carbon atoms in the group "cycloalkyl_{(C≤8)}" is three, and the minimum number of carbon atoms in the groups "aryl_{(C≤8)}" and "arenediyl_{(C≤8)}" is six. "Cn-n'" defines both the minimum (n) and maximum number (n') of carbon atoms in the group. Thus, "alkyl_{(C2-10)}" designates those alkyl groups having from 2 to 10 carbon atoms. These carbon number indicators may precede or follow the chemical groups or class it modifies and it may or may not be enclosed in parenthesis, without signifying any change in meaning. Thus, the terms "C5 olefin", "C5-olefin", "olefin_{(C5)}", and "olefin_{C5}" are all synonymous. When any of the chemical groups or compound classes defined herein is modified by the term "substituted", any carbon atom in the moiety replacing the hydrogen atom is not counted. Thus methoxyhexyl, which has a total of seven carbon atoms, is an example of a substituted alkyl_{(C1-6)}. Unless specified otherwise, any chemical group or compound class listed in a claim set without a carbon atom limit has a carbon atom limit of less than or equal to twelve.

The term "saturated" when used to modify a compound or chemical group means the compound or chemical group has no carbon-carbon double and no carbon-carbon triple bonds, except as noted below. When the term is used to modify an atom, it means that the atom is not part of any double or triple bond. In the case of substituted versions of saturated groups, one or more carbon oxygen double bond or a carbon nitrogen double bond may be present. And when such a bond is present, then carbon-carbon double bonds that may occur as part of keto-enol tautomerism or imine/enamine tautomerism are not precluded. When the term "saturated" is used to modify a solution of a substance, it means that no more of that substance can dissolve in that solution.

The term "aliphatic" when used without the "substituted" modifier signifies that the compound or chemical group so modified is an acyclic or cyclic, but non-aromatic hydrocarbon compound or group. In aliphatic compounds/groups, the carbon atoms can be joined together in straight chains, branched chains, or non-aromatic rings (alicyclic). Aliphatic compounds/groups can be saturated, that is joined by single carbon-carbon bonds (alkanes/alkyl), or unsaturated, with one or more carbon-carbon double bonds (alkenes/alkenyl) or with one or more carbon-carbon triple bonds (alkynes/alkynyl).

The term "aromatic" when used to modify a compound or a chemical group refers to a planar unsaturated ring of atoms with 4n +2 electrons in a fully conjugated cyclic π system.

The term "alkyl" when used without the "substituted" modifier refers to a monovalent saturated aliphatic group with a carbon atom as the point of attachment, a linear or branched acyclic structure, and no atoms other than carbon and hydrogen. The groups -CH₃ (Me), -CH₂CH₃ (Et), -CH₂CH₂CH₃ (*n*-Pr or propyl), -CH(CH₃)₂ (*i*-Pr, *ⁱ*Pr or isopropyl), -CH₂CH₂CH₂CH₃ (*n*-Bu), -CH(CH₃)CH₂CH₃ (*sec*-butyl), -CH₂CH(CH₃)₂ (isobutyl), -C(CH₃)₃ (*tert*-butyl, *t*-butyl, *t*-Bu or *^{t}*Bu), and -CH₂C(CH₃)₃ (*neo*-pentyl) are non-limiting examples of alkyl groups. The term "alkanediyl" when used without the "substituted" modifier refers to a divalent saturated aliphatic group, with one or two saturated carbon atom(s) as the point(s) of attachment, a linear or branched acyclic structure, no carbon-carbon double or triple bonds, and no atoms other than carbon and hydrogen. The groups -CH₂-(methylene), -CH₂CH₂-, -CH₂C(CH₃)₂CH₂-, and -CH₂CH₂CH₂- are non-limiting examples of alkanediyl groups. The term "alkylidene" when used without the "substituted" modifier refers to the divalent group =CRR' in which R and R' are independently hydrogen or alkyl. Non-limiting examples of alkylidene groups include: =CH₂, =CH(CH₂CH₃), and =C(CH₃)₂. An "alkane" refers to the class of compounds having the formula H-R, wherein R is alkyl as this term is defined above. When any of these terms is used with the "substituted" modifier, one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂. The following groups are non-limiting examples of substituted alkyl groups: -CH₂OH, -CH₂Cl, -CF₃, -CH₂CN, -CH₂C(O)OH, -CH₂C(O)OCH₃, -CH₂C(O)NH₂, -CH₂C(O)CH₃, -CH₂OCH₃, -CH₂OC(O)CH₃, -CH₂NH₂, -CH₂N(CH₃)₂, and -CH₂CH₂Cl. The term "haloalkyl" is a subset of substituted alkyl, in which the hydrogen atom replacement is limited to halo (*i.e.* -F, -Cl, -Br, or -I) such that no other atoms aside from carbon, hydrogen and halogen are present. The group, -CH₂Cl is a non-limiting example of a haloalkyl. The term "fluoroalkyl" is a subset of substituted alkyl, in which the hydrogen atom replacement is limited to fluoro such that no other atoms aside from carbon, hydrogen and fluorine are present. The groups -CH₂F, -CF₃, and -CH₂CF₃ are non-limiting examples of fluoroalkyl groups.

The term "cycloalkyl" when used without the "substituted" modifier refers to a monovalent saturated aliphatic group with a carbon atom as the point of attachment, said carbon atom forming part of one or more non-aromatic ring structures, no carbon-carbon double or triple bonds, and no atoms other than carbon and hydrogen. Non-limiting examples include: -CH(CH₂)₂ (cyclopropyl), cyclobutyl, cyclopentyl, or cyclohexyl (Cy). As used herein, the term does not preclude the presence of one or more alkyl groups (carbon number limitation permitting) attached to a carbon atom of the non-aromatic ring structure. The term "cycloalkanediyl" when used without the "substituted" modifier refers to a divalent saturated aliphatic group with two carbon atoms as points of attachment, no carbon-carbon double or triple bonds, and no atoms other than carbon and hydrogen. The group 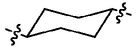 is a non-limiting example of cycloalkanediyl group. A "cycloalkane" refers to the class of compounds having the formula H-R, wherein R is cycloalkyl as this term is defined above. When any of these terms is used with the "substituted" modifier, one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The term "alkenyl" when used without the "substituted" modifier refers to a monovalent unsaturated aliphatic group with a carbon atom as the point of attachment, a linear or branched, acyclic structure, at least one nonaromatic carbon-carbon double bond, no carbon-carbon triple bonds, and no atoms other than carbon and hydrogen. Non-limiting examples include: -CH=CH₂ (vinyl), -CH=CHCH₃, -CH=CHCH₂CH₃, -CH₂CH=CH₂ (allyl), -CH₂CH=CHCH₃, and -CH=CHCH=CH₂. The term "alkenediyl" when used without the "substituted" modifier refers to a divalent unsaturated aliphatic group, with two carbon atoms as points of attachment, a linear or branched, a linear or branched acyclic structure, at least one nonaromatic carbon-carbon double bond, no carbon-carbon triple bonds, and no atoms other than carbon and hydrogen. The groups -CH=CH-, -CH=C(CH₃)CH₂-, -CH=CHCH₂-, and -CH₂CH=CHCH₂- are non-limiting examples of alkenediyl groups. It is noted that while the alkenediyl group is aliphatic, once connected at both ends, this group is not precluded from forming part of an aromatic structure. The terms "alkene" and "olefin" are synonymous and refer to the class of compounds having the formula H-R, wherein R is alkenyl as this term is defined above. Similarly, the terms "terminal alkene" and "α-olefin" are synonymous and refer to an alkene having just one carbon-carbon double bond, wherein that bond is part of a vinyl group at an end of the molecule. When any of these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂. The groups -CH=CHF, -CH=CHCl and -CH=CHBr are non-limiting examples of substituted alkenyl groups.

The term "alkynyl" when used without the "substituted" modifier refers to a monovalent unsaturated aliphatic group with a carbon atom as the point of attachment, a linear or branched acyclic structure, at least one carbon-carbon triple bond, and no atoms other than carbon and hydrogen. As used herein, the term alkynyl does not preclude the presence of one or more non-aromatic carbon-carbon double bonds. The groups -C≡CH, -C≡CCH₃, and -CH₂C≡CCH₃ are non-limiting examples of alkynyl groups. An "alkyne" refers to the class of compounds having the formula H-R, wherein R is alkynyl. When any of these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The term "aryl" when used without the "substituted" modifier refers to a monovalent unsaturated aromatic group with an aromatic carbon atom as the point of attachment, said carbon atom forming part of a one or more aromatic ring structures, each with six ring atoms that are all carbon, and wherein the group consists of no atoms other than carbon and hydrogen. If more than one ring is present, the rings may be fused or unfused. Unfused rings are connected with a covalent bond. As used herein, the term aryl does not preclude the presence of one or more alkyl groups (carbon number limitation permitting) attached to the first aromatic ring or any additional aromatic ring present. Non-limiting examples of aryl groups include phenyl (Ph), methylphenyl, (dimethyl)phenyl, -C₆H₄CH₂CH₃ (ethylphenyl), naphthyl, and a monovalent group derived from biphenyl (*e.g*., 4-phenylphenyl). The term "arenediyl" when used without the "substituted" modifier refers to a divalent aromatic group with two aromatic carbon atoms as points of attachment, said carbon atoms forming part of one or more six-membered aromatic ring structures, each with six ring atoms that are all carbon, and wherein the divalent group consists of no atoms other than carbon and hydrogen. As used herein, the term arenediyl does not preclude the presence of one or more alkyl groups (carbon number limitation permitting) attached to the first aromatic ring or any additional aromatic ring present. If more than one ring is present, the rings may be fused or unfused. Unfused rings are connected with a covalent bond. Non-limiting examples of arenediyl groups include: 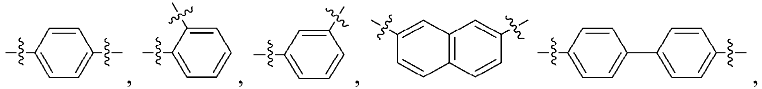 and 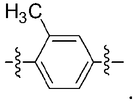 An "arene" refers to the class of compounds having the formula H-R, wherein R is aryl as that term is defined above. Benzene and toluene are non-limiting examples of arenes. When any of these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The term "aralkyl" when used without the "substituted" modifier refers to the monovalent group -alkanediyl-aryl, in which the terms alkanediyl and aryl are each used in a manner consistent with the definitions provided above. Non-limiting examples are: phenylmethyl (benzyl, Bn) and 2-phenyl-ethyl. When the term aralkyl is used with the "substituted" modifier one or more hydrogen atom from the alkanediyl and/or the aryl group has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂. Non-limiting examples of substituted aralkyls are: (3-chlorophenyl)-methyl, and 2-chloro-2-phenyl-eth-1-yl.

The term "heteroaryl" when used without the "substituted" modifier refers to a monovalent aromatic group with an aromatic carbon atom or nitrogen atom as the point of attachment, said carbon atom or nitrogen atom forming part of one or more aromatic ring structures, each with three to eight ring atoms, wherein at least one of the ring atoms is nitrogen, oxygen or sulfur, and wherein the heteroaryl group consists of no atoms other than carbon, hydrogen, aromatic nitrogen, aromatic oxygen and aromatic sulfur. If more than one ring is present, the rings may be fused or unfused. Unfused rings are connected with a covalent bond. As used herein, the term heteroaryl does not preclude the presence of one or more alkyl or aryl groups (carbon number limitation permitting) attached to the aromatic ring or aromatic ring system. Non-limiting examples of heteroaryl groups include furanyl, imidazolyl, indolyl, indazolyl (Im), isoxazolyl, methylpyridinyl, oxazolyl, phenylpyridinyl, pyridinyl (pyridyl), pyrrolyl, pyrimidinyl, pyrazinyl, quinolyl, quinazolyl, quinoxalinyl, triazinyl, tetrazolyl, thiazolyl, thienyl, and triazolyl. The term "N-heteroaryl" refers to a heteroaryl group with a nitrogen atom as the point of attachment. A "heteroarene" refers to the class of compounds having the formula H-R, wherein R is heteroaryl. Pyridine and quinoline are non-limiting examples of heteroarenes. When these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The term "heterocycloalkyl" when used without the "substituted" modifier refers to a monovalent non-aromatic group with a carbon atom or nitrogen atom as the point of attachment, said carbon atom or nitrogen atom forming part of one or more non-aromatic ring structures, each with three to eight ring atoms, wherein at least one of the ring atoms is nitrogen, oxygen or sulfur, and wherein the heterocycloalkyl group consists of no atoms other than carbon, hydrogen, nitrogen, oxygen and sulfur. If more than one ring is present, the rings may be fused or unfused. As used herein, the term does not preclude the presence of one or more alkyl groups (carbon number limitation permitting) attached to the ring or ring system. Also, the term does not preclude the presence of one or more double bonds in the ring or ring system, provided that the resulting group remains non-aromatic. Non-limiting examples of heterocycloalkyl groups include aziridinyl, azetidinyl, pyrrolidinyl, piperidinyl, piperazinyl, morpholinyl, thiomorpholinyl, tetrahydrofuranyl, tetrahydrothiofuranyl, tetrahydropyranyl, pyranyl, oxiranyl, and oxetanyl. The term "*N*-heterocycloalkyl" refers to a heterocycloalkyl group with a nitrogen atom as the point of attachment. *N*-pyrrolidinyl is an example of such a group. When these terms are used with the "substituted" modifier one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The term "acyl" when used without the "substituted" modifier refers to the group -C(O)R, in which R is a hydrogen, alkyl, cycloalkyl, or aryl as those terms are defined above. The groups, -CHO, -C(O)CH₃ (acetyl, Ac), -C(O)CH₂CH₃, -C(O)CH(CH₃)₂, -C(O)CH(CH₂)₂, -C(O)C₆H₅, and -C(O)C₆H₄CH₃ are non-limiting examples of acyl groups. A "thioacyl" is defined in an analogous manner, except that the oxygen atom of the group -C(O)R has been replaced with a sulfur atom, -C(S)R. The term "aldehyde" corresponds to an alkyl group, as defined above, attached to a -CHO group. When any of these terms are used with the "substituted" modifier one or more hydrogen atom (including a hydrogen atom directly attached to the carbon atom of the carbonyl or thiocarbonyl group, if any) has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂. The groups, -C(O)CH₂CF₃, -CO₂H (carboxyl), -CO₂CH₃ (methylcarboxyl), -CO₂CH₂CH₃, -C(O)NH₂ (carbamoyl), and -CON(CH₃)₂, are non-limiting examples of substituted acyl groups.

The term "alkoxy" when used without the "substituted" modifier refers to the group -OR, in which R is an alkyl, as that term is defined above. Non-limiting examples include: -OCH₃ (methoxy), -OCH₂CH₃ (ethoxy), -OCH₂CH₂CH₃, -OCH(CH₃)₂ (isopropoxy), or -OC(CH₃)₃ (*tert-*butoxy)*.* The terms "cycloalkoxy", "alkenyloxy", "alkynyloxy", "aryloxy", "aralkoxy", "heteroaryloxy", "heterocycloalkoxy", and "acyloxy", when used without the "substituted" modifier, refers to groups, defined as -OR, in which R is cycloalkyl, alkenyl, alkynyl, aryl, aralkyl, heteroaryl, heterocycloalkyl, and acyl, respectively. The term "alkylthio" and "acylthio" when used without the "substituted" modifier refers to the group -SR, in which R is an alkyl and acyl, respectively. The term "alcohol" corresponds to an alkane, as defined above, wherein at least one of the hydrogen atoms has been replaced with a hydroxy group. The term "ether" corresponds to an alkane, as defined above, wherein at least one of the hydrogen atoms has been replaced with an alkoxy group. When any of these terms is used with the "substituted" modifier, one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The term "alkylamino" when used without the "substituted" modifier refers to the group -NHR, in which R is an alkyl, as that term is defined above. Non-limiting examples include: -NHCH₃ and -NHCH₂CH₃. The term "dialkylamino" when used without the "substituted" modifier refers to the group -NRR', in which R and R' can be the same or different alkyl groups. Non-limiting examples of dialkylamino groups include: -N(CH₃)₂ and -N(CH₃)(CH₂CH₃). The terms "cycloalkylamino", "alkenylamino", "alkynylamino", "arylamino", "aralkylamino", "heteroarylamino", "heterocycloalkylamino", "alkoxyamino", "alkylsulfonylamino", or "cycloalkylsulfonylamino" when used without the "substituted" modifier, refers to groups, defined as -NHR, in which R is cycloalkyl, alkenyl, alkynyl, aryl, aralkyl, heteroaryl, heterocycloalkyl, alkoxy, alkylsulfonyl, and cycloalkylsulfonyl, respectively. A non-limiting example of an arylamino group is -NHC₆H₅. The term "amido" (acylamino), when used without the "substituted" modifier, refers to the group -NHR, in which R is acyl, as that term is defined above. A non-limiting example of an amido group is -NHC(O)CH₃. When any of these terms is used with the "substituted" modifier, one or more hydrogen atom attached to a carbon atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂. The groups -NHC(O)OCH₃ and -NHC(O)NHCH₃ are non-limiting examples of substituted amido groups.

The terms "alkylsulfonyl" and "alkylsulfinyl" when used without the "substituted" modifier refers to the groups -S(O)₂R and -S(O)R, respectively, in which R is an alkyl, as that term is defined above. The terms "cycloalkylsulfonyl", "alkenylsulfonyl", "alkynylsulfonyl", "arylsulfonyl", "aralkylsulfonyl", "heteroarylsulfonyl", and "heterocycloalkylsulfonyl" are defined in an analogous manner. When any of these terms is used with the "substituted" modifier, one or more hydrogen atom has been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, -C(O)N(CH₃)₂, -OC(O)CH₃, -NHC(O)CH₃, -S(O)₂OH, or -S(O)₂NH₂.

The use of the word "a" or "an," when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects or patients.

An "active ingredient" (AI) (also referred to as an active compound, active substance, active agent, pharmaceutical agent, agent, biologically active molecule, or a therapeutic compound) is the ingredient in a pharmaceutical drug or a pesticide that is biologically active. The similar terms active pharmaceutical ingredient (API) and bulk active are also used in medicine, and the term active substance may be used for pesticide formulations.

As used herein, average molecular weight refers to the weight average molecular weight (Mw) as determined by static light scattering.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

The term "effective," as that term is used in the specification and/or claims, means adequate to accomplish a desired, expected, or intended result. "Effective amount," "Therapeutically effective amount" or "pharmaceutically effective amount" when used in the context of treating a patient or a subject with a compound means that amount of the compound which, when administered to the patient or the subject for treating or preventing a disease, is an amount sufficient to effect the treatment or prevention of the disease. For example, within the context of Alport syndrome, one measure of an effective treatment is the reduction of one or more urine biomarkers such as the presence of blood or protein in the urine or the improved glomerular filtration rate. In a particular embodiment, a measure of an effective treatment is a reduction in the concentration of protein in the urine to less than 300 mg/dL. In a preferred embodiment, the therapy is sufficient to reduce the concentration of protein in the urine to less than 100 mg/dL or a more preferred embodiment, less than 30 mg/dL. When the presence of blood is used as a marker of therapeutic effectiveness, an effective therapy results in the absence of macroscopic blood in the urine while microscopic blood may still be present. In a preferred embodiment, an effective therapy results in the absence of any blood including microscopic blood which would only be visible using a microscope or in an urinalysis. Finally, an effective therapy would result in an improvement in the glomerular filtration rate. Glomerular filtration rate can be estimated using a variety of different methods using creatinine including the Cockcroft-Gault formula, the Modification of Diet in Renal Disease (MDRD) formula, the Chronic Kidney Disease Epidemiology Collaboration (CKD-EPI) formula, the Mayo Quadratic formula, or the Schwartz formula. In general, the Schwartz formula may be used for children below the age of 12. These methods are further elaborated on in the sections above and in the Examples below. For example, an effective treatment may result a glomerular filtration rate (or an estimated glomerular filtration rate) of greater than 60 mL/min/1.73 m². More preferably, the effective treatment may result in an glomerular filtration rate of greater than 90 mL/min/1.73 m².

An "excipient" is a pharmaceutically acceptable substance formulated along with the active ingredient(s) of a medication, pharmaceutical composition, formulation, or drug delivery system. Excipients may be used, for example, to stabilize the composition, to bulk up the composition (thus often referred to as "bulking agents," "fillers," or "diluents" when used for this purpose), or to confer a therapeutic enhancement on the active ingredient in the final dosage form, such as facilitating drug absorption, reducing viscosity, or enhancing solubility. Excipients include pharmaceutically acceptable versions of antiadherents, binders, coatings, colors, disintegrants, flavors, glidants, lubricants, preservatives, sorbents, sweeteners, and vehicles. The main excipient that serves as a medium for conveying the active ingredient is usually called the vehicle. Excipients may also be used in the manufacturing process, for example, to aid in the handling of the active substance, such as by facilitating powder flowability or non-stick properties, in addition to aiding *in vitro* stability such as prevention of denaturation or aggregation over the expected shelf life. The suitability of an excipient will typically vary depending on the route of administration, the dosage form, the active ingredient, as well as other factors.

The term "hydrate" when used as a modifier to a compound means that the compound has less than one (*e.g.,* hemihydrate), one (*e.g.,* monohydrate), or more than one (*e.g.,* dihydrate) water molecules associated with each compound molecule, such as in solid forms of the compound.

As used herein, the term "IC₅₀" refers to an inhibitory dose which is 50% of the maximum response obtained. This quantitative measure indicates how much of a particular drug or other substance (inhibitor) is needed to inhibit a given biological, biochemical or chemical process (or component of a process, i.e. an enzyme, cell, cell receptor or microorganism) by half.

An "isomer" of a first compound is a separate compound in which each molecule contains the same constituent atoms as the first compound, but where the configuration of those atoms in three dimensions differs.

As used herein, the term "patient" or "subject" refers to a living mammalian organism, such as a human, monkey, cow, sheep, goat, dog, cat, mouse, rat, guinea pig, or transgenic species thereof. In certain embodiments, the patient or subject is a primate. Non-limiting examples of human patients are adults, juveniles, infants and fetuses.

As generally used herein "pharmaceutically acceptable" refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

"Pharmaceutically acceptable salts" means salts of compounds of the present invention which are pharmaceutically acceptable, as defined above, and which possess the desired pharmacological activity. Non-limiting examples of such salts include acid addition salts formed with inorganic acids such as hydrochloric acid, hydrobromic acid, sulfuric acid, nitric acid, and phosphoric acid; or with organic acids such as 1,2-ethanedisulfonic acid, 2-hydroxyethanesulfonic acid, 2-naphthalenesulfonic acid, 3-phenylpropionic acid, 4,4'-methylenebis(3-hydroxy-2-ene-1-carboxylic acid), 4-methylbicyclo[2.2.2]oct-2-ene-1-carboxylic acid, acetic acid, aliphatic mono- and dicarboxylic acids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acids, propionic acid, *p*-toluenesulfonic acid, pyruvic acid, salicylic acid, stearic acid, succinic acid, tartaric acid, tertiarybutylacetic acid, and trimethylacetic acid. Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Non-limiting examples of acceptable organic bases include ethanolamine, diethanolamine, triethanolamine, tromethamine, and *N*-methylglucamine. It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts: Properties, and Use (P. H. Stahl & C. G. Wermuth eds., Verlag Helvetica Chimica Acta, 2002).

A "pharmaceutically acceptable carrier," "drug carrier," or simply "carrier" is a pharmaceutically acceptable substance formulated along with the active ingredient medication that is involved in carrying, delivering and/or transporting a chemical agent. Drug carriers may be used to improve the delivery and the effectiveness of drugs, including for example, controlled-release technology to modulate drug bioavailability, decrease drug metabolism, and/or reduce drug toxicity. Some drug carriers may increase the effectiveness of drug delivery to the specific target sites. Examples of carriers include: liposomes, microspheres (*e.g*., made of poly(lactic-co-glycolic) acid), albumin microspheres, synthetic polymers, nanofibers, protein-DNA complexes, protein conjugates, erythrocytes, virosomes, and dendrimers.

A "pharmaceutical drug" (also referred to as a pharmaceutical, pharmaceutical agent, pharmaceutical preparation, pharmaceutical composition, pharmaceutical formulation, pharmaceutical product, medicinal product, medicine, medication, medicament, or simply a drug) is a drug used to diagnose, cure, treat, or prevent disease. An active ingredient (AI) (defined above) is the ingredient in a pharmaceutical drug or a pesticide that is biologically active. The similar terms active pharmaceutical ingredient (API) and bulk active are also used in medicine, and the term active substance may be used for pesticide formulations. Some medications and pesticide products may contain more than one active ingredient. In contrast with the active ingredients, the inactive ingredients are usually called excipients (defined above) in pharmaceutical contexts.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

"Prodrug" means a compound that is convertible *in vivo* metabolically into an inhibitor according to the present invention. The prodrug itself may or may not also have activity with respect to a given target protein. For example, a compound comprising a hydroxy group may be administered as an ester that is converted by hydrolysis *in vivo* to the hydroxy compound. Non-limiting examples of suitable esters that may be converted *in vivo* into hydroxy compounds include acetates, citrates, lactates, phosphates, tartrates, malonates, oxalates, salicylates, propionates, succinates, fumarates, maleates, methylene-bis-β-hydroxynaphthoate, gentisates, isethionates, di-p-toluoyltartrates, methanesulfonates, ethanesulfonates, benzenesulfonates, *p*-toluenesulfonates, cyclohexylsulfamates, quinates, and esters of amino acids. Similarly, a compound comprising an amine group may be administered as an amide that is converted by hydrolysis *in vivo* to the amine compound.

A "stereoisomer" or "optical isomer" is an isomer of a given compound in which the same atoms are bonded to the same other atoms, but where the configuration of those atoms in three dimensions differs. "Enantiomers" are stereoisomers of a given compound that are mirror images of each other, like left and right hands. "Diastereomers" are stereoisomers of a given compound that are not enantiomers. Chiral molecules contain a chiral center, also referred to as a stereocenter or stereogenic center, which is any point, though not necessarily an atom, in a molecule bearing groups such that an interchanging of any two groups leads to a stereoisomer. In organic compounds, the chiral center is typically a carbon, phosphorus or sulfur atom, though it is also possible for other atoms to be stereocenters in organic and inorganic compounds. A molecule can have multiple stereocenters, giving it many stereoisomers. In compounds whose stereoisomerism is due to tetrahedral stereogenic centers (*e.g*., tetrahedral carbon), the total number of hypothetically possible stereoisomers will not exceed 2ⁿ, where n is the number of tetrahedral stereocenters. Molecules with symmetry frequently have fewer than the maximum possible number of stereoisomers. A 50:50 mixture of enantiomers is referred to as a racemic mixture. Alternatively, a mixture of enantiomers can be enantiomerically enriched so that one enantiomer is present in an amount greater than 50%. Typically, enantiomers and/or diastereomers can be resolved or separated using techniques known in the art. It is contemplated that that for any stereocenter or axis of chirality for which stereochemistry has not been defined, that stereocenter or axis of chirality can be present in its *R* form, *S* form, or as a mixture of the *R* and *S* forms, including racemic and non-racemic mixtures. As used herein, the phrase "substantially free from other stereoisomers" means that the composition contains ≤ 15%, more preferably ≤ 10%, even more preferably ≤ 5%, or most preferably ≤ 1% of another stereoisomer(s).

"Treatment" or "treating" includes (1) inhibiting a disease in a subject or patient experiencing or displaying the pathology or symptomatology of the disease (*e.g*., arresting further development of the pathology and/or symptomatology), (2) ameliorating a disease in a subject or patient that is experiencing or displaying the pathology or symptomatology of the disease (*e.g*., reversing the pathology and/or symptomatology), and/or (3) effecting any measurable decrease in a disease in a subject or patient that is experiencing or displaying the pathology or symptomatology of the disease.

The above definitions supersede any conflicting definition in any reference that is incorporated by reference herein. The fact that certain terms are defined, however, should not be considered as indicative that any term that is undefined is indefinite. Rather, all terms used are believed to describe the invention in terms such that one of ordinary skill can appreciate the scope and practice the present invention.

### VII. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Bardoxolone Methyl Increases Renal Function

Bardoxolone methyl has been studied in seven CKD studies enrolling approximately 2,600 patients with Type 2 diabetes and CKD. Improvements in renal function, including inulin clearance, creatinine clearance, and eGFR, have been observed with bardoxolone methyl treatment in a number of clinical studies. A recent study in Japanese CKD patients demonstrated that bardoxolone methyl treatment resulted in a significant improvement in measured GFR, as assessed by inulin clearance, after 16 weeks of treatment compared to placebo. Moreover, measured GFR increases were significantly and positively correlated with improvements in eGFR. Two separate studies (studies 402-C-0804 and 402-C-0903) showed that increases in eGFR in CKD patients treated with bardoxolone methyl were sustained for at least one year. Furthermore, after one year of treatment, a residual eGFR increase from baseline was observed in bardoxolone methyl patients after cessation of drug for four weeks, while an eGFR decline from baseline was observed in placebo patients. In other CKD studies, bardoxolone methyl has been shown to significantly reduce uremic solutes (BUN, uric acid, and phosphate) in inverse correlation to eGFR increases and to numerically reduce renal SAEs and ESRD events.

The data from these studies suggest that bardoxolone methyl has the potential to prevent renal function decline, which could ultimately prevent or delay ESRD. In Alport syndrome patients, who have average eGFR declines of 4.0 mL/min/1.73 m² per year, the potential impact of a sustained eGFR increase with bardoxolone methyl treatment is clinically meaningful and could provide a multi-year delay in disease progression to ESRD.

Key features of bardoxolone methyl's effects on renal function are summarized below.

### A. Bardoxolone Methyl Improves Kidney Function, as Assessed by Measured GFR (Inulin Clearance), Creatinine Clearance, and eGFR

In a study in Japanese patients with Stage 3 CKD, bardoxolone methyl treatment resulted in a significant improvement in measured GFR, as assessed by inulin clearance, after 16 weeks of treatment compared to placebo (Table 22).

**Table 22: Bardoxolone Methyl Increases GFR, as Assessed by Inulin Clearance**

| **Change from Baseline at Week 16 (mL/min/1.73 m²)** | **Placebo (N=23)** | **Bardoxolone Methyl (N=17)** |
|---|---|---|
| Mean ± SD | -0.42 ± 7.52 | 5.58 ± 7.90 |
| LS Mean | -0.69 | 5.95 |
| 95% CI (LL,UL) | -3.83, 2.45 | 2.29, 9.60 |
| p-value versus Placebo | - | 0.008 |

Moreover, measured GFR increases were significantly and positively correlated with improvements in eGFR (p = 0.002). In two studies, bardoxolone methyl significantly increased creatinine clearance. Importantly, these increases were not associated with a change in total 24-hour excretion of creatinine, which demonstrates that bardoxolone methyl does not affect creatinine metabolism. In other CKD studies, bardoxolone methyl has been shown to significantly reduce uremic solutes (BUN, uric acid, and phosphate) in inverse correlation to eGFR increases and to numerically reduce renal SAEs and ESRD events. These data demonstrate that the increases in eGFR observed in seven CKD studies of bardoxolone methyl treatment reflect true increases in GFR and support the use of eGFR as a reliable marker of renal function (Table 23).

**Table 13: Cross-Study Comparison of Increases in eGFR, Inulin Clearance, Creatinine Clearance with Bardoxolone Methyl Treatment**

| **Study** | **Phase/Country** | **Patient Population** | **Mean Placebo-corrected ΔeGFR (mL/min/1.73m²)⁽¹⁾** |
|---|---|---|---|
| 402-C-0801 (Stratum 1) | 2a/US | Diabetic nephropathy | 6.7 (*p* < 0.001)⁽²⁾ |
| 402-C-0801 (Stratum 2) | 2b/US | Diabetic nephropathy | 7.2 (*p* < 0.001)^{(2)⁽³⁾} |
| 402-C-0804 (BEAM) | 2/US | CKD/Diabetes | 8.6 (*p <* 0.001 *vs.* PBO) |
| 402-C-0902 | 2/US | CKD/Diabetes | 6.5 (*p* < 0.001)⁽²⁾ |
| 402-C-0903 (BEACON) | 3/Global | CKD/Diabetes | 6.4 (*p* < 0.001 *vs.* PBO)^{c} |
| 402-C-1102 | 1/US | CKD/Diabetes | 9.0 (*p* < 0.05)⁽²⁾ |
| RTA402-005 (TSUBAKI) | 2/Japan | CKD/Diabetes | 6.6 (inulin GFR) (*p* = 0.008 *vs.* PBO) |
| 402-C-0501 | 1/US | Cancer | 18.2 (*p* < 0.0001)⁽²⁾ |
| 402-C-0702 | 1/2/US | Cancer | *32.2* (*p* = 0.001)⁽²⁾ |
| 402-C-1302 (LARIAT) | 2/US | Pulmonary hypertension | 14.7 (*p* < 0.001 *vs.* PBO) |

| | | | |
|---|---|---|---|
| **Error! Reference source not found.** Unless noted, data are differences between mean eGFR changes from baseline for bardoxolone methyl versus placebo groups and p-values calculated comparing the difference in means between bardoxolone methyl and placebo groups. ⁽²⁾ Data are mean eGFR changes from baseline for bardoxolone methyl patients and p-values are calculated from two-sided paired t-tests comparing eGFR change to 0. ⁽³⁾ Study also demonstrated a significant increase in creatinine clearance. | | | |

### B. Bardoxolone Methyl Produces a Retained eGFR Increase After Withdrawal of Drug

After one year of treatment with bardoxolone methyl, a residual eGFR increase from baseline is observed after cessation of treatment for four weeks, while an eGFR decline from baseline is observed in placebo patients. Sub-therapeutic concentrations of drug are achieved within approximately 10 days after drug withdrawal. In studies 402-C-0804 and 402-C-0903, approximately 20% to 25% of the on-treatment eGFR increase (or 1 to 4 mL/min/1.73 m² improvement relative to baseline) was maintained in bardoxolone methyl-treated patients after withdrawal of drug. The increases above baseline were statistically significant in both studies. Placebo-treated patients in these studies lost approximately 1 mL/min/1.73 m² over the course of a year, and in study 402-C-0903, this loss from baseline was statistically significant.

The sustained increase in eGFR through one year of treatment and the presence of a sustained eGFR improvement after withdrawal of drug suggest that the maladaptive structural deficits that contribute to declining kidney function (such as mesangium expansion and interstitial fibrosis) may be improved over the course of longer-term treatment with bardoxolone methyl.

### Example 2 - Alport Syndrome Clinical Trial

CARDINAL is an international, multi-center Phase 2/3 study, initiated in February 2017, enrolling patients from 12 to 60 years old with a confirmed genetic or histological diagnosis of Alport syndrome. Patients must have baseline eGFR values between 30 to 90 mL/min/1.73 m² and must be receiving stable renin-angiotensin-aldosterone system blockade unless contraindicated. The Phase 2 portion of CARDINAL is open-label and enrolled 27 patients. The primary endpoint of the Phase 2 portion of the study is the eGFR change from baseline at 12 weeks.

The Phase 3 portion of CARDINAL is designed to support regulatory approval of bardoxolone methyl for the treatment of Alport syndrome. It will be double-blind, placebo-controlled and will randomize 150 patients on a 1:1 basis to once-daily, oral bardoxolone or placebo. The eGFR change after 48 weeks will be measured while the patient is on treatment, and after withdrawal of drug for four weeks (retained eGFR). After withdrawal, patients will be restarted on study drug with their original treatment assignments and will continue on study drug for an additional 48 weeks. The change from baseline in eGFR in bardoxolone methyl-treated patients relative to placebo will be measured again at the end of this second 48 week period (week 100 overall). The eGFR change will also be measured following the withdrawal of drug for four weeks (retained eGFR; week 104 overall). If the trial is successful, the year one retained eGFR data could support accelerated approval under subpart H of the Food, Drug, and Cosmetic Act, or the FDA Act, and the year two retained eGFR data could support full approval under the FDA Act.

Inclusion criteria include a positive genetic diagnosis of Alport syndrome and evidence of impaired renal function as measured by eGFR. The study will exclude patients with a history of clinically significant heart disease and elevated baseline BNP, as well as patients with severely compromised renal function (eGFR < 45 mL/min/1.73 m² and ACR > 2000 mg/g). Patients will be carefully monitored for potential fluid overload.

### A. Development of Exclusion Criteria for Clinical Trial

Study 402-C-0903, titled "Bardoxolone Methyl Evaluation in Patients with Chronic Kidney Disease and Type 2 Diabetes: The Occurrence of Renal Events" (BEACON) was a phase 3, randomized, double-blind, placebo-controlled, parallel-group, multinational, multicenter study designed to compare the efficacy and safety of bardoxolone methyl (BARD) to placebo (PBO) in patients with stage 4 chronic kidney disease and type 2 diabetes. A total of 2,185 patients were randomized 1:1 to once-daily administration of bardoxolone methyl (20 mg) or placebo. The primary efficacy endpoint of the study was the time-to-first event in the composite endpoint defined as end-stage renal disease (ESRD; need for chronic dialysis, renal transplantation, or renal death) or cardiovascular (CV) death. The study had three secondary efficacy endpoints: (1) change in estimated glomerular filtration rate (eGFR); (2) time-to-first hospitalization for heart failure or death due to heart failure; and (3) time-to-first event of the composite endpoint consisting of non-fatal myocardial infarction, non-fatal stroke, hospitalization for heart failure, or cardiovascular death.

The BEACON trial was terminated in October 2012, due to excessive mortality and episodes of heart failure in the treatment arm. Subsequent analysis indicated that the elevated risk was associated with fluid overload due to changes in renal processing of sodium, and was largely confined to patients having: (a) a history of left-sided myocardial disease; or (b) an elevated B-type natriuretic peptide (BNP) level. An elevated albumin-creatinine ratio and advanced (stage 4) CKD were also determined to contribute to the risk of fluid overload.

Based on this analysis, a trial of bardoxolone methyl in patients with pulmonary arterial hypertension, known as LARIAT, was proposed and was approved by the United States FDA. Exclusion criteria included the risk factors noted in the BEACON analysis. Based on initial indications of safety and efficacy in PAH patients, the LARIAT trial was expanded to include other forms of pulmonary hypertension. Based on further indications of safety and efficacy in PAH patients, a Phase 3 trial of bardoxolone methyl in patients with PAH associated with connective tissue disease (CTD-PAH), known as CATALYST, was proposed and approved by the FDA.

Alport syndrome patients normally do not have other cardiovascular or metabolic comorbidities (*e.g.*, diabetes), unlike most patients with CKD caused by diabetes. Therefore, Alport syndrome patients are unlikely to suffer from the two principal risk factors identified in the BEACON analysis.

### B. Phase 2 Results of the CARDINAL Trial

The Phase 2 portion of the trial enrolled 30 patients, and available data demonstrated that bardoxolone significantly improved kidney function in Alport syndrome patients as measured by estimated glomerular filtration rate ("eGFR"). All patients completed the treatment period without any discontinuations. The mean baseline eGFR (± SD) was 54.7 ± 24 mL/min/1.73 m².

For the first eight patients that reached Week 12, available data showed a mean improvement of 6.9 mL/min/1.73 m² at Week 4 (n = 19; *p* < 0.0005), increasing by 12.7 mL/min/1.73 m² at Week 12 (n = 8; *p* < 0.00005). Over 80% of patients demonstrated a clinically meaningful improvement in eGFR of at least 3.0 mL/min/1.73 m² by Week 8, and the 95% confidence interval at Week 12 was 7.9 mL/min/1.73 m² to 17.5 mL/min/1.73 m². The observed treatment effect surpassed the threshold of 3.0 mL/min/1.73 m², which was the minimum effect size necessary to proceed to the Phase 3 portion of the trial.

For the full cohort of thirty patients, the mean baseline eGFR (± SD) was 54 ± 24 mL/min/1.73 m². Bardoxolone increased eGFR by 13.4 mL/min/1.73 m² (n = 3-; *p* < 1 × 10⁻⁹; 95% CI 10.54 to 16.3) after 12 weeks of treatment. All patients had improvements from baseline, and 87% had an increase of at least 4 mL/min/1.73 m², which is the approximate annual rate of decline in kidney function in patients with Alport syndrome. The improvements in eGFR translated to an improvement in CKD stage for 22/30 (73%) patients.

No serious adverse events or discontinuations were reported in the trial, and reported adverse events were generally mild to moderate in intensity. An independent data monitoring committee reviewed all available safety data and voted to recommend opening the Phase 3 portion of the trial.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### VIII. References

The following references, to the extent that they provide exemplary procedural or other details supplementary to those set forth herein, are specifically incorporated herein by reference.
U.S. Pat. No. 5,480,792
U.S. Pat. No. 5,525,524
U.S. Pat. No. 5,631,170
U.S. Pat. No. 5,679,526
U.S. Pat. No. 5,824,799
U.S. Pat. No. 5,851,776
U.S. Pat. No. 5,885,527
U.S. Pat. No. 5,922,615
U.S. Pat. No. 5,939,272
U.S. Pat. No. 5,947,124
U.S. Pat. No. 5,955,377
U.S. Pat. No. 5,985,579
U.S. Pat. No. 6,019,944
U.S. Pat. No. 6,025,395
U.S. Pat. No. 6,113,855
U.S. Pat. No. 6,143,576
U.S. Pat. Pub. 2003/0232786
U.S. Pat. Pub. 2008/0261985
U.S. Pat. Pub. 2009/0048204
U.S. Pat. Pub. 2009/0326063
U.S. Pat. Pub. 2010/0041904
U.S. Pat. Pub. 2010/0048887
U.S. Pat. Pub. 2010/0048892
U.S. Pat. Pub. 2010/0048911
U.S. Pat. Pub. 2010/0056777
U.S. Pat. Pub. 2011/0201130
PCT Pub. WO 2009/023232
PCT Pub. WO 2009/048204
PCT Pub. WO 2010/093944
Ahmad et al., "Triterpenoid CDDO-Me blocks the NF-κB pathway by direct inhibition of IKKβ on Cys-179," J. Biol. Chem., 281:35764-35769, 2006
Ahmad et al., "Triterpenoid CDDO-Methyl Ester Inhibits the Janus-Activated Kinase-1 (JAK1)→Signal Transducer and Activator of Transciption-3 (STAT3) Pathway by Direct Inhibition of JAK1 and STAT3," Cancer Res., 68(8):2920-2926, 2008.
Anderson, Practical Process Research & Development - A Guide for Organic Chemists, 2nd ed., Academic Press, New York, 2012.
Dhaun et al., "Urinary endothelin-1 in chronic kidney disease and as a marker of disease activity in lupus nephritis," American Journal of Physiology - Renal Physiology, 296:F1477-F1483, 2009.
Dinkova-Kostova et al., "Extremely Potent Triterpenoid Inducers of the Phase 2 Response: Correlations of Protection Against Oxidant and Inflammatory Stress," Proc. Natl. Acad. Sci., 102(12):4584-4589, 2005.
Honda et al., "New Enone Derivativas of Oleanolic Acid and Ursolic Acid as Inhibitors of Nitric Oxide Production in Mouse Macrophages," 1997.
Honda et al., "Design and Synthesis of 2-cyano-3,12-dioxoolean-1,9-dien-28-oic acid, a Novel and Highly Active Inhibitor of Nitric Oxide Production in Mouse Macrophages," Bioorg. Med. Chem. Lett., 8(19):2711-2714, 1998.
Honda et al., "Novel Synthetic Oleanane Triterpenoids: A Series of Highly Active Inhibitors of Nitric Oxide Production in Mouse Macrophages," Bioorg. Med. Chem. Lett., 9(24):3429-3434, 1999.
Honda et al., "Novel Synthetic Oleanane and Ursane Triterpenoids with Various Enone Functionalities in Ring A as Inhibitors of Nitric Oxide Production in Mouse Macropahges," J. Med. Chem., 43:1866-1877, 2000a.
Honda et al., "Synthetic Oleanane and Ursane Triterpenoids with Modified Rings A and C: A Series of Highly Active Inhibitors of Nitric Oxide Production in Mouse Macrophages," J. Med. Chem., 43:4233-4246, 2000b.
Honda et al., "A novel dicyanotriterpenoid, 2-cyano-3,12-dioxooleana-1,9(11)-dien-28-onitrile, active at picomolar concentrations for Inhibition of Nitric Oxide Production," Bioorg. Med. Chem. Lett., 12:1027-1030, 2002.
Handbook of Pharmaceutical Salts: Properties, and Use, Stahl and Wermuth Eds.), Verlag Helvetica Chimica Acta, 2002.
Huang et al., "Inhibition of Skin Tumorigenesis by Rosemary and its Constituents Carnosol and Ursolic Acid," Cancer Res., 54:701-708, 1994.
Ikeda et al., "The Novel Triterpenoid CDDO and its Derivatives Induce Apoptosis by Disruption of Intracellular Redox Balance," Cancer Res., 63:5551-5558, 2003.
Ikeda et al., "Induction of Redox Imbalance and Apoptosis in Multiple Myeloma Cells by the Novel Triterpenoid 2-cyano-3,12-dioxoolean-1,9-dien-28-oic acid," Mol. Cancer Ther., 3:39-45, 2004.
Joint Specialty Committee on Renal Medicine of the Royal College of Physicians and the Renal Association, and the Royal College of General Practitioners. Chronic kidney disease in adults: UK guidelines for identification, management and referral. London: Royal College of Physicians, 2006.
Kobayashi and Yamamoto, "Molecular Mechanism Activating the Nrf2-Keap 1 Pathway of Antioxidant Gene Regulation," Antioxid. Redox. Signal., 7:385-394, 2005.
Liby et al., "The Synthetic Triterpenoids, CDDO and CDDO-imidazolide, are Potent Inducers of Heme Oxygenase-1 and Nrf2/ARE signaling," Cancer Res., 65:4789-4798, 2005.
Maines and Gibbs, "30 Some Years of Heme Oxygenase: From a 'molecular wrecking ball' to a 'mesmerizing' Trigger of Cellular Events," Biochem. Biophys. Res. Commun., 338:568-577, 2005.
March's Advanced Organic Chemistry: Reactions, Mechanisms, and Structure, 2007*.*
Nishino et al., "Inhibition of the Tumor-Promoting Action of 12-O tetradecanoylphorbol-13-acetate by some Oleanane-type Triterpenoid Compounds," Cancer Res., 48:5210-5215, 1988
Peake and Whiting, "Measurement of Serum Creatinine-Current Status and Future Goals," Clin. Biochem. Rev., 27:173-184, 2006.
Pergola et al., "Bardoxolone methyl and kidney function in CKD with type 2 diabetes," New Engl. J. Med., 365, 327-336, 2011.
Place et al., "The Novel Synthetic Triterpenoid, CDDO-Imidazolide, Inhibits Inflammatory Response and Tumor Growth In Vivo," Clin. Cancer Res., 9:2798-2806, 2003.
Rawlins et al., "Performance Characteristics of Four Automated Natriuretic Peptide Assays," Am. J. Clin. Pathol., 123:439-445, 2005.
Reagan-Shaw et al., "Dose Translation From Animal to Human Studies Revisited," FASEB J., 22(3):659-661, 2008
Repka et al., "Encyclopedia of Pharmaceutical Technology," New York:Marcel Dekker, 2002.
Schneider et al., "Contrasting actions of endothelin ETA and ETB receptors in cardiovascular disease," Annual Review of Pharmacology and Toxicology, 47:731-759, 2007.
Shishodia et al., "A Synthetic Triterpenoid, CDDO-Me, Inhibits IκBα Kinase and Enhances Apoptosis Induced by TNF and Chemotherapeutic Agents Through Down-Regulation of Expression of Nuclear Factor κB-Regulated Gene Products in Human Leukemic Cells," Clin. Cancer Res., 12(6):1828-1838, 2006.
Suh et al., "Novel Triterpenoids Suppress Inducible Nitric Oxide Synthase (iNOS) and Inducible Cyclooxygenase (COX-2) in Mouse Macrophages," Cancer Res., 58:717-723, 1998.
Suh et al., "A Novel Synthetic Oleanane Triterpenoid, 2-cyano-3,12-dioxoolean-1,9-dien-28-oic acid, with potent differentiating, antiproliferative, and anti-inflammatory activity," Cancer Res., 59(2):336-341, 1999.
Suh et al., "Synthetic Triterpenoids Enhance Transforming Growth Factor β/Smad Signaling," Cancer Res., 63:1371-1376, 2003.
Vachiéry and Davenport, "The endothelin system in pulmonary and renal vasculopathy: les liaisons dangereuses," European Respiratory Review, 18:260-271, 2009.
Vasan et al., "Congestive heart failure in subjects with normal versus reduced left ventricular ejection fraction: Prevalence and mortality in a population-based cohort," Journal of the American College of Cardiology, 33:1948-1955, 1999.
Wang et al., "A Synthetic Triterpenoid, 2-cyano-3,12-dioxooleana-1,9-dien-28-oic acid (CDDO), is a Ligand for the Peroxisome Proliferator-Activated Receptor γ," Mol. Endocrin., 14(10):1550-1556, 2000.
Yates et al., "Pharmacodynamic Characterization of Chemopreventive Triterpenoids as Exceptionally Potent Inducers of Nrf2-regulated Genes," Mol. Cancer. Ther., 6:154-162, 2007.

In particular embodiments, the invention may be defined according to the following paragraphs.
Paragraph 1. A method of treating or preventing Alport syndrome in a patient in need thereof, comprising administering to the patient a therapeutically effective amount of a compound of the formula: 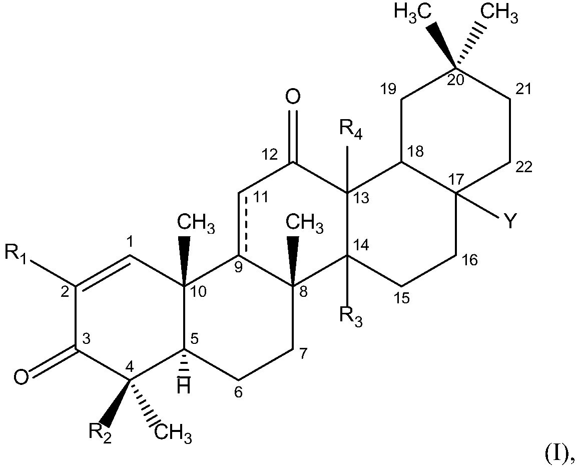 wherein:
   R₁ is -CN, halo, -CF₃, or -C(O)Rₐ, wherein Rₐ is -OH, alkoxy_{(C1-4)}, -NH₂, alkylamino_{(C1-4)}, or -NH-S(O)₂-alkyl_{(C1-4)};
   R₂ is hydrogen or methyl;
   R₃ and R₄ are each independently hydrogen, hydroxy, methyl or as defined below when either of these groups is taken together with group R_{c}; and
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C-12)}, heteroaryl_{(C≤8)}, heterocycloalkyl_{(C≤12)}, alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)},
         -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
         R_{c} and R₃, taken together, are -O- or -NR_{d}-, wherein R_{d} is hydrogen or alkyl_{(C≤4)}; or
         R_{c} and R₄, taken together, are -O- or -NR_{d}-, wherein R_{d} is hydrogen or alkyl_{(C≤4)}; or
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 2. The method of paragraph 1, wherein the compound is further defined as: 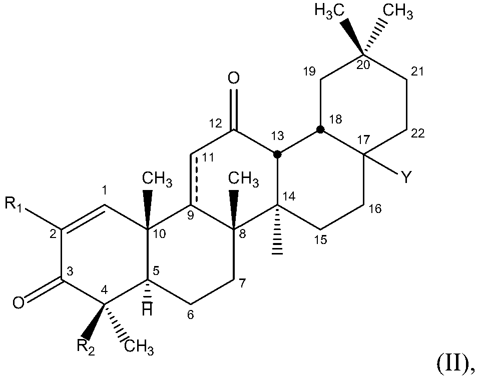 wherein:
   R₁ is -CN, halo, -CF₃, or -C(O)Rₐ, wherein Rₐ is -OH, alkoxy_{(C1-4)}, -NH₂, alkylamino_{(C1-4)}, or -NH-S(O)₂-alkyl_{(C1-4)};
   R₂ is hydrogen or methyl;
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C-12)}, heteroaryl_{(C≤8)}, heterocycloalkyl_{(C≤12)}, alkoxy_{(C≤8)}, cycloalkoxy_{(C≤8)}, aryloxy_{(C≤12}), acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)},
         -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 3. The method of paragraph 2, wherein the compound is further defined as: 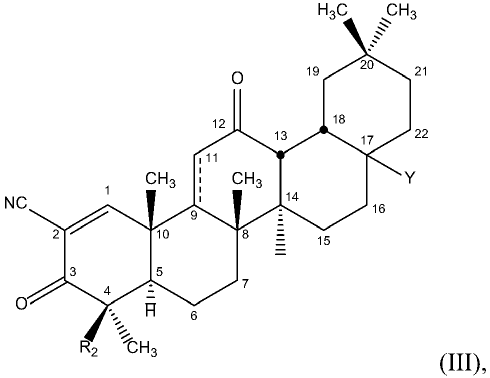 wherein:
   R₂ is hydrogen or methyl;
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C-12)}, heteroaryl_{(C≤8)}, heterocycloalkyl_{(C≤12)}, alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 4. The method of paragraph 3, wherein the compound is further defined as: 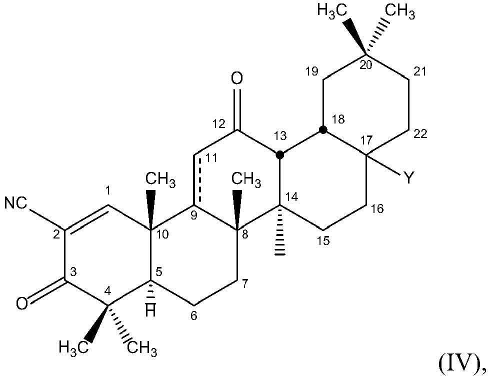 wherein:
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C-12)}, heteroaryl_{(C≤8)}, heterocycloalkyl_{(C≤12)}, alkoxy_{(C≤8)}, cycloalkoxy_{(C≤8)}, aryloxy_{(C≤12}), acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 5. The method of paragraph 4, wherein the compound is further defined as: 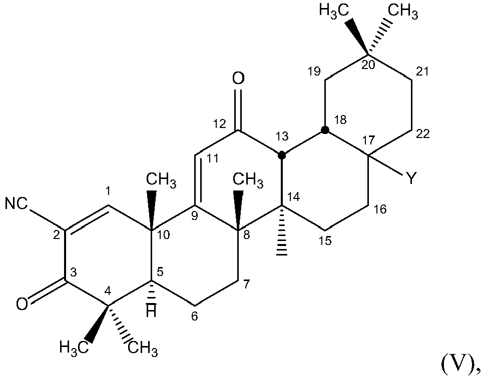 wherein:
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C≤12)}, heteroaryl_{(C≤8)}, heterocycloalkyl_{(C≤12)}, alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonylamino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 6. The method of paragraph 5, wherein the compound is further defined as: 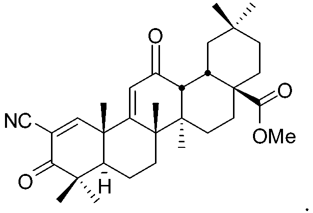
Paragraph 7. The method according to any one of paragraphs 1-6, wherein the patient does not have cardiovascular disease.
Paragraph 8. The method according to any one of paragraphs 1-6, wherein the patient has cardiovascular disease.
Paragraph 9. The method according to either paragraph 7 or paragraph 8, wherein the cardiovascular disease is left-sided myocardial disease.
Paragraph 10. The method according to either paragraph 7 or paragraph 8, wherein the cardiovascular disease is atherosclerosis.
Paragraph 11. The method according to either paragraph 7 or paragraph 8, wherein the cardiovascular disease is restenosis.
Paragraph 12. The method according to either paragraph 7 or paragraph 8, wherein the cardiovascular disease is thrombosis.
Paragraph 13. The method according to either paragraph 7 or paragraph 8, wherein the cardiovascular disease is pulmonary hypertension.
Paragraph 14. The method of paragraph 13, wherein the pulmonary hypertension is World Health Organization (WHO) Class I pulmonary hypertension (pulmonary arterial hypertension or PAH).
Paragraph 15. The method of paragraph 14, wherein the pulmonary hypertension is pulmonary arterial hypertension associated with connective tissue disease.
Paragraph 16. The method of paragraph 14, wherein the pulmonary hypertension is idiopathic pulmonary arterial hypertension.
Paragraph 17. The method of paragraph 13, wherein the pulmonary hypertension is WHO Class II pulmonary hypertension.
Paragraph 18. The method of paragraph 13, wherein the pulmonary hypertension is WHO Class III pulmonary hypertension.
Paragraph 19. The method of paragraph 13, wherein the pulmonary hypertension is WHO Class IV pulmonary hypertension.
Paragraph 20. The method of paragraph 13, wherein the pulmonary hypertension is WHO Class V pulmonary hypertension.
Paragraph 21. The method according to any one of paragraphs 1-20, wherein the patient does not have endothelial dysfunction.
Paragraph 22. The method according to any one of paragraphs 1-20, wherein the patient has endothelial dysfunction.
Paragraph 23. The method according to any one of paragraphs 1-22, wherein the patient does not have Stage 4 or higher chronic kidney disease.
Paragraph 24. The method according to any one of paragraphs 1-22, wherein the patient does not have an estimated glomerular filtration rate (eGFR) less than 45 mL/min/1.73 m².
Paragraph 25. The method according to any one of paragraphs 1-22, wherein the patient does not have an elevated albumin/creatinine ratio (ACR) is greater than 2000 mg/g.
Paragraph 26. The method according to any one of paragraphs 1-25, wherein the patient does not have diabetes.
Paragraph 27. The method according to any one of paragraphs 1-25, wherein the patient has diabetes.
Paragraph 28. The method of either paragraph 26 or paragraph 27, wherein the diabetes is Type 2 diabetes.
Paragraph 29. The method according to any one of paragraphs 1-28, wherein the patient does not have a complication associated with diabetes.
Paragraph 30. The method according to any one of paragraphs 1-28, wherein the patient has a complication associated with diabetes.
Paragraph 31. The method according to either paragraph 29 or paragraph 30, wherein the complication associated with diabetes is diabetic nephropathy.
Paragraph 32. The method according to either paragraph 29 or paragraph 30, wherein the complication is selected from the group consisting of obesity, stroke, peripheral vascular disease, neuropathy, myonecrosis, retinopathy and metabolic syndrome (syndrome X).
Paragraph 33. The method according to any one of paragraphs 1-32, wherein the patient does not have insulin resistance.
Paragraph 34. The method according to any one of paragraphs 1-32, wherein the patient has insulin resistance.
Paragraph 35. The method according to any one of paragraphs 1-34, wherein the patient does not have fatty liver disease.
Paragraph 36. The method according to any one of paragraphs 1-34, wherein the patient has fatty liver disease.
Paragraph 37. The method according to any one of paragraphs 1-34, wherein the patient does not have hepatic impairment.
Paragraph 38. The method according to any one of paragraphs 1-34, wherein the patient has hepatic impairment.
Paragraph 39. The method according to any one of paragraphs 1-38, wherein the patient is not overweight.
Paragraph 40. The method according to any one of paragraphs 1-38, wherein the patient is overweight.
Paragraph 41. The method according to either paragraph 39 or paragraph 40, where the patient is obese.
Paragraph 42. The method of paragraph 41, where the obesity is class I.
Paragraph 43. The method of paragraph 41, where the obesity is class II.
Paragraph 44. The method of paragraph 41, where the obesity is class III.
Paragraph 45. The method according to either paragraph 40 or paragraph 41, where the patient's body mass index (BMI) is from 25 kg/m² to 30 kg/m².
Paragraph 46. The method according to either paragraph 40 or paragraph 41, where the patient's BMI is from 30 kg/m² to 35 kg/m².
Paragraph 47. The method according to either paragraph 40 or paragraph 41, where the patient's BMI is from 35 kg/m² to 40 kg/m².
Paragraph 48. The method according to either paragraph 40 or paragraph 41, where the patient's BMI is from 40 kg/m² to 80 kg/m².
Paragraph 49. The method according to any one of paragraphs 1-36, wherein the patient has cancer.
Paragraph 50. The method according to any one of paragraphs 1-36, wherein the patient does not have cancer.
Paragraph 51. The method of either paragraph 49 or paragraph 50, wherein the cancer is an advanced solid tumor or lymphoid malignancy.
Paragraph 52. The method of either paragraph 49 or paragraph 50, wherein the cancer is selected from the groups consisting of breast cancer, prostate cancer, colon cancer, brain cancer, melanoma, pancreatic cancer, ovarian cancer, leukemia, or bone cancer.
Paragraph 53. The method of paragraph 52, wherein the cancer is an advanced malignant melanoma.
Paragraph 54. The method of paragraph 52, wherein the cancer is pancreatic cancer.
Paragraph 55. The method according to any one of paragraphs 1-54, wherein the patient does not have chronic obstructive pulmonary disease (COPD).
Paragraph 56. The method according to any one of paragraphs 1-54, wherein the patient has chronic obstructive pulmonary disease (COPD).
Paragraph 57. The method according to any one of paragraphs 1-56, wherein the patient is a smoker.
Paragraph 58. The method according to any one of paragraphs 1-56, wherein the patient is not a smoker.
Paragraph 59. The method according to any one of paragraphs 1-58, wherein the patient has impaired renal function.
Paragraph 60. The method according to any one of paragraphs 1-59, wherein the patient has elevated levels of at least one biomarker associated with renal disease.
Paragraph 61. The method of paragraph 60, wherein the biomarker is serum creatinine.
Paragraph 62. The method of paragraph 60, wherein the biomarker is cystatin C.
Paragraph 63. The method of paragraph 60, wherein the biomarker is uric acid.
Paragraph 64. The method according to any one of paragraphs 1-63, wherein the Alport syndrome is X-linked.
Paragraph 65. The method of paragraph 64, further comprising obtaining results from a test that determined the patient's Alport syndrome is X-linked.
Paragraph 66. The method of paragraph 64, wherein the Alport syndrome is autosomal recessive.
Paragraph 67. The method of paragraph 66, further comprising obtaining results from a test that determined the patient's Alport syndrome is autosomal recessive.
Paragraph 68. The method of paragraph 64, wherein the Alport syndrome is autosomal dominant.
Paragraph 69. The method of paragraph 68, further comprising obtaining results from a test that determined the patient's Alport syndrome is autosomal dominant.
Paragraph 70. The method according to any one of paragraphs 1-63, wherein the patient has a deletion or nonsense mutation of *COL4A5.*
Paragraph 71. The method of paragraph 70, further comprising obtaining results from a test that determined the patient has a deletion or nonsense mutation of *COL4A5.*
Paragraph 72. The method according to any one of paragraphs 1-63, wherein the patient has a splicing mutation of *COL4A5.*
Paragraph 73. The method of paragraph 72, further comprising obtaining results from a test that determined the patient has a splicing mutation of *COL4A5.*
Paragraph 74. The method according to any one of paragraphs 1-63, wherein the patient has a missense mutation of *COL4A5.*
Paragraph 75. The method of paragraph 74, further comprising obtaining results from a test that determined the patient has a missense mutation of *COL4A5.*
Paragraph 76. The method according to any one of paragraphs 1-63, wherein the patient has a mutation in either *COL4A3* or *COL4A4.*
Paragraph 77. The method of paragraph 76, further comprising obtaining results from a test that determined the patient has a mutation in either *COL4A3* or *COL4A4.*
Paragraph 78. The method according to any one of paragraphs 1-77, wherein the patient exhibits microhematuria.
Paragraph 79. The method of paragraph 78, wherein the patient exhibits hematuria.
Paragraph 80. The method according to any one of paragraphs 1-79, wherein the patient further exhibits microalbuminuria.
Paragraph 81. The method of paragraph 80, wherein the patient exhibits albuminuria.
Paragraph 82. The method of either paragraph 80 or paragraph 81, wherein the concentration of albumin in the urine is between 30 µg per mg of creatinine and 300 µg per mg of creatinine.
Paragraph 83. The method of either paragraph 80 or paragraph 81, wherein the concentration of albumin in the urine is greater than 300 µg per mg of creatinine.
Paragraph 84. The method according to any one of paragraphs 1-83, wherein the patient further exhibits proteinuria.
Paragraph 85. The method of paragraph 84, wherein the patient exhibits overt proteinuria.
Paragraph 86. The method of either paragraph 84 or paragraph 85, wherein the patient's urine exhibits the presence of multiple proteins.
Paragraph 87. The method according to any one of paragraphs 84-86, wherein the patient's urine exhibits a protein to creatinine ratio of greater than 0.2 mg/g.
Paragraph 88. The method of paragraph 87, wherein the patient's urine exhibits a protein to creatinine ratio of greater than 1.0 mg/g.
Paragraph 89. The method according to any one of paragraphs 1-88, wherein the patient has an eGFR less than 45 mL/min/1.73 m².
Paragraph 90. The method of paragraph 89, wherein the patient exhibits an ACR of less than 2000 mg/g.
Paragraph 91. The method according to any one of paragraphs 1-90, wherein the method results in reducing the protein to creatinine ratio in the patient's urine to less than 50% of the untreated level.
Paragraph 92. The method according to any one of paragraphs 1-91, wherein the method results in reducing the protein to creatinine ratio in the patient's urine to less than 1.0 mg/g.
Paragraph 93. The method according to any one of paragraphs 1-92, wherein the method results in reducing the albumin to creatinine ratio in patient's urine to less than 100 µg/mg.
Paragraph 94. The method of paragraph 93, wherein the method results in reducing the albumin to creatinine ratio in the patient's urine to less than 50 µg/mg.
Paragraph 95. The method according to any one of paragraphs 1-94, wherein the patient is less than 75 years old.
Paragraph 96. The method of paragraph 95, wherein the patient is less than 60 years old.
Paragraph 97. The method of paragraph 96, wherein the patient is less than 40 years old.
Paragraph 98. The method of paragraph 97, wherein the patient is less than 30 years old.
Paragraph 99. The method of paragraph 98, wherein the patient is less than 25 years old.
Paragraph 100. The method according to any one of paragraphs 1-99, wherein the patient does not have at least one of the following characteristics:
   (A) a cardiovascular disease;
   (B) an elevated baseline B-type natriuretic peptide (BNP) level;
   (C) an estimated glomerular filtration rate (eGFR) < 45 mL/min/1.73 m²; and
   (D) an elevated albumin/creatinine ratio (ACR) > 2000 mg/g.
Paragraph 101. The method of paragraph 100, wherein the patient does not have two of the characteristics.
Paragraph 102. The method of paragraph 100, wherein the patient does not have three of the characteristics.
Paragraph 103. The method of paragraph 102, wherein the patient does not have any of said characteristics.
Paragraph 104. The method according to any one of paragraph 1-103, wherein the patient is a human.
Paragraph 105. The method according to any one of paragraphs 1-104, wherein the patient is male.
Paragraph 106. The method according to any one of paragraphs 1-104, wherein the patient is female.
Paragraph 107. A method of improving the kidney function of a patient who has been diagnosed with Alport syndrome, comprising administering to the patient a therapeutically effective amount of a compound of the formula: 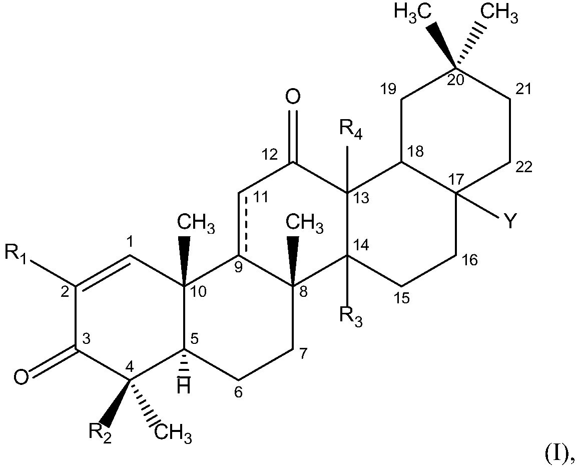 wherein:
   R₁ is -CN, halo, -CF₃, or -C(O)Rₐ, wherein Rₐ is -OH, alkoxy_{(C1-4)}, -NH₂, alkylamino(_{C1-4}), or -NH-S(O)₂-alkyl_{(C1-4)};
   R₂ is hydrogen or methyl;
   R₃ and R₄ are each independently hydrogen, hydroxy, methyl or as defined below when either of these groups is taken together with group R_{c}; and
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C≤12)}, heteroaryl_{(C≤8)}, heterocycloalkyl_{(C≤12}), alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonyl-amino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8}), alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8}), heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
         R_{c} and R₃, taken together, are -O- or -NR_{d}-, wherein R_{d} is hydrogen or alkyl_{(C≤8)}; or
         R_{c} and R₄, taken together, are -O- or -NR_{d}-, wherein R_{d} is hydrogen or alkyl_{(C≤4)}; or
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalky_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 108. The method of paragraph 107, wherein the compound is further defined as: 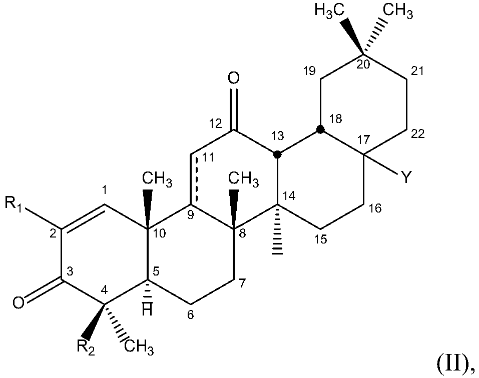 wherein:
   R₁ is -CN, halo, -CF₃, or -C(O)Rₐ, wherein Rₐ is -OH, alkoxy_{(C1-4)}, -NH₂, alkylamino_{(C1-4)}, or -NH-S(O)₂-alkyl_{(C1-4)};
   R₂ is hydrogen or methyl;
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C≤12)}, heteroaryl_{(C≤8)}, heterocycloalkyl_{(C≤12)}, alkoxy_{(C≤8)}, cycloalkoxy_{(C≤8)}, aryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonyl-amino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 109. The method of paragraph 108, wherein the compound is further defined as: 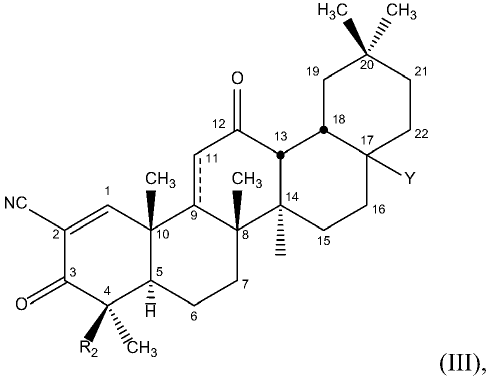 wherein:
   R₂ is hydrogen or methyl;
   Y is:
      -H, -OH, -SH, -CN, -F, -CF₃, -NH₂ or -NCO;
      alkyl_{(C≤8)}, cycloalkyl_{(C≤8)}, alkenyl_{(C≤8)}, alkynyl_{(C≤8)}, aryl_{(C≤12)}, aralkyl_{(C≤12)}, heteroaryl_{(C≤8)}, heterocycloalkyl_{(C≤12)}, alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonyl-amino_{(C≤8)}, cycloalkylsulfonylamino_{(C≤8)}, or substituted versions of any of these groups;
      -alkanediyl_{(C≤8)}-R_{b}, -alkenediyl_{(C≤8)}-R_{b}, or a substituted version of any of these groups, wherein R_{b} is:
         hydrogen, hydroxy, halo, amino or mercapto; or
         heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
      -(CH₂)ₘC(O)R_{c}, wherein m is 0-6 and R_{c} is:
         hydrogen, hydroxy, halo, amino, -NHOH, or mercapto; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
      -NHC(O)Rₑ, wherein Rₑ is:
         hydrogen, hydroxy, amino; or
         alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 110. The method of paragraph 109, wherein the compound is further defined as: 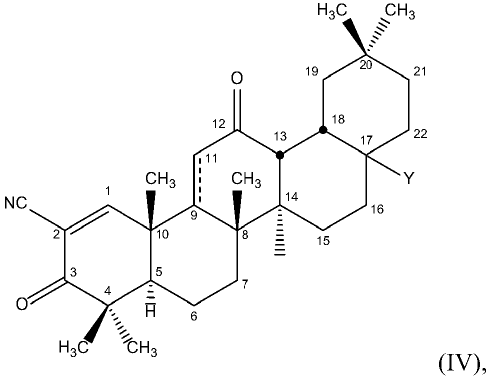 wherein:
   Y is:
   -H, -OH, -SH, -CN, -F, -CF₃, -NH₂ or -NCO;
   alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤12)}, alkoxy_{(C≤8)}, cycloalkoxy_{(C≤8)}, aryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonyl-amino_{(C≤8)}, cycloalkylsulfonylamino_{(C≤8)}, or substituted versions of any of these groups;
   -alkanediyl_{(C≤8)}-R_{d}, -alkenediyl_{(C≤8)}-R_{b}, or a substituted version of any of these groups, wherein R_{b} is:
      hydrogen, hydroxy, halo, amino or mercapto; or
      heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
   -(CH₂)ₘC(O)R_{c}, wherein m is 0-6 and R_{c} is:
      hydrogen, hydroxy, halo, amino, -NHOH, or mercapto; or
      alkyl_{(C≤8)}, cycloalkyl_{(C≤8)}, alkenyl_{(C≤8)}, alkynyl_{(C≤8)}, aryl_{(C≤8)}, aralkyl_{(C≤8)}**,** heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
   -NHC(O)Rₑ, wherein Rₑ is:
      hydrogen, hydroxy, amino; or
      alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}**,** heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 111. The method of paragraph 110, wherein the compound is further defined as: 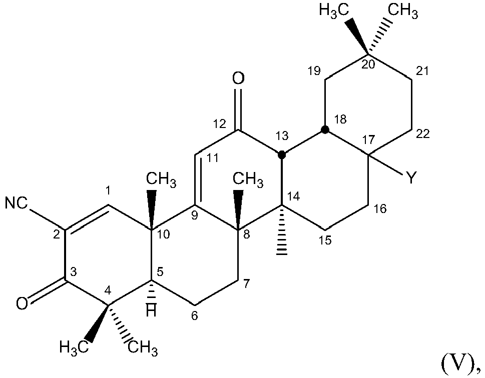 wherein:
   Y is:
   -H, -OH, -SH, -CN, -F, -CF₃, -NH₂ or -NCO;
   alkyl_{(C≤8)}, cycloalkyl_{(C≤8)}, alkenyl_{(C≤8)}, alkynyl_{(C≤8)}, aryl_{(C≤8)}, aralkyl_{(C≤12)}, heteroaryl_{(C≤8)}, heterocycloalkyl_{(C≤12)}, alkoxy_{(C≤8)}, cycloalkoxy_{(C≤8)}, aryloxy_{(C≤12)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, alkylthio_{(C≤8)}, acylthio_{(C≤8)}, alkylsulfonyl-amino_{(C≤8)} cycloalkylsulfonylamino_{(C≤8)}, or substituted versions of any of these groups;
   -alkanediyl_{(C≤8)}-R_{b}, -alkenediyl_{(C≤8)}-R_{b}, or a substituted version of any of these groups, wherein R_{b} is:
      hydrogen, hydroxy, halo, amino or mercapto; or
      heteroaryl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, aralkylamino_{(C≤8)}, heteroarylamino_{(C≤8)}, alkylsulfonylamino_{(C≤8)}, cycloalkylsulfonylamino_{(C≤8)}, amido_{(C≤8)}, -OC(O)NH-alkyl_{(C≤8)}, or a substituted version of any of these groups;
   -(CH₂)ₘC(O)R_{c}, wherein m is 0-6 and R_{c} is:
      hydrogen, hydroxy, halo, amino, -NHOH, or mercapto; or
      alkyl_{(C≤8)}, cycloalkyl_{(C≤8)}, alkenyl_{(C≤8)}, alkynyl(c≤₈), aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, alkenyloxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, alkylsulfonylamino_{(C≤8)}, cycloalkylsulfonylamino_{(C≤8)}, amido_{(C≤8)}, -NH-alkoxy_{(C≤8)}, -NH-heterocycloalkyl_{(C≤8)}, -NH-amido_{(C≤8)}, or a substituted version of any of these groups;
   -NHC(O)Rₑ, wherein Rₑ is:
      hydrogen, hydroxy, amino; or
      alkyl_{(C≤8)}, cycloalkyl_{(C≤8)}, alkenyl_{(C≤8)}, alkynyl_{(C≤8)}, aryl_{(C≤8)}, aralkyl_{(C≤8)}, heteroaryl_{(C≤8)}, heterocycloalkyl_{(C≤8)}, alkoxy_{(C≤8)}, cycloalkoxy_{(C≤8)}, aryloxy_{(C≤8)}, aralkoxy_{(C≤8)}, heteroaryloxy_{(C≤8)}, acyloxy_{(C≤8)}, alkylamino_{(C≤8)}, cycloalkylamino_{(C≤8)}, dialkylamino_{(C≤8)}, arylamino_{(C≤8)}, or a substituted version of any of these groups;
   or a pharmaceutically acceptable salt thereof.
Paragraph 112. The method of paragraph 111, wherein the compound is further defined as: 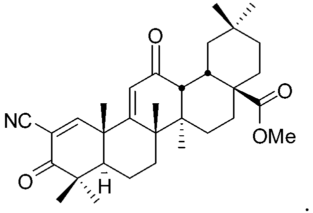
Paragraph 113. The method according to any one of paragraphs 107-112, wherein the patient does not have cardiovascular disease.
Paragraph 114. The method according to any one of paragraphs 107-112, wherein the patient has cardiovascular disease.
Paragraph 115. The method according to either paragraph 113 or paragraph 114, wherein the cardiovascular disease is left-sided myocardial disease.
Paragraph 116. The method according to either paragraph 113 or paragraph 114, wherein the cardiovascular disease is atherosclerosis.
Paragraph 117. The method according to either paragraph 113 or paragraph 114, wherein the cardiovascular disease is restenosis.
Paragraph 118. The method according to either paragraph 113 or paragraph 114, wherein the cardiovascular disease is thrombosis.
Paragraph 119. The method according to either paragraph 113 or paragraph 114, wherein the cardiovascular disease is pulmonary hypertension.
Paragraph 120. The method of paragraph 119, wherein the pulmonary hypertension is World Health Organization (WHO) Class I pulmonary hypertension (pulmonary arterial hypertension or PAH).
Paragraph 121. The method of paragraph 120, wherein the pulmonary hypertension is pulmonary arterial hypertension associated with connective tissue disease.
Paragraph 122. The method of paragraph 120, wherein the pulmonary hypertension is idiopathic pulmonary arterial hypertension.
Paragraph 123. The method of paragraph 119, wherein the pulmonary hypertension is WHO Class II pulmonary hypertension.
Paragraph 124. The method of paragraph 119, wherein the pulmonary hypertension is WHO Class III pulmonary hypertension.
Paragraph 125. The method of paragraph 119, wherein the pulmonary hypertension is WHO Class IV pulmonary hypertension.
Paragraph 126. The method of paragraph 119, wherein the pulmonary hypertension is WHO Class V pulmonary hypertension.
Paragraph 127. The method according to any one of paragraphs 107-126, wherein the patient does not have endothelial dysfunction.
Paragraph 128. The method according to any one of paragraphs 107-126, wherein the patient has endothelial dysfunction.
Paragraph 129. The method according to any one of paragraphs 107-128, wherein the patient does not have Stage 4 or higher chronic kidney disease.
Paragraph 130. The method according to any one of paragraphs 107-128, wherein the patient does not have an estimated glomerular filtration rate (eGFR) less than 45 mL/min/1.73 m².
Paragraph 131. The method according to any one of paragraphs 107-128, wherein the patient does not have an elevated albumin/creatinine ratio (ACR) is greater than 2000 mg/g.
Paragraph 132. The method according to any one of paragraphs 107-131, wherein the patient does not have diabetes.
Paragraph 133. The method according to any one of paragraphs 107-131, wherein the patient has diabetes.
Paragraph 134. The method of either paragraph 132 or paragraph 133, wherein the diabetes is Type 2 diabetes.
Paragraph 135. The method according to any one of paragraphs 107-134, wherein the patient does not have a complication associated with diabetes.
Paragraph 136. The method according to any one of paragraphs 107-134, wherein the patient has a complication associated with diabetes.
Paragraph 137. The method according to either paragraph 135 or paragraph 136, wherein the complication associated with diabetes is diabetic nephropathy.
Paragraph 138. The method according to either paragraph 135 or paragraph 136, wherein the complication is selected from the group consisting of obesity, stroke, peripheral vascular disease, neuropathy, myonecrosis, retinopathy and metabolic syndrome (syndrome X).
Paragraph 139. The method according to any one of paragraphs 107-138, wherein the patient does not have insulin resistance.
Paragraph 140. The method according to any one of paragraphs 107-138, wherein the patient has insulin resistance.
Paragraph 141. The method according to any one of paragraphs 107-140, wherein the patient does not have fatty liver disease.
Paragraph 142. The method according to any one of paragraphs 107-140, wherein the patient has fatty liver disease.
Paragraph 143. The method according to any one of paragraphs 107-140, wherein the patient does not have hepatic impairment.
Paragraph 144. The method according to any one of paragraphs 107-140, wherein the patient has hepatic impairment.
Paragraph 145. The method according to any one of paragraphs 107-144, wherein the patient is not overweight.
Paragraph 146. The method according to any one of paragraphs 107-144, wherein the patient is overweight.
Paragraph 147. The method according to either paragraph 145 or paragraph 146, where the patient is obese.
Paragraph 148. The method of paragraph 147, where the obesity is class I.
Paragraph 149. The method of paragraph 147, where the obesity is class II.
Paragraph 150. The method of paragraph 147, where the obesity is class III.
Paragraph 151. The method according to either paragraph 146 or paragraph 147, where the patient's body mass index (BMI) is from 25 kg/m² to 30 kg/m².
Paragraph 152. The method according to either paragraph 146 or paragraph 147, where the patient's BMI is from 30 kg/m² to 35 kg/m².
Paragraph 153. The method according to either paragraph 146 or paragraph 147, where the patient's BMI is from 35 kg/m² to 40 kg/m².
Paragraph 154. The method according to either paragraph 146 or paragraph 147, where the patient's BMI is from 40 kg/m² to 80 kg/m².
Paragraph 155. The method according to any one of paragraphs 107-154, wherein the patient has cancer.
Paragraph 156. The method according to any one of paragraphs 107-154, wherein the patient does not have cancer.
Paragraph 157. The method of either paragraph 155 or paragraph 156, wherein the cancer is an advanced solid tumor or lymphoid malignancy.
Paragraph 158. The method of either paragraph 155 or paragraph 156, wherein the cancer is selected from the groups consisting of breast cancer, prostate cancer, colon cancer, brain cancer, melanoma, pancreatic cancer, ovarian cancer, leukemia, or bone cancer.
Paragraph 159. The method of paragraph 158, wherein the cancer is an advanced malignant melanoma.
Paragraph 160. The method of paragraph 158, wherein the cancer is pancreatic cancer.
Paragraph 161. The method according to any one of paragraphs 107-160, wherein the patient does not have chronic obstructive pulmonary disease (COPD).
Paragraph 162. The method according to any one of paragraphs 107-160, wherein the patient has chronic obstructive pulmonary disease (COPD).
Paragraph 163. The method according to any one of paragraphs 107-162, wherein the patient is a smoker.
Paragraph 164. The method according to any one of paragraphs 107-162, wherein the patient is not a smoker.
Paragraph 165. The method according to any one of paragraphs 107-164, wherein the patient has impaired renal function.
Paragraph 166. The method according to any one of paragraphs 107-165, wherein the patient has elevated levels of at least one biomarker associated with renal disease.
Paragraph 167. The method of paragraph 166, wherein the biomarker is serum creatinine.
Paragraph 168. The method of paragraph 166, wherein the biomarker is cystatin C.
Paragraph 169. The method of paragraph 166, wherein the biomarker is uric acid.
Paragraph 170. The method according to any one of paragraphs 107-169, wherein the Alport syndrome is X-linked.
Paragraph 171. The method of paragraph 170, further comprising obtaining results from a test that determined the patient's Alport syndrome is X-linked.
Paragraph 172. The method of paragraph 170, wherein the Alport syndrome is autosomal recessive.
Paragraph 173. The method of paragraph 172, further comprising obtaining results from a test that determined the patient's Alport syndrome is autosomal recessive.
Paragraph 174. The method of paragraph 170, wherein the Alport syndrome is autosomal dominant.
Paragraph 175. The method of paragraph 174, further comprising obtaining results from a test that determined the patient's Alport syndrome is autosomal dominant.
Paragraph 176. The method according to any one of paragraphs 107-169, wherein the patient has a deletion or nonsense mutation of *COL4A5.*
Paragraph 177. The method of paragraph 176, further comprising obtaining results from a test that determined the patient has a deletion or nonsense mutation of *COL4A5.*
Paragraph 178. The method according to any one of paragraphs 107-169, wherein the patient has a splicing mutation of *COL4A5.*
Paragraph 179. The method of paragraph 178, further comprising obtaining results from a test that determined the patient has a splicing mutation of *COL4A5.*
Paragraph 180. The method according to any one of paragraphs 107-169, wherein the patient has a missense mutation of *COL4A5.*
Paragraph 181. The method of paragraph 180, further comprising obtaining results from a test that determined the patient has a missense mutation of *COL4A5.*
Paragraph 182. The method according to any one of paragraphs 107-169, wherein the patient has a mutation in either *COL4A3* or *COL4A4.*
Paragraph 183. The method of paragraph 182, further comprising obtaining results from a test that determined the patient has a mutation in either *COL4A3* or *COL4A4.*
Paragraph 184. The method according to any one of paragraphs 107-183, wherein the patient exhibits microhematuria.
Paragraph 185. The method of paragraph 184, wherein the patient exhibits hematuria.
Paragraph 186. The method according to any one of paragraphs 107-185, wherein the patient further exhibits microalbuminuria.
Paragraph 187. The method of paragraph 186, wherein the patient exhibits albuminuria.
Paragraph 188. The method of either paragraph 186 or paragraph 187, wherein the concentration of albumin in the urine is between 30 µg per mg of creatinine and 300 µg per mg of creatinine.
Paragraph 189. The method of either paragraph 186 or paragraph 187, wherein the concentration of albumin in the urine is greater than 300 µg per mg of creatinine.
Paragraph 190. The method according to any one of paragraphs 107-189, wherein the patient further exhibits proteinuria.
Paragraph 191. The method of paragraph 190, wherein the patient exhibits overt proteinuria.
Paragraph 192. The method of either paragraph 190 or paragraph 191, wherein the patient's urine exhibits the presence of multiple proteins.
Paragraph 193. The method according to any one of paragraphs 190-192, wherein the patient's urine exhibits a protein to creatinine ratio of greater than 0.2 mg/g.
Paragraph 194. The method of paragraph 193, wherein the patient's urine exhibits a protein to creatinine ratio of greater than 1.0 mg/g.
Paragraph 195. The method according to any one of paragraphs 107-194, wherein the patient has an eGFR less than 45 mL/min/1.73 m².
Paragraph 196. The method of paragraph 195, wherein the patient exhibits an ACR of less than 2000 mg/g.
Paragraph 197. The method according to any one of paragraphs 107-196, wherein the method results in reducing the protein to creatinine ratio in the patient's urine to less than 50% of the untreated level.
Paragraph 198. The method according to any one of paragraphs 107-197, wherein the method results in reducing the protein to creatinine ratio in the patient's urine to less than 1.0 mg/g.
Paragraph 199. The method according to any one of paragraphs 107-198, wherein the method results in reducing the albumin to creatinine ratio in patient's urine to less than 100 µg/mg.
Paragraph 200. The method of paragraph 199, wherein the method results in reducing the albumin to creatinine ratio in the patient's urine to less than 50 µg/mg.
Paragraph 201. The method according to any one of paragraphs 107-200, wherein the patient is less than 75 years old.
Paragraph 202. The method of paragraph 201, wherein the patient is less than 60 years old.
Paragraph 203. The method of paragraph 202, wherein the patient is less than 40 years old.
Paragraph 204. The method of paragraph 203, wherein the patient is less than 30 years old.
Paragraph 205. The method of paragraph 204, wherein the patient is less than 25 years old.
Paragraph 206. The method according to any one of paragraphs 107-205, wherein the patient does not have at least one of the following characteristics:
   (A) a cardiovascular disease;
   (B) an elevated baseline B-type natriuretic peptide (BNP) level;
   (C) an estimated glomerular filtration rate (eGFR) < 45 mL/min/1.73 m²; and
   (D) an elevated albumin/creatinine ratio (ACR) > 2000 mg/g.
Paragraph 207. The method of paragraph 206, wherein the patient does not have two of the characteristics.
Paragraph 208. The method of paragraph 206, wherein the patient does not have three of the characteristics.
Paragraph 209. The method of paragraph 208, wherein the patient does not have any of said characteristics.
Paragraph 210. The method according to any one of paragraphs 107-209, wherein the patient is a human.
Paragraph 211. The method according to any one of paragraphs 107-210, wherein the patient is male.
Paragraph 212. The method according to any one of paragraphs 107-210, wherein the patient is female.
Paragraph 213. The method according to any one of paragraphs 1-212, wherein at least a portion of the compound is present as a crystalline form having an X-ray diffraction pattern (CuKα) comprising significant diffraction peaks at about 8.8, 12.9, 13.4, 14.2 and 17.4 °2θ .
Paragraph 214. The method of paragraph 213, wherein the X-ray diffraction pattern (CuKα) is substantially as shown in FIG. 1A or FIG. 1B.
Paragraph 215. The method according to any one of paragraphs 1-212, wherein at least a portion of the compound is present as an amorphous form having an X-ray diffraction pattern (CuKα) with a peak at approximately 13.5 °2θ, substantially as shown in FIG. 1C, and a transition glass temperature (T_{g}).
Paragraph 216. The method of paragraph 215, wherein the T_{g} value is in the range of about 120 °C to about 135 °C.
Paragraph 217. The method of paragraph 216, wherein the T_{g} value is in the range of about 125 °C to about 130 °C.
Paragraph 218. The method according to any one of paragraphs 1-217, wherein the therapeutically effective amount is a daily dose from about 0.1 mg to about 300 mg of the compound.
Paragraph 219. The method of paragraph 218, wherein the daily dose is from about 0.5 mg to about 200 mg of the compound.
Paragraph 220. The method of paragraph 219, wherein the daily dose is from about 1 mg to about 150 mg of the compound.
Paragraph 221. The method of paragraph 220, wherein the daily dose is from about 1 mg to about 75 mg of the compound.
Paragraph 222. The method of paragraph 221, wherein the daily dose is from about 1 mg to about 20 mg of the compound.
Paragraph 223. The method of paragraph 218, wherein the daily dose is from about 2.5 mg to about 30 mg of the compound.
Paragraph 224. The method of paragraph 223, wherein the daily dose is about 2.5 mg of the compound.
Paragraph 225. The method of paragraph 223, wherein the daily dose is about 5 mg of the compound.
Paragraph 226. The method of paragraph 223, wherein the daily dose is about 10 mg of the compound.
Paragraph 227. The method of paragraph 223, wherein the daily dose is about 15 mg of the compound.
Paragraph 228. The method of paragraph 223, wherein the daily dose is about 20 mg of the compound.
Paragraph 229. The method of paragraph 223, wherein the daily dose is about 30 mg of the compound.
Paragraph 230. The method according to any of paragraphs 1-217, wherein the therapeutically effective amount is a daily dose is 0.01 - 100 mg of compound per kg of body weight.
Paragraph 231. The method of paragraph 230, wherein the daily dose is 0.05 - 30 mg of compound per kg of body weight.
Paragraph 232. The method of paragraph 231, wherein the daily dose is 0.1 - 10 mg of compound per kg of body weight.
Paragraph 233. The method of paragraph 232, wherein the daily dose is 0.1 - 5 mg of compound per kg of body weight.
Paragraph 234. The method of paragraph 233, wherein the daily dose is 0.1 - 2.5 mg of compound per kg of body weight.
Paragraph 235. The method according to any of paragraphs 1-217, wherein the compound is administered as a single dose to the patient per day.
Paragraph 236. The method according to any of paragraphs 1-217, wherein the compound is administered as two or more doses to the patient per day.
Paragraph 237. The method according to any one of paragraphs 1-217, wherein the compound is administered orally, intraarterially or intravenously.
Paragraph 238. The method according to any one of paragraphs 1-217, wherein the compound is formulated as a hard or soft capsule or a tablet.
Paragraph 239. The method according to any one of paragraphs 1-217, wherein the compound is formulated as a solid dispersion comprising (i) the compound and (ii) an excipient.
Paragraph 240. The method of paragraph 239, wherein the excipient is a methacrylic acid - ethyl acrylate copolymer.
Paragraph 241. The method of paragraph 240, wherein the copolymer comprises methacrylic acid and ethyl acrylate at a 1:1 ratio.
Paragraph 242. The method according to any of paragraphs 1-241, further comprising administering to patient a second therapy.
Paragraph 243. The method of paragraph 242, wherein the second therapy comprises administering to said patient a therapeutically effective amount of a second drug.
Paragraph 244. The method of paragraph 243, wherein the second drug is an angiotensin-converting enzyme inhibitor, an angiotensin receptor blocker, or an aldosterone antagonist.
Paragraph 245. The method of paragraph 244, wherein the angiotensin-converting enzyme inhibitor is Ramipril, enalapril, Lisinopril, benazepril, fosinopril, quinapril, cilazapril, perinopril, or trandolapril.
Paragraph 246. The method of paragraph 244, wherein the angiotensin receptor blocker is losartan, candesartan, irbesartan, telmisartan, valsartan, or epresartan.
Paragraph 247. The method of paragraph 244, wherein the aldosterone antagonist is spironolactone.

## Claims

1. A compound of the formula: 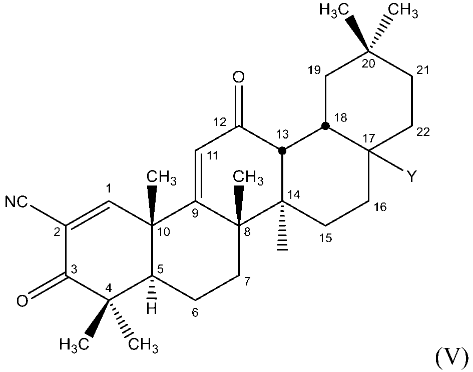 wherein:
Y is:
-(CH₂)ₘC(O)R_{c}, wherein m is 0 and R_{c} is:
heteroaryl_{(C≤8)}, alkoxy_{(C≤8)}, alkylamino_{(C≤8)}, or a substituted alkylamino_{(C≤8)} wherein one or more hydrogen atom has been replaced by -F;
-NHC(O)Rₑ, wherein Rₑ is:
alkyl_{(C≤8)} or a substituted alkyl_{(C≤8)} wherein one or more hydrogen atom has been replaced by -F;
or a pharmaceutically acceptable salt thereof,
for use in treating or preventing one or more symptoms of Alport syndrome in a patient in need thereof, wherein the patient has been identified as having a B-type natriuretic peptide (BNP) level that is not greater than 200 pg/mL.

2. A compound of the formula: 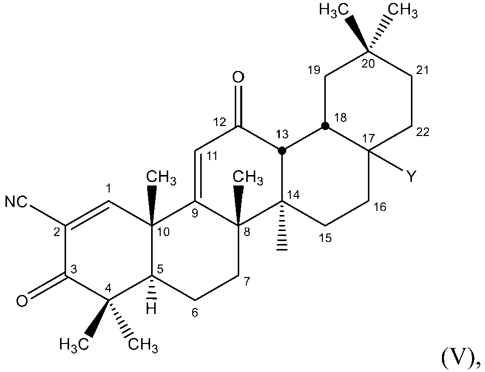 wherein:
Y is:
-(CH₂)ₘC(O)Rₑ, wherein m is 0 and R_{c} is:
heteroaryl_{(C≤8)}, alkoxy_{(C≤8)}, alkylamino_{(C≤8)}, or a substituted alkylamino_{(C≤8)} wherein one or more hydrogen atom has been replaced by -F;
-NHC(O)Rₑ, wherein Rₑ is:
alkyl_{(C≤8)} or a substituted alkyl_{(C≤8)} wherein one or more hydrogen atom has been replaced by -F;
or a pharmaceutically acceptable salt thereof,
for use in improving the kidney function of a patient who has been diagnosed with Alport syndrome, wherein the patient has been identified as having a B-type natriuretic peptide (BNP) level that is not greater than 200 pg/mL.

3. The compound for use according to claim 1 or claim 2, wherein the patient does not have cardiovascular disease, optionally wherein: the cardiovascular disease is left-sided myocardial disease, atherosclerosis, restenosis, thrombosis or pulmonary hypertension.

4. The compound for use according to any one of claims 1 to 3, wherein:
(i) the patient does not have endothelial dysfunction;
(ii) the patient does not have Stage 4 or higher chronic kidney disease;
(iii) the patient does not have an estimated glomerular filtration rate (eGFR) less than 45 mL/min/1.73 m²;
(iv) patient does not have an elevated albumin/creatinine ratio (ACR) that is greater than 2000 mg/g;
(v) the patient does not have diabetes, optionally wherein the patient does not have Type 2 diabetes;
(vi) the patient does not have a complication associated with diabetes, optionally wherein the patient does not have diabetic nephropathy;
(vii) the patient does not have insulin resistance;
(viii) the patient does not have fatty liver disease;
(ix) the patient does not have hepatic impairment;
(x) the patient is not overweight;
(xi) the patient does not have cancer;
(xii) the patient does not have chronic obstructive pulmonary disease (COPD); or
(xiii) the patient is not a smoker.

5. The compound for use according to any one of claims 1 to 4, wherein
(i) the patient has impaired renal function; or
(ii) the patient has elevated levels of at least one biomarker associated with renal disease, optionally wherein the biomarker is serum creatinine, cystatin C or uric acid.

6. The compound for use according to any one of claims 1 to 5, wherein the Alport syndrome is X-linked; optionally wherein:
(i) the Alport syndrome is autosomal recessive; or
(ii) the Alport syndrome is autosomal dominant.

7. The compound for use according to any one of claims 1 to 6, wherein:
(i) the patient has a deletion or nonsense mutation of *COL4A5*;
(ii) the patient has a splicing mutation of *COL4A5*;
(iii) the patient has a missense mutation of *COL4A5*; or
(iv) the patient has a mutation in either *COL4A3* or *COL4A4.*

8. The compound for use according to any one of claims 1 to 7, wherein:
(i) the patient exhibits microhematuria, optionally wherein the patient exhibits hematuria;
(ii) the patient exhibits microalbuminuria, optionally wherein the patient exhibits albuminuria; or
(iii) the patient exhibits proteinuria, optionally wherein the patient exhibits overt proteinuria.

9. The compound for use according to any one of claims 1 to 8, wherein the patient is less than 75 years old.

10. The compound for use according to any one of claims 1 to 9, wherein the patient does not have at least one of the following characteristics:
(A) a cardiovascular disease;
(B) an estimated glomerular filtration rate (eGFR) < 45 mL/min/1.73 m²; and
(C) an elevated albumin/creatinine ratio (ACR) > 2000 mg/g.

11. The compound for use according to any one of claims 1 to 10, wherein the compound is present in a daily dose of from about 0.1 mg to about 300 mg of the compound.

12. The compound for use according to any one of claims 1 to 11, wherein the compound is formulated for oral, intraarterial or intravenous administration.

13. The compound for use according to any one of claims 1 to 12, wherein the compound is formulated as a hard or soft capsule or a tablet.

14. The compound for use according to any one of claims 1 to 13, wherein the compound is formulated as a solid dispersion comprising (i) the compound and (ii) an excipient; optionally wherein the excipient is a methacrylic acid - ethyl acrylate copolymer.
